# EUROPEAN PATENT APPLICATION

(11) **EP 2 511 382 A1**
(43) Date of publication of application: **17.10.2012**
(21) Application number: 11305447.2
(22) Date of filing: 14.04.2011
(51) Int. Cl.: C12Q 1/68

(54) **Process for identifying novel anti-inflammatory molecules with reduced direct transrepression of genes induced by glucocorticoids**

(71) Applicant: Université de Strasbourg, 67000 Strasbourg (FR)
(72) Inventor: Chambon, Pierre, 67113 Blaesheim (FR); Metzger, Daniel, 67200 Strasbourg (FR); Surjit, Milan, 67400 Illkirch (FR)
(74) Representative: Faivre Petit, Frédérique

(57) **Abstract**

The present invention relates to a new process for identifying novel anti-inflammatory molecules with reduced direct transrepression of genes induced by glucocorticoids.

The inventors have discovered that GCs-mediated transrepression can be mediated not only via the tethering indirect pathway, but also through direct binding of GR to "simple" negative GREs (nGRE), which belongs to a novel family of evolutionary-conserved cis-acting negative response elements (IR nGREs), and are found in numerous GC-repressed genes.

SEQUENCE LISTING

<110>  Universite de Strasbourg

<120>  PROCESS FOR IDENTIFYING NOVEL ANTI-INFLAMMATORY MOLECULES WITH
       REDUCED DIRECT TRANSREPRESSION OF GENES INDUCED BY
       GLUCOCORTICOIDS

<130>  D29637

<160>  56

<170>  PatentIn version 3.3

<210>  1
<211>  10
<212>  DNA
<213>  artificial

<220>
<223>  IR1 nGC Response Element (GRE)


<220>
<221>  misc_feature
<222>  (5)..(5)
<223>  n is a, c, g, or t

<400>  1
ctccnggaga                                                              10


<210>  2
<211>  9
<212>  DNA
<213>  artificial

<220>
<223>  IR0 GRE

<400>  2
ctccggaga                                                                9


<210>  3
<211>  11
<212>  DNA
<213>  artificial

<220>
<223>  IR2 GRE


<220>
<221>  misc_feature
<222>  (5)..(6)
<223>  n is a, c, g, or t

<400>  3
ctccnnggag a                                                            11

<210>  4
<211>  10
<212>  DNA
<213>  artificial

<220>
<223>  NFkB DNA Binding Site (DBS)


<220>
<221>  misc_feature
<222>  (5)..(6)
<223>  n is a, c, g, or t

<400>  4
gggrnnyycc                                                              10


<210>  5
<211>  10
<212>  DNA
<213>  artificial

<220>
<223>  NFkB (DBS)

<400>  5
gggaatttcc                                                              10


<210>  6
<211>  10
<212>  DNA
<213>  artificial

<220>
<223>  NFkB DBS

<400>  6
gggactttcc                                                              10


<210>  7
<211>  7
<212>  DNA
<213>  artificial

<220>
<223>  AP1 DBS

<400>  7
tgagtca                                                                  7


<210>  8
<211>  7
<212>  DNA
<213>  artificial

<220>

<223>  AP1 DBS

<400>  8
tgactca                                                                 7


<210>  9
<211>  15
<212>  DNA
<213>  artificial

<220>
<223>  (+)GRE DBS consensus


<220>
<221>  misc_feature
<222>  (3)..(3)
<223>  n is a, c, g, or t

<220>
<221>  misc_feature
<222>  (7)..(9)
<223>  n is a, c, g, or t

<220>
<221>  misc_feature
<222>  (13)..(13)
<223>  n is a, c, g, or t

<400>  9
rgnacannnt gtnct                                                        15


<210>  10
<211>  15
<212>  DNA
<213>  artificial

<220>
<223>  (+) GRE DBS

<400>  10
tgtacaggat gttct                                                        15


<210>  11
<211>  10
<212>  DNA
<213>  artificial

<220>
<223>  tolerable variants of IR nGRE DBS

<400>  11
atccaggaga                                                              10


<210>  12
<211>  10
<212>  DNA

<213>  artificial

<220>
<223>  tolerable variants of IR nGRE DBS

<400>  12
gtccaggaga                                                              10


<210>  13
<211>  10
<212>  DNA
<213>  artificial

<220>
<223>  tolerable variants of IR nGRE DBS

<400>  13
ttccaggaga                                                              10


<210>  14
<211>  10
<212>  DNA
<213>  artificial

<220>
<223>  tolerable variants of IR nGRE DBS

<400>  14
caccaggaga                                                              10


<210>  15
<211>  10
<212>  DNA
<213>  artificial

<220>
<223>  tolerable variants of IR nGRE DBS

<400>  15
ccccaggaga                                                              10


<210>  16
<211>  10
<212>  DNA
<213>  artificial

<220>
<223>  tolerable variants of IR nGRE DBS

<400>  16
ctgcaggaga                                                              10


<210>  17
<211>  10
<212>  DNA
<213>  artificial

<220>
<223>  tolerable variants of IR nGRE DBS

<400>  17
cttcaggaga                                                              10


<210>  18
<211>  10
<212>  DNA
<213>  artificial

<220>
<223>  tolerable variants of IR nGRE DBS

<400>  18
ctcgaggaga                                                              10


<210>  19
<211>  10
<212>  DNA
<213>  artificial

<220>
<223>  tolerable variants of IR nGRE DBS

<400>  19
ctctaggaga                                                              10


<210>  20
<211>  10
<212>  DNA
<213>  artificial

<220>
<223>  tolerable variants of IR nGRE DBS

<400>  20
ctccatgaga                                                              10


<210>  21
<211>  10
<212>  DNA
<213>  artificial

<220>
<223>  tolerable variants of IR nGRE DBS

<400>  21
ctccaggcga                                                              10


<210>  22
<211>  10
<212>  DNA
<213>  artificial

<220>
<223>  tolerable variants of IR nGRE DBS

<400>  22
   ctccagggga                                                              10


<210>  23
<211>  10
<212>  DNA
<213>  artificial

<220>
<223>  tolerable variants of IR nGRE DBS

<400>  23
ctccaggtga                                                             10


<210>  24
<211>  10
<212>  DNA
<213>  artificial

<220>
<223>  tolerable variants of IR nGRE DBS

<400>  24
ctccaggaaa                                                             10


<210>  25
<211>  10
<212>  DNA
<213>  artificial

<220>
<223>  tolerable variants of IR nGRE DBS

<400>  25
ctccaggata                                                             10


<210>  26
<211>  10
<212>  DNA
<213>  artificial

<220>
<223>  tolerable variants of IR nGRE DBS

<400>  26
ctccaggagc                                                             10


<210>  27
<211>  10
<212>  DNA
<213>  artificial

<220>

<223>  tolerable variants of IR nGRE DBS

<400>  27
ctccaggagg                                                              10


<210>  28
<211>  11
<212>  DNA
<213>  artificial

<220>
<223>  tolerable variants of IR nGRE DBS

<400>  28
ctccacggag a                                                            11


<210>  29
<211>  11
<212>  DNA
<213>  artificial

<220>
<223>  tolerable variants of IR nGRE DBS

<400>  29
atccacggag a                                                            11


<210>  30
<211>  11
<212>  DNA
<213>  artificial

<220>
<223>  tolerable variants of IR nGRE DBS

<400>  30
gtccacggag a                                                            11


<210>  31
<211>  11
<212>  DNA
<213>  artificial

<220>
<223>  tolerable variants of IR nGRE DBS

<400>  31
ttccacggag a                                                            11


<210>  32
<211>  11
<212>  DNA
<213>  artificial

<220>
<223>  tolerable variants of IR nGRE DBS

<400>  32
caccacggag a                                                            11


<210>  33
<211>  11
<212>  DNA
<213>  artificial

<220>
<223>  tolerable variants of IR nGRE DBS

<400>  33
ccccacggag a                                                            11


<210>  34
<211>  11
<212>  DNA
<213>  artificial

<220>
<223>  tolerable variants of IR nGRE DBS

<400>  34
ctgcacggag a                                                            11


<210>  35
<211>  11
<212>  DNA
<213>  artificial

<220>
<223>  tolerable variants of IR nGRE DBS

<400>  35
cttcacggag a                                                            11


<210>  36
<211>  11
<212>  DNA
<213>  artificial

<220>
<223>  tolerable variants of IR nGRE DBS

<400>  36
ctcgacggag a                                                            11


<210>  37
<211>  11
<212>  DNA
<213>  artificial

<220>
<223>  tolerable variants of IR nGRE DBS

<400>  37
ctctacggag a                                                            11


<210>  38
<211>  11
<212>  DNA
<213>  artificial

<220>
<223>  tolerable variants of IR nGRE DBS

<400>  38
ctccaccgag a                                                            11


<210>  39
<211>  11
<212>  DNA
<213>  artificial

<220>
<223>  tolerable variants of IR nGRE DBS

<400>  39
ctccactgag a                                                            11


<210>  40
<211>  11
<212>  DNA
<213>  artificial

<220>
<223>  tolerable variants of IR nGRE DBS

<400>  40
ctccacgcag a                                                            11


<210>  41
<211>  11
<212>  DNA
<213>  artificial

<220>
<223>  tolerable variants of IR nGRE DBS

<400>  41
ctccacggcg a                                                            11


<210>  42
<211>  11
<212>  DNA
<213>  artificial

<220>
<223>  tolerable variants of IR nGRE DBS

<400>  42

ctccacgggg a                                                             11


<210>  43
<211>  11
<212>  DNA
<213>  artificial

<220>
<223>  tolerable variants of IR nGRE DBS

<400>  43
ctccacggtg a                                                             11


<210>  44
<211>  11
<212>  DNA
<213>  artificial

<220>
<223>  tolerable variants of IR nGRE DBS

<400>  44
ctccacggaa a                                                             11


<210>  45
<211>  11
<212>  DNA
<213>  artificial

<220>
<223>  tolerable variants of IR nGRE DBS

<400>  45
ctccacggac a                                                             11


<210>  46
<211>  11
<212>  DNA
<213>  artificial

<220>
<223>  tolerable variants of IR nGRE DBS

<400>  46
ctccacggat a                                                             11


<210>  47
<211>  11
<212>  DNA
<213>  artificial

<220>
<223>  tolerable variants of IR nGRE DBS

<400>  47
ctccacggag c                                                             11

<210>  48
<211>  11
<212>  DNA
<213>  artificial

<220>
<223>  tolerable variants of IR nGRE DBS

<400>  48
ctccacggag g                                                            11


<210>  49
<211>  5557
<212>  DNA
<213>  artificial

<220>
<223>  PGL3(SV40/VDRE) IR1 nGRE

<400>  49
ggtaccctgt ggaatgtgtg tcagttaggg tgtggaaagt ccccaggctc cccagcaggc      60

agaagtatgc aaagcatgca tctcaattag tcagcaacca ggtgtggaaa gtccccaggc     120

tccccagcag gcagaagtat gcaaagcatg catctcaatt agtcagcaac caggtaccag     180

gtcagagagg tcaaccacta gaagacaggt cagagaggtc atgtggaaat caaaggaaag     240

gattttgtta cattggccac agaaagtttc tcatttcacc ttctagtctt ccatatccta     300

aagagtctag agggactttg tgaattcaat ggctcaggcc aataaagcaa tctgaacatc     360

atgtatacta caaataagtg agcaaagtgg acagtcgtca cagtcatcac aaaccacaca     420

gcctatagcg tattacctgg tcactatgcc aaattgggac taatcaaaaa gaatataaac     480

aatgaaacaa ggggttgaag caatggttca gcaattaaga gcacacaggc actgcgagct     540

cttacgcgtg ctagcccggg gctgagctcc aggagagtag ggagatctgc gatctgcatc     600

tcaattagtc agcaaccata gtcccgcccc taactccgcc catcccgccc ctaactccgc     660

ccagttccgc ccattctccg ccccatcgct gactaatttt ttttatttat gcagaggccg     720

aggccgcctc ggcctctgag ctattccaga agtagtgagg aggctttttt ggaggcctag     780

gcttttgcaa aaagcttggc attccggtac tgttggtaaa gccaccatgg aagacgccaa     840

aaacataaag aaaggcccgg cgccattcta tccgctggaa gatggaaccg ctggagagca     900

actgcataag gctatgaaga gatacgccct ggttcctgga acaattgctt ttacagatgc     960

acatatcgag gtggacatca cttacgctga gtacttcgaa atgtccgttc ggttggcaga    1020

agctatgaaa cgatatgggc tgaatacaaa tcacagaatc gtcgtatgca gtgaaaactc    1080

tcttcaattc tttatgccgg tgttgggcgc gttatttatc ggagttgcag ttgcgcccgc    1140

gaacgacatt tataatgaac gtgaattgct caacagtatg ggcatttcgc agcctaccgt 1200
ggtgttcgtt tccaaaaagg ggttgcaaaa aattttgaac gtgcaaaaaa agctcccaat 1260
catccaaaaa attattatca tggattctaa aacggattac cagggatttc agtcgatgta 1320
cacgttcgtc acatctcatc tacctcccgg ttttaatgaa tacgattttg tgccagagtc 1380
cttcgatagg gacaagacaa ttgcactgat catgaactcc tctggatcta ctggtctgcc 1440
taaaggtgtc gctctgcctc atagaactgc ctgcgtgaga ttctcgcatg ccagagatcc 1500
tatttttggc aatcaaatca ttccggatac tgcgatttta agtgttgttc cattccatca 1560
cggttttgga atgtttacta cactcggata tttgatatgt ggatttcgag tcgtcttaat 1620
gtatagattt gaagaagagc tgtttctgag gagccttcag gattacaaga ttcaaagtgc 1680
gctgctggtg ccaaccctat tctccttctt cgccaaaagc actctgattg acaaatacga 1740
tttatctaat ttacacgaaa ttgcttctgg tggcgctccc ctctctaagg aagtcgggga 1800
agcggttgcc aagaggttcc atctgccagg tatcaggcaa ggatatgggc tcactgagac 1860
tacatcagct attctgatta cacccgaggg ggatgataaa ccgggcgcgg tcggtaaagt 1920
tgttccattt tttgaagcga aggttgtgga tctggatacc gggaaaacgc tgggcgttaa 1980
tcaaagaggc gaactgtgtg tgagaggtcc tatgattatg tccggttatg taaacaatcc 2040
ggaagcgacc aacgccttga ttgacaagga tggatggcta cattctggag acatagctta 2100
ctgggacgaa gacgaacact tcttcatcgt tgaccgcctg aagtctctga ttaagtacaa 2160
aggctatcag gtggctcccg ctgaattgga atccatcttg ctccaacacc ccaacatctt 2220
cgacgcaggt gtcgcaggtc ttcccgacga tgacgccggt gaacttcccg ccgccgttgt 2280
tgttttggag cacggaaaga cgatgacgga aaaagagatc gtggattacg tcgccagtca 2340
agtaacaacc gcgaaaaagt tgcgcggagg agttgtgttt gtggacgaag taccgaaagg 2400
tcttaccgga aaactcgacg caagaaaaat cagagagatc ctcataaagg ccaagaaggg 2460
cggaaagatc gccgtgtaat tctagagtcg gggcggccgg ccgcttcgag cagacatgat 2520
aagatacatt gatgagtttg gacaaaccac aactagaatg cagtgaaaaa aatgctttat 2580
ttgtgaaatt tgtgatgcta ttgctttatt tgtaaccatt ataagctgca ataaacaagt 2640
taacaacaac aattgcattc attttatgtt tcaggttcag ggggaggtgt gggaggtttt 2700
ttaaagcaag taaaacctct acaaatgtgg taaaatcgat aaggatccgt cgaccgatgc 2760
ccttgagagc cttcaaccca gtcagctcct tccggtgggc gcggggcatg actatcgtcg 2820
ccgcacttat gactgtcttc tttatcatgc aactcgtagg acaggtgccg gcagcgctct 2880
tccgcttcct cgctcactga ctcgctgcgc tcggtcgttc ggctgcggcg agcggtatca 2940
gctcactcaa aggcggtaat acggttatcc acagaatcag gggataacgc aggaaagaac 3000

atgtgagcaa aaggccagca aaaggccagg aaccgtaaaa aggccgcgtt gctggcgttt 3060

ttccataggc tccgcccccc tgacgagcat cacaaaaatc gacgctcaag tcagaggtgg 3120

cgaaacccga caggactata aagataccag gcgtttcccc ctggaagctc cctcgtgcgc 3180

tctcctgttc cgaccctgcc gcttaccgga tacctgtccg cctttctccc ttcgggaagc 3240

gtggcgcttt ctcatagctc acgctgtagg tatctcagtt cggtgtaggt cgttcgctcc 3300

aagctgggct gtgtgcacga accccccgtt cagcccgacc gctgcgcctt atccggtaac 3360

tatcgtcttg agtccaaccc ggtaagacac gacttatcgc cactggcagc agccactggt 3420

aacaggatta gcagagcgag gtatgtaggc ggtgctacag agttcttgaa gtggtggcct 3480

aactacggct acactagaag aacagtattt ggtatctgcg ctctgctgaa gccagttacc 3540

ttcggaaaaa gagttggtag ctcttgatcc ggcaaacaaa ccaccgctgg tagcggtggt 3600

ttttttgttt gcaagcagca gattacgcgc agaaaaaaag gatctcaaga agatcctttg 3660

atcttttcta cggggtctga cgctcagtgg aacgaaaact cacgttaagg gattttggtc 3720

atgagattat caaaaaggat cttcacctag atccttttaa attaaaaatg aagttttaaa 3780

tcaatctaaa gtatatatga gtaaacttgg tctgacagtt accaatgctt aatcagtgag 3840

gcacctatct cagcgatctg tctatttcgt tcatccatag ttgcctgact ccccgtcgtg 3900

tagataacta cgatacggga gggcttacca tctggcccca gtgctgcaat gataccgcga 3960

gacccacgct caccggctcc agatttatca gcaataaacc agccagccgg aagggccgag 4020

cgcagaagtg gtcctgcaac tttatccgcc tccatccagt ctattaattg ttgccgggaa 4080

gctagagtaa gtagttcgcc agttaatagt ttgcgcaacg ttgttgccat tgctacaggc 4140

atcgtggtgt cacgctcgtc gtttggtatg gcttcattca gctccggttc ccaacgatca 4200

aggcgagtta catgatcccc catgttgtgc aaaaaagcgg ttagctcctt cggtcctccg 4260

atcgttgtca gaagtaagtt ggccgcagtg ttatcactca tggttatggc agcactgcat 4320

aattctctta ctgtcatgcc atccgtaaga tgcttttctg tgactggtga gtactcaacc 4380

aagtcattct gagaatagtg tatgcggcga ccgagttgct cttgcccggc gtcaatacgg 4440

gataataccg cgccacatag cagaacttta aaagtgctca tcattggaaa acgttcttcg 4500

gggcgaaaac tctcaaggat cttaccgctg ttgagatcca gttcgatgta acccactcgt 4560

gcacccaact gatcttcagc atcttttact ttcaccagcg tttctgggtg agcaaaaaca 4620

ggaaggcaaa atgccgcaaa aaagggaata agggcgacac ggaaatgttg aatactcata 4680

ctcttccttt ttcaatatta ttgaagcatt tatcagggtt attgtctcat gagcggatac 4740

atatttgaat gtatttagaa aaataaacaa ataggggttc cgcgcacatt tccccgaaaa 4800

gtgccacctg acgcgccctg tagcggcgca ttaagcgcgg cgggtgtggt ggttacgcgc 4860

agcgtgaccg ctacacttgc cagcgcccta gcgcccgctc ctttcgcttt cttcccttcc 4920

tttctcgcca cgttcgccgg ctttccccgt caagctctaa atcgggggct ccctttaggg 4980

ttccgattta gtgctttacg gcacctcgac cccaaaaaac ttgattaggg tgatggttca 5040

cgtagtgggc catcgccctg atagacggtt tttcgccctt tgacgttgga gtccacgttc 5100

tttaatagtg gactcttgtt ccaaactgga acaacactca accctatctc ggtctattct 5160

tttgatttat aagggatttt gccgatttcg gcctattggt taaaaaatga gctgatttaa 5220

caaaaattta acgcgaattt taacaaaata ttaacgctta caatttgcca ttcgccattc 5280

aggctgcgca actgttggga agggcgatcg gtgcgggcct cttcgctatt acgccagccc 5340

aagctaccat gataagtaag taatattaag gtacgggagg tacttggagc ggccgcaata 5400

aaatatcttt attttcatta catctgtgtg ttggtttttt gtgtgaatcg atagtactaa 5460

catacgctct ccatcaaaac aaaacgaaac aaaacaaact agcaaaatag gctgtcccca 5520

gtgcaagtgc aggtgccaga acatttctct atcgata 5557

<210> 50
<211> 5385
<212> DNA
<213> artificial

<220>
<223> PGL3 VDRE IR1 nGRE

<400> 50
ggtaccaggt cagagaggtc aaccactaga agacaggtca gagaggtcat gtggaaatca 60

aaggaaagga ttttgttaca ttggccacag aaagtttctc atttcacctt ctagtcttcc 120

atatcctaaa gagtctagag ggactttgtg aattcaatgg ctcaggccaa taaagcaatc 180

tgaacatcat gtatactaca aataagtgag caaagtggac agtcgtcaca gtcatcacaa 240

accacacagc ctatagcgta ttacctggtc actatgccaa attgggacta atcaaaaaga 300

atataaacaa tgaaacaagg ggttgaagca atggttcagc aattaagagc acacaggcac 360

tgcgagctct tacgcgtgct agcccggggc tgagctccag gagagtaggg agatctgcga 420

tctgcatctc aattagtcag caaccatagt cccgccccta actccgccca tcccgcccct 480

aactccgccc agttccgccc attctccgcc ccatcgctga ctaatttttt ttatttatgc 540

agaggccgag gccgcctcgg cctctgagct attccagaag tagtgaggag gcttttttgg 600

aggcctaggc ttttgcaaaa agcttggcat tccggtactg ttggtaaagc caccatggaa 660

gacgccaaaa acataaagaa aggcccggcg ccattctatc cgctggaaga tggaaccgct 720

ggagagcaac tgcataaggc tatgaagaga tacgccctgg ttcctggaac aattgctttt 780

acagatgcac atatcgaggt ggacatcact tacgctgagt acttcgaaat gtccgttcgg 840

ttggcagaag ctatgaaacg atatgggctg aatacaaatc acagaatcgt cgtatgcagt 900

gaaaactctc ttcaattctt tatgccggtg ttgggcgcgt tatttatcgg agttgcagtt 960

gcgcccgcga acgacattta taatgaacgt gaattgctca acagtatggg catttcgcag 1020

cctaccgtgg tgttcgtttc caaaaagggg ttgcaaaaaa ttttgaacgt gcaaaaaaag 1080

ctcccaatca tccaaaaaat tattatcatg gattctaaaa cggattacca gggatttcag 1140

tcgatgtaca cgttcgtcac atctcatcta cctcccggtt ttaatgaata cgattttgtg 1200

ccagagtcct tcgataggga caagacaatt gcactgatca tgaactcctc tggatctact 1260

ggtctgccta aaggtgtcgc tctgcctcat agaactgcct gcgtgagatt ctcgcatgcc 1320

agagatccta tttttggcaa tcaaatcatt ccggatactg cgattttaag tgttgttcca 1380

ttccatcacg gttttggaat gtttactaca ctcggatatt tgatatgtgg atttcgagtc 1440

gtcttaatgt atagatttga agaagagctg tttctgagga gccttcagga ttacaagatt 1500

caaagtgcgc tgctggtgcc aaccctattc tccttcttcg ccaaaagcac tctgattgac 1560

aaatacgatt tatctaattt acacgaaatt gcttctggtg gcgctcccct ctctaaggaa 1620

gtcggggaag cggttgccaa gaggttccat ctgccaggta tcaggcaagg atatgggctc 1680

actgagacta catcagctat tctgattaca cccgaggggg atgataaacc gggcgcggtc 1740

ggtaaagttg ttccattttt tgaagcgaag gttgtggatc tggataccgg gaaaacgctg 1800

ggcgttaatc aaagaggcga actgtgtgtg agaggtccta tgattatgtc cggttatgta 1860

aacaatccgg aagcgaccaa cgccttgatt gacaaggatg gatggctaca ttctggagac 1920

atagcttact gggacgaaga cgaacacttc ttcatcgttg accgcctgaa gtctctgatt 1980

aagtacaaag gctatcaggt ggctcccgct gaattggaat ccatcttgct ccaacacccc 2040

aacatcttcg acgcaggtgt cgcaggtctt cccgacgatg acgccggtga acttcccgcc 2100

gccgttgttg ttttggagca cggaaagacg atgacggaaa aagagatcgt ggattacgtc 2160

gccagtcaag taacaaccgc gaaaaagttg cgcggaggag ttgtgtttgt ggacgaagta 2220

ccgaaaggtc ttaccggaaa actcgacgca agaaaaatca gagagatcct cataaaggcc 2280

aagaagggcg gaaagatcgc cgtgtaattc tagagtcggg gcggccggcc gcttcgagca 2340

gacatgataa gatacattga tgagtttgga caaaccacaa ctagaatgca gtgaaaaaaa 2400

tgctttattt gtgaaatttg tgatgctatt gctttatttg taaccattat aagctgcaat 2460

aaacaagtta acaacaacaa ttgcattcat tttatgtttc aggttcaggg ggaggtgtgg 2520

gaggtttttt aaagcaagta aaacctctac aaatgtggta aaatcgataa ggatccgtcg 2580

accgatgccc ttgagagcct tcaacccagt cagctccttc cggtgggcgc ggggcatgac 2640

tatcgtcgcc gcacttatga ctgtcttctt tatcatgcaa ctcgtaggac aggtgccggc 2700

agcgctcttc cgcttcctcg ctcactgact cgctgcgctc ggtcgttcgg ctgcggcgag 2760

cggtatcagc tcactcaaag gcggtaatac ggttatccac agaatcaggg gataacgcag 2820

gaaagaacat gtgagcaaaa ggccagcaaa aggccaggaa ccgtaaaaag gccgcgttgc 2880

tggcgttttt ccataggctc cgcccccctg acgagcatca caaaaatcga cgctcaagtc 2940

agaggtggcg aaacccgaca ggactataaa gataccaggc gtttccccct ggaagctccc 3000

tcgtgcgctc tcctgttccg accctgccgc ttaccggata cctgtccgcc tttctccctt 3060

cgggaagcgt ggcgctttct catagctcac gctgtaggta tctcagttcg gtgtaggtcg 3120

ttcgctccaa gctgggctgt gtgcacgaac cccccgttca gcccgaccgc tgcgccttat 3180

ccggtaacta tcgtcttgag tccaacccgg taagacacga cttatcgcca ctggcagcag 3240

ccactggtaa caggattagc agagcgaggt atgtaggcgg tgctacagag ttcttgaagt 3300

ggtggcctaa ctacggctac actagaagaa cagtatttgg tatctgcgct ctgctgaagc 3360

cagttacctt cggaaaaaga gttggtagct cttgatccgg caaacaaacc accgctggta 3420

gcggtggttt ttttgtttgc aagcagcaga ttacgcgcag aaaaaaagga tctcaagaag 3480

atcctttgat cttttctacg gggtctgacg ctcagtggaa cgaaaactca cgttaaggga 3540

ttttggtcat gagattatca aaaaggatct tcacctagat ccttttaaat taaaaatgaa 3600

gttttaaatc aatctaaagt atatatgagt aaacttggtc tgacagttac caatgcttaa 3660

tcagtgaggc acctatctca gcgatctgtc tatttcgttc atccatagtt gcctgactcc 3720

ccgtcgtgta gataactacg atacgggagg gcttaccatc tggccccagt gctgcaatga 3780

taccgcgaga cccacgctca ccggctccag atttatcagc aataaaccag ccagccggaa 3840

gggccgagcg cagaagtggt cctgcaactt tatccgcctc catccagtct attaattgtt 3900

gccgggaagc tagagtaagt agttcgccag ttaatagttt gcgcaacgtt gttgccattg 3960

ctacaggcat cgtggtgtca cgctcgtcgt ttggtatggc ttcattcagc tccggttccc 4020

aacgatcaag gcgagttaca tgatccccca tgttgtgcaa aaaagcggtt agctccttcg 4080

gtcctccgat cgttgtcaga agtaagttgg ccgcagtgtt atcactcatg gttatggcag 4140

cactgcataa ttctcttact gtcatgccat ccgtaagatg cttttctgtg actggtgagt 4200

actcaaccaa gtcattctga gaatagtgta tgcggcgacc gagttgctct tgcccggcgt 4260

caatacggga taataccgcg ccacatagca gaactttaaa agtgctcatc attggaaaac 4320

gttcttcggg gcgaaaactc tcaaggatct taccgctgtt gagatccagt tcgatgtaac 4380

ccactcgtgc acccaactga tcttcagcat cttttacttt caccagcgtt tctgggtgag 4440

caaaaacagg aaggcaaaat gccgcaaaaa agggaataag ggcgacacgg aaatgttgaa 4500

tactcatact cttccttttt caatattatt gaagcattta tcagggttat tgtctcatga 4560

gcggatacat atttgaatgt atttagaaaa ataaacaaat aggggttccg cgcacatttc 4620

cccgaaaagt gccacctgac gcgccctgta gcggcgcatt aagcgcggcg ggtgtggtgg 4680

ttacgcgcag cgtgaccgct acacttgcca gcgccctagc gcccgctcct ttcgctttct 4740

tcccttcctt tctcgccacg ttcgccggct ttccccgtca agctctaaat cgggggctcc 4800

ctttagggtt ccgatttagt gctttacggc acctcgaccc caaaaaactt gattagggtg 4860

atggttcacg tagtgggcca tcgccctgat agacggtttt tcgccctttg acgttggagt 4920

ccacgttctt taatagtgga ctcttgttcc aaactggaac aacactcaac cctatctcgg 4980

tctattcttt tgatttataa gggattttgc cgatttcggc ctattggtta aaaaatgagc 5040

tgatttaaca aaaatttaac gcgaatttta acaaaatatt aacgcttaca atttgccatt 5100

cgccattcag gctgcgcaac tgttgggaag ggcgatcggt gcgggcctct tcgctattac 5160

gccagcccaa gctaccatga taagtaagta atattaaggt acgggaggta cttggagcgg 5220

ccgcaataaa atatctttat tttcattaca tctgtgtgtt ggttttttgt gtgaatcgat 5280

agtactaaca tacgctctcc atcaaaacaa aacgaaacaa aacaaactag caaaataggc 5340

tgtccccagt gcaagtgcag gtgccagaac atttctctat cgata 5385

<210> 51
<211> 5384
<212> DNA
<213> artificial

<220>
<223> PGL3(VDRE) IR0 nGRE

<400> 51
ggtaccaggt cagagaggtc aaccactaga agacaggtca gagaggtcat gtggaaatca 60

aaggaaagga ttttgttaca ttggccacag aaagtttctc atttcacctt ctagtcttcc 120

atatcctaaa gagtctagag ggactttgtg aattcaatgg ctcaggccaa taaagcaatc 180

tgaacatcat gtatactaca aataagtgag caaagtggac agtcgtcaca gtcatcacaa 240

accacacagc ctatagcgta ttacctggtc actatgccaa attgggacta atcaaaaaga 300

atataaacaa tgaaacaagg ggttgaagca atggttcagc aattaagagc acacaggcac 360

tgcgagctct tacgcgtgct agcccggggc tgagctccgg agagtaggga gatctgcgat 420

ctgcatctca attagtcagc aaccatagtc ccgcccctaa ctccgcccat cccgccccta 480

actccgccca gttccgccca ttctccgccc catcgctgac taattttttt tatttatgca 540

gaggccgagg ccgcctcggc ctctgagcta ttccagaagt agtgaggagg cttttttgga 600

ggcctaggct tttgcaaaaa gcttggcatt ccggtactgt tggtaaagcc accatggaag 660

acgccaaaaa cataaagaaa ggcccggcgc cattctatcc gctggaagat ggaaccgctg 720

gagagcaact gcataaggct atgaagagat acgccctggt tcctggaaca attgctttta 780

cagatgcaca tatcgaggtg gacatcactt acgctgagta cttcgaaatg tccgttcggt 840

tggcagaagc tatgaaacga tatgggctga atacaaatca cagaatcgtc gtatgcagtg 900

aaaactctct tcaattcttt atgccggtgt tgggcgcgtt atttatcgga gttgcagttg 960

cgcccgcgaa cgacatttat aatgaacgtg aattgctcaa cagtatgggc atttcgcagc 1020

ctaccgtggt gttcgtttcc aaaaaggggt tgcaaaaaat tttgaacgtg caaaaaaagc 1080

tcccaatcat ccaaaaaatt attatcatgg attctaaaac ggattaccag ggatttcagt 1140

cgatgtacac gttcgtcaca tctcatctac ctcccggttt taatgaatac gattttgtgc 1200

cagagtcctt cgatagggac aagacaattg cactgatcat gaactcctct ggatctactg 1260

gtctgcctaa aggtgtcgct ctgcctcata gaactgcctg cgtgagattc tcgcatgcca 1320

gagatcctat ttttggcaat caaatcattc cggatactgc gattttaagt gttgttccat 1380

tccatcacgg ttttggaatg tttactacac tcggatattt gatatgtgga tttcgagtcg 1440

tcttaatgta tagatttgaa gaagagctgt ttctgaggag ccttcaggat tacaagattc 1500

aaagtgcgct gctggtgcca accctattct ccttcttcgc caaaagcact ctgattgaca 1560

aatacgattt atctaattta cacgaaattg cttctggtgg cgctcccctc tctaaggaag 1620

tcggggaagc ggttgccaag aggttccatc tgccaggtat caggcaagga tatgggctca 1680

ctgagactac atcagctatt ctgattacac ccgaggggga tgataaaccg ggcgcggtcg 1740

gtaaagttgt tccatttttt gaagcgaagg ttgtggatct ggataccggg aaaacgctgg 1800

gcgttaatca aagaggcgaa ctgtgtgtga gaggtcctat gattatgtcc ggttatgtaa 1860

acaatccgga agcgaccaac gccttgattg acaaggatgg atggctacat tctggagaca 1920

tagcttactg ggacgaagac gaacacttct tcatcgttga ccgcctgaag tctctgatta 1980

agtacaaagg ctatcaggtg gctcccgctg aattggaatc catcttgctc caacacccca 2040

acatcttcga cgcaggtgtc gcaggtcttc ccgacgatga cgccggtgaa cttcccgccg 2100

ccgttgttgt tttggagcac ggaaagacga tgacggaaaa agagatcgtg gattacgtcg 2160

ccagtcaagt aacaaccgcg aaaaagttgc gcggaggagt tgtgtttgtg gacgaagtac 2220

cgaaaggtct taccggaaaa ctcgacgcaa gaaaaatcag agagatcctc ataaaggcca 2280

agaagggcgg aaagatcgcc gtgtaattct agagtcgggg cggccggccg cttcgagcag 2340

acatgataag atacattgat gagtttggac aaaccacaac tagaatgcag tgaaaaaaat 2400

gctttatttg tgaaatttgt gatgctattg ctttatttgt aaccattata agctgcaata 2460

aacaagttaa caacaacaat tgcattcatt ttatgtttca ggttcagggg gaggtgtggg 2520

aggtttttta aagcaagtaa aacctctaca aatgtggtaa aatcgataag gatccgtcga 2580

ccgatgccct tgagagcctt caacccagtc agctccttcc ggtgggcgcg gggcatgact 2640

atcgtcgccg cacttatgac tgtcttcttt atcatgcaac tcgtaggaca ggtgccggca 2700

gcgctcttcc gcttcctcgc tcactgactc gctgcgctcg gtcgttcggc tgcggcgagc 2760

ggtatcagct cactcaaagg cggtaatacg gttatccaca gaatcagggg ataacgcagg 2820

aaagaacatg tgagcaaaag gccagcaaaa ggccaggaac cgtaaaaagg ccgcgttgct 2880

ggcgtttttc cataggctcc gcccccctga cgagcatcac aaaaatcgac gctcaagtca 2940

gaggtggcga aacccgacag gactataaag ataccaggcg tttccccctg gaagctccct 3000

cgtgcgctct cctgttccga ccctgccgct taccggatac ctgtccgcct ttctcccttc 3060

gggaagcgtg gcgctttctc atagctcacg ctgtaggtat ctcagttcgg tgtaggtcgt 3120

tcgctccaag ctgggctgtg tgcacgaacc ccccgttcag cccgaccgct gcgccttatc 3180

cggtaactat cgtcttgagt ccaacccggt aagacacgac ttatcgccac tggcagcagc 3240

cactggtaac aggattagca gagcgaggta tgtaggcggt gctacagagt tcttgaagtg 3300

gtggcctaac tacggctaca ctagaagaac agtatttggt atctgcgctc tgctgaagcc 3360

agttaccttc ggaaaaagag ttggtagctc ttgatccggc aaacaaacca ccgctggtag 3420

cggtggtttt tttgtttgca agcagcagat tacgcgcaga aaaaaaggat ctcaagaaga 3480

tcctttgatc ttttctacgg ggtctgacgc tcagtggaac gaaaactcac gttaagggat 3540

tttggtcatg agattatcaa aaaggatctt cacctagatc cttttaaatt aaaaatgaag 3600

ttttaaatca atctaaagta tatatgagta aacttggtct gacagttacc aatgcttaat 3660

cagtgaggca cctatctcag cgatctgtct atttcgttca tccatagttg cctgactccc 3720

cgtcgtgtag ataactacga tacgggaggg cttaccatct ggccccagtg ctgcaatgat 3780

accgcgagac ccacgctcac cggctccaga tttatcagca ataaaccagc cagccggaag 3840

ggccgagcgc agaagtggtc ctgcaacttt atccgcctcc atccagtcta ttaattgttg 3900

ccgggaagct agagtaagta gttcgccagt taatagtttg cgcaacgttg ttgccattgc 3960

tacaggcatc gtggtgtcac gctcgtcgtt tggtatggct tcattcagct ccggttccca 4020

acgatcaagg cgagttacat gatcccccat gttgtgcaaa aaagcggtta gctccttcgg 4080

tcctccgatc gttgtcagaa gtaagttggc cgcagtgtta tcactcatgg ttatggcagc 4140

actgcataat tctcttactg tcatgccatc cgtaagatgc ttttctgtga ctggtgagta 4200

ctcaaccaag tcattctgag aatagtgtat gcggcgaccg agttgctctt gcccggcgtc 4260

aatacgggat aataccgcgc cacatagcag aactttaaaa gtgctcatca ttggaaaacg 4320

ttcttcgggg cgaaaactct caaggatctt accgctgttg agatccagtt cgatgtaacc 4380

cactcgtgca cccaactgat cttcagcatc ttttactttc accagcgttt ctgggtgagc 4440

aaaaacagga aggcaaaatg ccgcaaaaaa gggaataagg gcgacacgga aatgttgaat 4500

actcatactc ttcctttttc aatattattg aagcatttat cagggttatt gtctcatgag 4560

cggatacata tttgaatgta tttagaaaaa taaacaaata ggggttccgc gcacatttcc 4620

ccgaaaagtg ccacctgacg cgccctgtag cggcgcatta agcgcggcgg gtgtggtggt 4680

tacgcgcagc gtgaccgcta cacttgccag cgccctagcg cccgctcctt tcgctttctt 4740

cccttccttt ctcgccacgt tcgccggctt tccccgtcaa gctctaaatc gggggctccc 4800

tttagggttc cgatttagtg ctttacggca cctcgacccc aaaaaacttg attagggtga 4860

tggttcacgt agtgggccat cgccctgata gacggttttt cgccctttga cgttggagtc 4920

cacgttcttt aatagtggac tcttgttcca aactggaaca acactcaacc ctatctcggt 4980

ctattctttt gatttataag ggattttgcc gatttcggcc tattggttaa aaaatgagct 5040

gatttaacaa aaatttaacg cgaattttaa caaaatatta acgcttacaa tttgccattc 5100

gccattcagg ctgcgcaact gttgggaagg gcgatcggtg cgggcctctt cgctattacg 5160

ccagcccaag ctaccatgat aagtaagtaa tattaaggta cgggaggtac ttggagcggc 5220

cgcaataaaa tatctttatt ttcattacat ctgtgtgttg gttttttgtg tgaatcgata 5280

gtactaacat acgctctcca tcaaaacaaa acgaaacaaa acaaactagc aaaataggct 5340

gtccccagtg caagtgcagg tgccagaaca tttctctatc gata 5384

<210> 52
<211> 5386
<212> DNA
<213> artificial

<220>
<223> PGL3(VDRE) IR2 nGRE

<400> 52
ggtaccaggt cagagaggtc aaccactaga agacaggtca gagaggtcat gtggaaatca 60

aaggaaagga ttttgttaca ttggccacag aaagtttctc atttcacctt ctagtcttcc 120

atatcctaaa gagtctagag ggactttgtg aattcaatgg ctcaggccaa taaagcaatc 180

tgaacatcat gtatactaca aataagtgag caaagtggac agtcgtcaca gtcatcacaa 240

accacacagc ctatagcgta ttacctggtc actatgccaa attgggacta atcaaaaaga 300

atataaacaa tgaaacaagg ggttgaagca atggttcagc aattaagagc acacaggcac 360

tgcgagctct tacgcgtgct agcccggggc tgagctccac ggagagtagg gagatctgcg 420

atctgcatct caattagtca gcaaccatag tcccgcccct aactccgccc atcccgcccc 480

taactccgcc cagttccgcc cattctccgc cccatcgctg actaattttt tttatttatg 540

cagaggccga ggccgcctcg gcctctgagc tattccagaa gtagtgagga ggcttttttg 600

gaggcctagg cttttgcaaa aagcttggca ttccggtact gttggtaaag ccaccatgga 660

agacgccaaa aacataaaga aaggcccggc gccattctat ccgctggaag atggaaccgc 720

tggagagcaa ctgcataagg ctatgaagag atacgccctg gttcctggaa caattgcttt 780

tacagatgca catatcgagg tggacatcac ttacgctgag tacttcgaaa tgtccgttcg 840

gttggcagaa gctatgaaac gatatgggct gaatacaaat cacagaatcg tcgtatgcag 900

tgaaaactct cttcaattct ttatgccggt gttgggcgcg ttatttatcg gagttgcagt 960

tgcgcccgcg aacgacattt ataatgaacg tgaattgctc aacagtatgg gcatttcgca 1020

gcctaccgtg gtgttcgttt ccaaaaaggg gttgcaaaaa attttgaacg tgcaaaaaaa 1080

gctcccaatc atccaaaaaa ttattatcat ggattctaaa acggattacc agggatttca 1140

gtcgatgtac acgttcgtca catctcatct acctcccggt tttaatgaat acgattttgt 1200

gccagagtcc ttcgataggg acaagacaat tgcactgatc atgaactcct ctggatctac 1260

tggtctgcct aaaggtgtcg ctctgcctca tagaactgcc tgcgtgagat tctcgcatgc 1320

cagagatcct atttttggca atcaaatcat tccggatact gcgattttaa gtgttgttcc 1380

attccatcac ggttttggaa tgtttactac actcggatat ttgatatgtg gatttcgagt 1440

cgtcttaatg tatagatttg aagaagagct gtttctgagg agccttcagg attacaagat 1500

tcaaagtgcg ctgctggtgc caaccctatt ctccttcttc gccaaaagca ctctgattga 1560

caaatacgat ttatctaatt tacacgaaat tgcttctggt ggcgctcccc tctctaagga 1620

agtcggggaa gcggttgcca agaggttcca tctgccaggt atcaggcaag gatatgggct 1680

cactgagact acatcagcta ttctgattac acccgagggg gatgataaac cgggcgcggt 1740

cggtaaagtt gttccatttt ttgaagcgaa ggttgtggat ctggataccg ggaaaacgct 1800

gggcgttaat caaagaggcg aactgtgtgt gagaggtcct atgattatgt ccggttatgt 1860

aaacaatccg gaagcgacca acgccttgat tgacaaggat ggatggctac attctggaga 1920

catagcttac tgggacgaag acgaacactt cttcatcgtt gaccgcctga agtctctgat 1980

taagtacaaa ggctatcagg tggctcccgc tgaattggaa tccatcttgc tccaacaccc 2040

caacatcttc gacgcaggtg tcgcaggtct tcccgacgat gacgccggtg aacttcccgc 2100

cgccgttgtt gttttggagc acggaaagac gatgacggaa aaagagatcg tggattacgt 2160

cgccagtcaa gtaacaaccg cgaaaaagtt gcgcggagga gttgtgtttg tggacgaagt 2220

accgaaaggt cttaccggaa aactcgacgc aagaaaaatc agagagatcc tcataaaggc 2280

caagaagggc ggaaagatcg ccgtgtaatt ctagagtcgg ggcggccggc cgcttcgagc 2340

agacatgata agatacattg atgagtttgg acaaaccaca actagaatgc agtgaaaaaa 2400

atgctttatt tgtgaaattt gtgatgctat tgctttattt gtaaccatta taagctgcaa 2460

taaacaagtt aacaacaaca attgcattca ttttatgttt caggttcagg gggaggtgtg 2520

ggaggttttt taaagcaagt aaaacctcta caaatgtggt aaaatcgata aggatccgtc 2580

gaccgatgcc cttgagagcc ttcaacccag tcagctcctt ccggtgggcg cggggcatga 2640

ctatcgtcgc cgcacttatg actgtcttct ttatcatgca actcgtagga caggtgccgg 2700

cagcgctctt ccgcttcctc gctcactgac tcgctgcgct cggtcgttcg gctgcggcga 2760

gcggtatcag ctcactcaaa ggcggtaata cggttatcca cagaatcagg ggataacgca 2820

ggaaagaaca tgtgagcaaa aggccagcaa aaggccagga accgtaaaaa ggccgcgttg 2880

ctggcgtttt tccataggct ccgcccccct gacgagcatc acaaaaatcg acgctcaagt 2940

cagaggtggc gaaacccgac aggactataa agataccagg cgtttccccc tggaagctcc 3000

ctcgtgcgct ctcctgttcc gaccctgccg cttaccggat acctgtccgc ctttctccct 3060

tcgggaagcg tggcgctttc tcatagctca cgctgtaggt atctcagttc ggtgtaggtc 3120

gttcgctcca agctgggctg tgtgcacgaa ccccccgttc agcccgaccg ctgcgcctta 3180

tccggtaact atcgtcttga gtccaacccg gtaagacacg acttatcgcc actggcagca 3240

gccactggta acaggattag cagagcgagg tatgtaggcg gtgctacaga gttcttgaag 3300

tggtggccta actacggcta cactagaaga acagtatttg gtatctgcgc tctgctgaag 3360

ccagttacct tcggaaaaag agttggtagc tcttgatccg gcaaacaaac caccgctggt 3420

agcggtggtt tttttgtttg caagcagcag attacgcgca gaaaaaaagg atctcaagaa 3480

gatcctttga tcttttctac ggggtctgac gctcagtgga acgaaaactc acgttaaggg 3540

attttggtca tgagattatc aaaaaggatc ttcacctaga tccttttaaa ttaaaaatga 3600

agttttaaat caatctaaag tatatatgag taaacttggt ctgacagtta ccaatgctta 3660

atcagtgagg cacctatctc agcgatctgt ctatttcgtt catccatagt tgcctgactc 3720

cccgtcgtgt agataactac gatacgggag ggcttaccat ctggccccag tgctgcaatg 3780

ataccgcgag acccacgctc accggctcca gatttatcag caataaacca gccagccgga 3840

agggccgagc gcagaagtgg tcctgcaact ttatccgcct ccatccagtc tattaattgt 3900

tgccgggaag ctagagtaag tagttcgcca gttaatagtt tgcgcaacgt tgttgccatt 3960

gctacaggca tcgtggtgtc acgctcgtcg tttggtatgg cttcattcag ctccggttcc 4020

caacgatcaa ggcgagttac atgatccccc atgttgtgca aaaaagcggt tagctccttc 4080

ggtcctccga tcgttgtcag aagtaagttg gccgcagtgt tatcactcat ggttatggca 4140

gcactgcata attctcttac tgtcatgcca tccgtaagat gcttttctgt gactggtgag 4200

tactcaacca agtcattctg agaatagtgt atgcggcgac cgagttgctc ttgcccggcg 4260

tcaatacggg ataataccgc gccacatagc agaactttaa aagtgctcat cattggaaaa 4320

cgttcttcgg ggcgaaaact ctcaaggatc ttaccgctgt tgagatccag ttcgatgtaa 4380

cccactcgtg cacccaactg atcttcagca tcttttactt tcaccagcgt ttctgggtga 4440

gcaaaaacag gaaggcaaaa tgccgcaaaa aagggaataa gggcgacacg gaaatgttga 4500

atactcatac tcttcctttt tcaatattat tgaagcattt atcagggtta ttgtctcatg 4560

agcggataca tatttgaatg tatttagaaa aataaacaaa taggggttcc gcgcacattt 4620

ccccgaaaag tgccacctga cgcgccctgt agcggcgcat taagcgcggc gggtgtggtg 4680

gttacgcgca gcgtgaccgc tacacttgcc agcgccctag cgcccgctcc tttcgctttc 4740

ttcccttcct ttctcgccac gttcgccggc tttccccgtc aagctctaaa tcgggggctc 4800

cctttagggt tccgatttag tgctttacgg cacctcgacc ccaaaaaact tgattagggt 4860

gatggttcac gtagtgggcc atcgccctga tagacggttt ttcgcccttt gacgttggag 4920

tccacgttct ttaatagtgg actcttgttc caaactggaa caacactcaa ccctatctcg 4980

gtctattctt ttgatttata agggattttg ccgatttcgg cctattggtt aaaaaatgag 5040

ctgatttaac aaaaatttaa cgcgaatttt aacaaaatat taacgcttac aatttgccat 5100

tcgccattca ggctgcgcaa ctgttgggaa gggcgatcgg tgcgggcctc ttcgctatta 5160

cgccagccca agctaccatg ataagtaagt aatattaagg tacgggaggt acttggagcg 5220

gccgcaataa aatatcttta ttttcattac atctgtgtgt tggttttttg tgtgaatcga 5280

tagtactaac atacgctctc catcaaaaca aaacgaaaca aaacaaacta gcaaaatagg 5340

ctgtccccag tgcaagtgca ggtgccagaa catttctcta tcgata 5386

<210> 53
<211> 5562
<212> DNA
<213> artificial

<220>
<223> PGL3(SV40/VDRE) (+) GRE

<400> 53
ggtaccctgt ggaatgtgtg tcagttaggg tgtggaaagt ccccaggctc cccagcaggc 60

agaagtatgc aaagcatgca tctcaattag tcagcaacca ggtgtggaaa gtccccaggc 120

tccccagcag gcagaagtat gcaaagcatg catctcaatt agtcagcaac caggtaccag 180

gtcagagagg tcaaccacta gaagacaggt cagagaggtc atgtggaaat caaaggaaag 240

gattttgtta cattggccac agaaagtttc tcatttcacc ttctagtctt ccatatccta 300

aagagtctag agggactttg tgaattcaat ggctcaggcc aataaagcaa tctgaacatc 360

atgtatacta caaataagtg agcaaagtgg acagtcgtca cagtcatcac aaaccacaca 420

gcctatagcg tattacctgg tcactatgcc aaattgggac taatcaaaaa gaatataaac 480

aatgaaacaa ggggttgaag caatggttca gcaattaaga gcacacaggc actgcgagct 540

cttacgcgtg ctagcccggg agaggatctg tacaggatgt tctagataga tctgcgatct 600

gcatctcaat tagtcagcaa ccatagtccc gcccctaact ccgcccatcc cgcccctaac 660

tccgcccagt tccgcccatt ctccgcccca tcgctgacta atttttttta tttatgcaga 720

ggccgaggcc gcctcggcct ctgagctatt ccagaagtag tgaggaggct tttttggagg 780

cctaggcttt tgcaaaaagc ttggcattcc ggtactgttg gtaaagccac catggaagac 840

gccaaaaaca taaagaaagg cccggcgcca ttctatccgc tggaagatgg aaccgctgga 900

gagcaactgc ataaggctat gaagagatac gccctggttc ctggaacaat tgcttttaca 960

gatgcacata tcgaggtgga catcacttac gctgagtact tcgaaatgtc cgttcggttg 1020

gcagaagcta tgaaacgata tgggctgaat acaaatcaca gaatcgtcgt atgcagtgaa 1080

aactctcttc aattctttat gccggtgttg ggcgcgttat ttatcggagt tgcagttgcg 1140

cccgcgaacg acatttataa tgaacgtgaa ttgctcaaca gtatgggcat ttcgcagcct 1200

accgtggtgt tcgtttccaa aaaggggttg caaaaaattt tgaacgtgca aaaaaagctc 1260

ccaatcatcc aaaaaattat tatcatggat tctaaaacgg attaccaggg atttcagtcg 1320

atgtacacgt tcgtcacatc tcatctacct cccggtttta atgaatacga ttttgtgcca 1380

gagtccttcg atagggacaa gacaattgca ctgatcatga actcctctgg atctactggt 1440

ctgcctaaag gtgtcgctct gcctcataga actgcctgcg tgagattctc gcatgccaga 1500

gatcctattt ttggcaatca aatcattccg gatactgcga ttttaagtgt tgttccattc 1560

catcacggtt ttggaatgtt tactacactc ggatatttga tatgtggatt tcgagtcgtc 1620

ttaatgtata gatttgaaga agagctgttt ctgaggagcc ttcaggatta caagattcaa 1680

agtgcgctgc tggtgccaac cctattctcc ttcttcgcca aaagcactct gattgacaaa 1740

tacgatttat ctaatttaca cgaaattgct tctggtggcg ctcccctctc taaggaagtc 1800

ggggaagcgg ttgccaagag gttccatctg ccaggtatca ggcaaggata tgggctcact 1860

gagactacat cagctattct gattacaccc gagggggatg ataaaccggg cgcggtcggt 1920

aaagttgttc cattttttga agcgaaggtt gtggatctgg ataccgggaa aacgctgggc 1980

gttaatcaaa gaggcgaact gtgtgtgaga ggtcctatga ttatgtccgg ttatgtaaac 2040

aatccggaag cgaccaacgc cttgattgac aaggatggat ggctacattc tggagacata 2100

gcttactggg acgaagacga acacttcttc atcgttgacc gcctgaagtc tctgattaag 2160

tacaaaggct atcaggtggc tcccgctgaa ttggaatcca tcttgctcca acaccccaac 2220

atcttcgacg caggtgtcgc aggtcttccc gacgatgacg ccggtgaact tcccgccgcc 2280

gttgttgttt tggagcacgg aaagacgatg acggaaaaag agatcgtgga ttacgtcgcc 2340

agtcaagtaa caaccgcgaa aaagttgcgc ggaggagttg tgtttgtgga cgaagtaccg 2400

aaaggtctta ccggaaaact cgacgcaaga aaaatcagag agatcctcat aaaggccaag 2460

aagggcggaa agatcgccgt gtaattctag agtcggggcg gccggccgct tcgagcagac 2520

atgataagat acattgatga gtttggacaa accacaacta gaatgcagtg aaaaaaatgc 2580

tttatttgtg aaatttgtga tgctattgct ttatttgtaa ccattataag ctgcaataaa 2640

caagttaaca acaacaattg cattcatttt atgtttcagg ttcaggggga ggtgtgggag 2700

gttttttaaa gcaagtaaaa cctctacaaa tgtggtaaaa tcgataagga tccgtcgacc 2760

gatgcccttg agagccttca acccagtcag ctccttccgg tgggcgcggg gcatgactat 2820

cgtcgccgca cttatgactg tcttctttat catgcaactc gtaggacagg tgccggcagc 2880

gctcttccgc ttcctcgctc actgactcgc tgcgctcggt cgttcggctg cggcgagcgg 2940

tatcagctca ctcaaaggcg gtaatacggt tatccacaga atcaggggat aacgcaggaa 3000

agaacatgtg agcaaaaggc cagcaaaagg ccaggaaccg taaaaaggcc gcgttgctgg 3060

cgtttttcca taggctccgc ccccctgacg agcatcacaa aaatcgacgc tcaagtcaga 3120

ggtggcgaaa cccgacagga ctataaagat accaggcgtt tccccctgga agctccctcg 3180

tgcgctctcc tgttccgacc ctgccgctta ccggatacct gtccgccttt ctcccttcgg 3240

gaagcgtggc gctttctcat agctcacgct gtaggtatct cagttcggtg taggtcgttc 3300

gctccaagct gggctgtgtg cacgaacccc ccgttcagcc cgaccgctgc gccttatccg 3360

gtaactatcg tcttgagtcc aacccggtaa gacacgactt atcgccactg gcagcagcca 3420

ctggtaacag gattagcaga gcgaggtatg taggcggtgc tacagagttc ttgaagtggt 3480

ggcctaacta cggctacact agaagaacag tatttggtat ctgcgctctg ctgaagccag 3540

ttaccttcgg aaaaagagtt ggtagctctt gatccggcaa acaaaccacc gctggtagcg 3600

gtggtttttt tgtttgcaag cagcagatta cgcgcagaaa aaaaggatct caagaagatc 3660

ctttgatctt ttctacgggg tctgacgctc agtggaacga aaactcacgt taagggattt 3720

tggtcatgag attatcaaaa aggatcttca cctagatcct tttaaattaa aaatgaagtt 3780

ttaaatcaat ctaaagtata tatgagtaaa cttggtctga cagttaccaa tgcttaatca 3840

gtgaggcacc tatctcagcg atctgtctat ttcgttcatc catagttgcc tgactccccg 3900

tcgtgtagat aactacgata cgggagggct taccatctgg ccccagtgct gcaatgatac 3960

cgcgagaccc acgctcaccg gctccagatt tatcagcaat aaaccagcca gccggaaggg 4020

ccgagcgcag aagtggtcct gcaactttat ccgcctccat ccagtctatt aattgttgcc 4080

gggaagctag agtaagtagt tcgccagtta atagtttgcg caacgttgtt gccattgcta 4140

caggcatcgt ggtgtcacgc tcgtcgtttg gtatggcttc attcagctcc ggttcccaac 4200

gatcaaggcg agttacatga tcccccatgt tgtgcaaaaa agcggttagc tccttcggtc 4260

ctccgatcgt tgtcagaagt aagttggccg cagtgttatc actcatggtt atggcagcac 4320

tgcataattc tcttactgtc atgccatccg taagatgctt ttctgtgact ggtgagtact 4380

caaccaagtc attctgagaa tagtgtatgc ggcgaccgag ttgctcttgc ccggcgtcaa 4440

tacgggataa taccgcgcca catagcagaa ctttaaaagt gctcatcatt ggaaaacgtt 4500

cttcggggcg aaaactctca aggatcttac cgctgttgag atccagttcg atgtaaccca 4560

ctcgtgcacc caactgatct tcagcatctt ttactttcac cagcgtttct gggtgagcaa 4620

aaacaggaag gcaaaatgcc gcaaaaaagg gaataagggc gacacggaaa tgttgaatac 4680

tcatactctt cctttttcaa tattattgaa gcatttatca gggttattgt ctcatgagcg 4740

gatacatatt tgaatgtatt tagaaaaata aacaaatagg ggttccgcgc acatttcccc 4800

gaaaagtgcc acctgacgcg ccctgtagcg gcgcattaag cgcggcgggt gtggtggtta 4860

cgcgcagcgt gaccgctaca cttgccagcg ccctagcgcc cgctcctttc gctttcttcc 4920

cttcctttct cgccacgttc gccggctttc cccgtcaagc tctaaatcgg gggctccctt 4980

tagggttccg atttagtgct ttacggcacc tcgaccccaa aaaacttgat tagggtgatg 5040

gttcacgtag tgggccatcg ccctgataga cggtttttcg ccctttgacg ttggagtcca 5100

cgttctttaa tagtggactc ttgttccaaa ctggaacaac actcaaccct atctcggtct 5160

attcttttga tttataaggg attttgccga tttcggccta ttggttaaaa aatgagctga 5220

tttaacaaaa atttaacgcg aattttaaca aaatattaac gcttacaatt tgccattcgc 5280

cattcaggct gcgcaactgt tgggaagggc gatcggtgcg ggcctcttcg ctattacgcc 5340

agcccaagct accatgataa gtaagtaata ttaaggtacg ggaggtactt ggagcggccg 5400

caataaaata tctttatttt cattacatct gtgtgttggt tttttgtgtg aatcgatagt 5460

actaacatac gctctccatc aaaacaaaac gaaacaaaac aaactagcaa aataggctgt 5520

ccccagtgca agtgcaggtg ccagaacatt tctctatcga ta 5562

<210>  54
<211>  5390
<212>  DNA
<213>  artificial

<220>
<223>  PGL3 VDRE (+) GRE

<400>  54
ggtaccaggt cagagaggtc aaccactaga agacaggtca gagaggtcat gtggaaatca      60

aaggaaagga ttttgttaca ttggccacag aaagtttctc atttcacctt ctagtcttcc     120

atatcctaaa gagtctagag ggactttgtg aattcaatgg ctcaggccaa taaagcaatc     180

tgaacatcat gtatactaca aataagtgag caaagtggac agtcgtcaca gtcatcacaa     240

accacacagc ctatagcgta ttacctggtc actatgccaa attgggacta atcaaaaaga     300

atataaacaa tgaaacaagg ggttgaagca atggttcagc aattaagagc acacaggcac     360

tgcgagctct tacgcgtgct agcccgggag aggatctgta caggatgttc tagatagatc     420

tgcgatctgc atctcaatta gtcagcaacc atagtcccgc ccctaactcc gcccatcccg     480

cccctaactc cgcccagttc cgcccattct ccgccccatc gctgactaat tttttttatt     540

tatgcagagg ccgaggccgc ctcggcctct gagctattcc agaagtagtg aggaggcttt     600

tttggaggcc taggcttttg caaaaagctt ggcattccgg tactgttggt aaagccacca     660

tggaagacgc caaaaacata aagaaaggcc cggcgccatt ctatccgctg gaagatggaa     720

ccgctggaga gcaactgcat aaggctatga agagatacgc cctggttcct ggaacaattg     780

cttttacaga tgcacatatc gaggtggaca tcacttacgc tgagtacttc gaaatgtccg     840

ttcggttggc agaagctatg aaacgatatg ggctgaatac aaatcacaga atcgtcgtat     900

gcagtgaaaa ctctcttcaa ttctttatgc cggtgttggg cgcgttattt atcggagttg     960

cagttgcgcc cgcgaacgac atttataatg aacgtgaatt gctcaacagt atgggcattt    1020

cgcagcctac cgtggtgttc gtttccaaaa aggggttgca aaaaattttg aacgtgcaaa    1080

aaaagctccc aatcatccaa aaaattatta tcatggattc taaaacggat taccagggat    1140

ttcagtcgat gtacacgttc gtcacatctc atctacctcc cggttttaat gaatacgatt    1200

ttgtgccaga gtccttcgat agggacaaga caattgcact gatcatgaac tcctctggat    1260

ctactggtct gcctaaaggt gtcgctctgc ctcatagaac tgcctgcgtg agattctcgc    1320

atgccagaga tcctattttt ggcaatcaaa tcattccgga tactgcgatt ttaagtgttg    1380

ttccattcca tcacggtttt ggaatgttta ctacactcgg atatttgata tgtggatttc    1440

gagtcgtctt aatgtataga tttgaagaag agctgtttct gaggagcctt caggattaca    1500

agattcaaag tgcgctgctg gtgccaaccc tattctcctt cttcgccaaa agcactctga    1560

ttgacaaata cgatttatct aatttacacg aaattgcttc tggtggcgct cccctctcta 1620

aggaagtcgg ggaagcggtt gccaagaggt tccatctgcc aggtatcagg caaggatatg 1680

ggctcactga gactacatca gctattctga ttacacccga gggggatgat aaaccgggcg 1740

cggtcggtaa agttgttcca ttttttgaag cgaaggttgt ggatctggat accgggaaaa 1800

cgctgggcgt taatcaaaga ggcgaactgt gtgtgagagg tcctatgatt atgtccggtt 1860

atgtaaacaa tccggaagcg accaacgcct tgattgacaa ggatggatgg ctacattctg 1920

gagacatagc ttactgggac gaagacgaac acttcttcat cgttgaccgc ctgaagtctc 1980

tgattaagta caaaggctat caggtggctc ccgctgaatt ggaatccatc ttgctccaac 2040

accccaacat cttcgacgca ggtgtcgcag gtcttcccga cgatgacgcc ggtgaacttc 2100

ccgccgccgt tgttgttttg gagcacggaa agacgatgac ggaaaaagag atcgtggatt 2160

acgtcgccag tcaagtaaca accgcgaaaa agttgcgcgg aggagttgtg tttgtggacg 2220

aagtaccgaa aggtcttacc ggaaaactcg acgcaagaaa aatcagagag atcctcataa 2280

aggccaagaa gggcggaaag atcgccgtgt aattctagag tcggggcggc cggccgcttc 2340

gagcagacat gataagatac attgatgagt ttggacaaac cacaactaga atgcagtgaa 2400

aaaaatgctt tatttgtgaa atttgtgatg ctattgcttt atttgtaacc attataagct 2460

gcaataaaca agttaacaac aacaattgca ttcattttat gtttcaggtt cagggggagg 2520

tgtgggaggt tttttaaagc aagtaaaacc tctacaaatg tggtaaaatc gataaggatc 2580

cgtcgaccga tgcccttgag agccttcaac ccagtcagct ccttccggtg ggcgcggggc 2640

atgactatcg tcgccgcact tatgactgtc ttctttatca tgcaactcgt aggacaggtg 2700

ccggcagcgc tcttccgctt cctcgctcac tgactcgctg cgctcggtcg ttcggctgcg 2760

gcgagcggta tcagctcact caaaggcggt aatacggtta tccacagaat caggggataa 2820

cgcaggaaag aacatgtgag caaaaggcca gcaaaaggcc aggaaccgta aaaaggccgc 2880

gttgctggcg tttttccata ggctccgccc ccctgacgag catcacaaaa atcgacgctc 2940

aagtcagagg tggcgaaacc cgacaggact ataaagatac caggcgtttc cccctggaag 3000

ctccctcgtg cgctctcctg ttccgaccct gccgcttacc ggatacctgt ccgcctttct 3060

cccttcggga agcgtggcgc tttctcatag ctcacgctgt aggtatctca gttcggtgta 3120

ggtcgttcgc tccaagctgg gctgtgtgca cgaacccccc gttcagcccg accgctgcgc 3180

cttatccggt aactatcgtc ttgagtccaa cccggtaaga cacgacttat cgccactggc 3240

agcagccact ggtaacagga ttagcagagc gaggtatgta ggcggtgcta cagagttctt 3300

gaagtggtgg cctaactacg gctacactag aagaacagta tttggtatct gcgctctgct 3360

gaagccagtt accttcggaa aaagagttgg tagctcttga tccggcaaac aaaccaccgc 3420

tggtagcggt ggtttttttg tttgcaagca gcagattacg cgcagaaaaa aaggatctca 3480

agaagatcct ttgatctttt ctacggggtc tgacgctcag tggaacgaaa actcacgtta 3540

agggattttg gtcatgagat tatcaaaaag gatcttcacc tagatccttt taaattaaaa 3600

atgaagtttt aaatcaatct aaagtatata tgagtaaact tggtctgaca gttaccaatg 3660

cttaatcagt gaggcaccta tctcagcgat ctgtctattt cgttcatcca tagttgcctg 3720

actccccgtc gtgtagataa ctacgatacg ggagggctta ccatctggcc ccagtgctgc 3780

aatgataccg cgagacccac gctcaccggc tccagattta tcagcaataa accagccagc 3840

cggaagggcc gagcgcagaa gtggtcctgc aactttatcc gcctccatcc agtctattaa 3900

ttgttgccgg gaagctagag taagtagttc gccagttaat agtttgcgca acgttgttgc 3960

cattgctaca ggcatcgtgg tgtcacgctc gtcgtttggt atggcttcat tcagctccgg 4020

ttcccaacga tcaaggcgag ttacatgatc ccccatgttg tgcaaaaaag cggttagctc 4080

cttcggtcct ccgatcgttg tcagaagtaa gttggccgca gtgttatcac tcatggttat 4140

ggcagcactg cataattctc ttactgtcat gccatccgta agatgctttt ctgtgactgg 4200

tgagtactca accaagtcat tctgagaata gtgtatgcgg cgaccgagtt gctcttgccc 4260

ggcgtcaata cgggataata ccgcgccaca tagcagaact ttaaaagtgc tcatcattgg 4320

aaaacgttct tcggggcgaa aactctcaag gatcttaccg ctgttgagat ccagttcgat 4380

gtaacccact cgtgcaccca actgatcttc agcatctttt actttcacca gcgtttctgg 4440

gtgagcaaaa acaggaaggc aaaatgccgc aaaaaaggga ataagggcga cacggaaatg 4500

ttgaatactc atactcttcc tttttcaata ttattgaagc atttatcagg gttattgtct 4560

catgagcgga tacatatttg aatgtattta gaaaaataaa caaatagggg ttccgcgcac 4620

atttccccga aaagtgccac ctgacgcgcc ctgtagcggc gcattaagcg cggcgggtgt 4680

ggtggttacg cgcagcgtga ccgctacact tgccagcgcc ctagcgcccg ctcctttcgc 4740

tttcttccct tcctttctcg ccacgttcgc cggctttccc cgtcaagctc taaatcgggg 4800

gctcccttta gggttccgat ttagtgcttt acggcacctc gaccccaaaa aacttgatta 4860

gggtgatggt tcacgtagtg ggccatcgcc ctgatagacg gtttttcgcc ctttgacgtt 4920

ggagtccacg ttctttaata gtggactctt gttccaaact ggaacaacac tcaaccctat 4980

ctcggtctat tcttttgatt tataagggat tttgccgatt tcggcctatt ggttaaaaaa 5040

tgagctgatt taacaaaaat ttaacgcgaa ttttaacaaa atattaacgc ttacaatttg 5100

ccattcgcca ttcaggctgc gcaactgttg ggaagggcga tcggtgcggg cctcttcgct 5160

attacgccag cccaagctac catgataagt aagtaatatt aaggtacggg aggtacttgg 5220

agcggccgca ataaaatatc tttattttca ttacatctgt gtgttggttt tttgtgtgaa 5280

tcgatagtac taacatacgc tctccatcaa aacaaaacga aacaaaacaa actagcaaaa 5340

taggctgtcc ccagtgcaag tgcaggtgcc agaacatttc tctatcgata 5390

<210> 55
<211> 6036
<212> DNA
<213> artificial

<220>
<223> PGL4 vector AP1

<400> 55
   ggcctaactg gccggtacct gagctcgcta gctgagtcag tgagtcactg actcactgac 60
   tcatgagtca gagatctggc ctcggcggcc aagcttagac actagagggt atataatgga 120
   agctcgactt ccagcttggc aatccggtac tgttggtaaa gccaccatgg aagatgccaa 180
   aaacattaag aagggcccag cgccattcta cccactcgaa gacgggaccg ccggcgagca 240
   gctgcacaaa gccatgaagc gctacgccct ggtgcccggc accatcgcct ttaccgacgc 300
   acatatcgag gtggacatta cctacgccga gtacttcgag atgagcgttc ggctggcaga 360
   agctatgaag cgctatgggc tgaatacaaa ccatcggatc gtggtgtgca gcgagaatag 420
   cttgcagttc ttcatgcccg tgttgggtgc cctgttcatc ggtgtggctg tggccccagc 480
   taacgacatc tacaacgagc gcgagctgct gaacagcatg ggcatcagcc agcccaccgt 540
   cgtattcgtg agcaagaaag ggctgcaaaa gatcctcaac gtgcaaaaga agctaccgat 600
   catacaaaag atcatcatca tggatagcaa gaccgactac cagggcttcc aaagcatgta 660
   caccttcgtg acttcccatt tgccacccgg cttcaacgag tacgacttcg tgcccgagag 720
   cttcgaccgg gacaaaacca tcgccctgat catgaacagt agtggcagta ccggattgcc 780
   caagggcgta gccctaccgc accgcaccgc ttgtgtccga ttcagtcatg cccgcgaccc 840
   catcttcggc aaccagatca tccccgacac cgctatcctc agcgtggtgc catttcacca 900
   cggcttcggc atgttcacca cgctgggcta cttgatctgc ggctttcggg tcgtgctcat 960
   gtaccgcttc gaggaggagc tattcttgcg cagcttgcaa gactataaga ttcaatctgc 1020
   cctgctggtg cccacactat ttagcttctt cgctaagagc actctcatcg acaagtacga 1080
   cctaagcaac ttgcacgaga tcgccagcgg cggggcgccg ctcagcaagg aggtaggtga 1140
   ggccgtggcc aaacgcttcc acctaccagg catccgccag ggctacggcc tgacagaaac 1200
   aaccagcgcc attctgatca cccccgaagg ggacgacaag cctggcgcag taggcaaggt 1260
   ggtgcccttc ttcgaggcta aggtggtgga cttggacacc ggtaagacac tgggtgtgaa 1320
   ccagcgcggc gagctgtgcg tccgtggccc catgatcatg agcggctacg ttaacaaccc 1380
   cgaggctaca aacgctctca tcgacaagga cggctggctg cacagcggcg acatcgccta 1440
   ctgggacgag gacgagcact tcttcatcgt ggaccggctg aagagcctga tcaaatacaa 1500
   gggctaccag gtagccccag ccgaactgga gagcatcctg ctgcaacacc ccaacatctt 1560
   cgacgccggg gtcgccggcc tgcccgacga cgatgccggc gagctgcccg ccgcagtcgt 1620
   cgtgctggaa cacggtaaaa ccatgaccga gaaggagatc gtggactatg tggccagcca 1680
   ggttacaacc gccaagaagc tgcgcggtgg tgttgtgttc gtggacgagg tgcctaaagg 1740
   actgaccggc aagttggacg cccgcaagat ccgcgagatt ctcattaagg ccaagaaggg 1800
   cggcaagatc gccgtgaatt ctcacggctt ccctcccgag gtggaggagc aggccgccgg 1860
   caccctgccc atgagctgcg cccaggagag cggcatggat agacaccctg ctgcttgcgc 1920
   cagcgccagg atcaacgtct aaggccgcga ctctagagtc ggggcggccg gccgcttcga 1980
   gcagacatga taagatacat tgatgagttt ggacaaacca caactagaat gcagtgaaaa 2040
   aaatgcttta tttgtgaaat ttgtgatgct attgctttat ttgtaaccat tataagctgc 2100
   aataaacaag ttaacaacaa caattgcatt cattttatgt ttcaggttca gggggaggtg 2160
   tgggaggttt tttaaagcaa gtaaaacctc tacaaatgtg gtaaaatcga taaggatccg 2220
   tttgcgtatt gggcgctctt ccgctgatct gcgcagcacc atggcctgaa ataacctctg 2280
   aaagaggaac ttggttagct accttctgag gcggaaagaa ccagctgtgg aatgtgtgtc 2340
   agttagggtg tggaaagtcc ccaggctccc cagcaggcag aagtatgcaa agcatgcatc 2400
   tcaattagtc agcaaccagg tgtggaaagt ccccaggctc cccagcaggc agaagtatgc 2460
   aaagcatgca tctcaattag tcagcaacca tagtcccgcc cctaactccg cccatcccgc 2520
   ccctaactcc gcccagttcc gcccattctc cgccccatgg ctgactaatt ttttttattt 2580
   atgcagaggc cgaggccgcc tctgcctctg agctattcca gaagtagtga ggaggctttt 2640
   ttggaggcct aggcttttgc aaaaagctcg attcttctga cactagcgcc accatgaaga 2700
   agcccgaact caccgctacc agcgttgaaa aatttctcat cgagaagttc gacagtgtga 2760
   gcgacctgat gcagttgtcg gagggcgaag agagccgagc cttcagcttc gatgtcggcg 2820
   gacgcggcta tgtactgcgg gtgaatagct gcgctgatgg cttctacaaa gaccgctacg 2880
   tgtaccgcca cttcgccagc gctgcactac ccatccccga agtgttggac atcggcgagt 2940
   tcagcgagag cctgacatac tgcatcagta gacgcgccca aggcgttact ctccaagacc 3000
   tccccgaaac agagctgcct gctgtgttac agcctgtcgc cgaagctatg gatgctattg 3060
   ccgccgccga cctcagtcaa accagcggct tcggcccatt cgggccccaa ggcatcggcc 3120
   agtacacaac ctggcgggat ttcatttgcg ccattgctga tccccatgtc taccactggc 3180
   agaccgtgat ggacgacacc gtgtccgcca gcgtagctca agccctggac gaactgatgc 3240
   tgtgggccga agactgtccc gaggtgcgcc acctcgtcca tgccgacttc ggcagcaaca 3300
   acgtcctgac cgacaacggc cgcatcaccg ccgtaatcga ctggtccgaa gctatgttcg 3360
   gggacagtca gtacgaggtg gccaacatct tcttctggcg gccctggctg gcttgcatgg 3420
   agcagcagac tcgctacttc gagcgccggc atcccgagct ggccggcagc cctcgtctgc 3480
   gagcctacat gctgcgcatc ggcctggatc agctctacca gagcctcgtg gacggcaact 3540
   tcgacgatgc tgcctgggct caaggccgct gcgatgccat cgtccgcagc ggggccggca 3600
   ccgtcggtcg cacacaaatc gctcgccgga gcgcagccgt atggaccgac ggctgcgtcg 3660
   aggtgctggc cgacagcggc aaccgccggc ccagtacacg accgcgcgct aaggaggtag 3720
   gtcgagttta aactctagaa ccggtcatgg ccgcaataaa atatctttat tttcattaca 3780
   tctgtgtgtt ggttttttgt gtgttcgaac tagatgctgt cgaccgatgc ccttgagagc 3840
   cttcaaccca gtcagctcct tccggtgggc gcggggcatg actatcgtcg ccgcacttat 3900
   gactgtcttc tttatcatgc aactcgtagg acaggtgccg gcagcgctct tccgcttcct 3960
   cgctcactga ctcgctgcgc tcggtcgttc ggctgcggcg agcggtatca gctcactcaa 4020
   aggcggtaat acggttatcc acagaatcag gggataacgc aggaaagaac atgtgagcaa 4080
   aaggccagca aaaggccagg aaccgtaaaa aggccgcgtt gctggcgttt ttccataggc 4140
   tccgcccccc tgacgagcat cacaaaaatc gacgctcaag tcagaggtgg cgaaacccga 4200
   caggactata aagataccag gcgtttcccc ctggaagctc cctcgtgcgc tctcctgttc 4260
   cgaccctgcc gcttaccgga tacctgtccg cctttctccc ttcgggaagc gtggcgcttt 4320
   ctcatagctc acgctgtagg tatctcagtt cggtgtaggt cgttcgctcc aagctgggct 4380
   gtgtgcacga accccccgtt cagcccgacc gctgcgcctt atccggtaac tatcgtcttg 4440
   agtccaaccc ggtaagacac gacttatcgc cactggcagc agccactggt aacaggatta 4500
   gcagagcgag gtatgtaggc ggtgctacag agttcttgaa gtggtggcct aactacggct 4560
   acactagaag aacagtattt ggtatctgcg ctctgctgaa gccagttacc ttcggaaaaa 4620
   gagttggtag ctcttgatcc ggcaaacaaa ccaccgctgg tagcggtggt ttttttgttt 4680
   gcaagcagca gattacgcgc agaaaaaaag gatctcaaga agatcctttg atcttttcta 4740
   cggggtctga cgctcagtgg aacgaaaact cacgttaagg gattttggtc atgagattat 4800
   caaaaaggat cttcacctag atccttttaa attaaaaatg aagttttaaa tcaatctaaa 4860
   gtatatatga gtaaacttgg tctgacagcg gccgcaaatg ctaaaccact gcagtggtta 4920
   ccagtgcttg atcagtgagg caccgatctc agcgatctgc ctatttcgtt cgtccatagt 4980
   ggcctgactc cccgtcgtgt agatcactac gattcgtgag ggcttaccat caggccccag 5040
   cgcagcaatg atgccgcgag agccgcgttc accggccccc gatttgtcag caatgaacca 5100
   gccagcaggg agggccgagc gaagaagtgg tcctgctact ttgtccgcct ccatccagtc 5160
   tatgagctgc tgtcgtgatg ctagagtaag aagttcgcca gtgagtagtt tccgaagagt 5220
   tgtggccatt gctactggca tcgtggtatc acgctcgtcg ttcggtatgg cttcgttcaa 5280
   ctctggttcc cagcggtcaa gccgggtcac atgatcaccc atattatgaa gaaatgcagt 5340
   cagctcctta gggcctccga tcgttgtcag aagtaagttg gccgcggtgt tgtcgctcat 5400
   ggtaatggca gcactacaca attctcttac cgtcatgcca tccgtaagat gcttttccgt 5460
   gaccggcgag tactcaacca agtcgttttg tgagtagtgt atacggcgac caagctgctc 5520
   ttgcccggcg tctatacggg acaacaccgc gccacatagc agtactttga aagtgctcat 5580
   catcgggaat cgttcttcgg ggcggaaaga ctcaaggatc ttgccgctat tgagatccag 5640
   ttcgatatag cccactcttg cacccagttg atcttcagca tcttttactt tcaccagcgt 5700
   ttcggggtgt gcaaaaacag gcaagcaaaa tgccgcaaag aagggaatga gtgcgacacg 5760
   aaaatgttgg atgctcatac tcgtcctttt tcaatattat tgaagcattt atcagggtta 5820
   ctagtacgtc tctcaaggat aagtaagtaa tattaaggta cgggaggtat tggacaggcc 5880
   gcaataaaat atctttattt tcattacatc tgtgtgttgg ttttttgtgt gaatcgatag 5940
   tactaacata cgctctccat caaaacaaaa cgaaacaaaa caaactagca aaataggctg 6000
   tccccagtgc aagtgcaggt gccagaacat ttctct 6036

<210> 56
<211> 6059
<212> DNA
<213> artificial

<220>
<223> PGL4 vector NFKB

<400> 56
ggcctaactg gccggtacct gagctcgcta gcgggaattt ccggggactt tccgggaatt 60

tccggggact ttccgggaat ttccgggaat ttccagatct ggcctcggcg gccaagctta 120

gacactagag ggtatataat ggaagctcga cttccagctt ggcaatccgg tactgttggt 180

aaagccacca tggaagatgc caaaaacatt aagaagggcc cagcgccatt ctacccactc 240

gaagacggga ccgccggcga gcagctgcac aaagccatga agcgctacgc cctggtgccc 300

ggcaccatcg cctttaccga cgcacatatc gaggtggaca ttacctacgc cgagtacttc 360

gagatgagcg ttcggctggc agaagctatg aagcgctatg ggctgaatac aaaccatcgg 420

atcgtggtgt gcagcgagaa tagcttgcag ttcttcatgc ccgtgttggg tgccctgttc 480

atcggtgtgg ctgtggcccc agctaacgac atctacaacg agcgcgagct gctgaacagc 540

atgggcatca gccagcccac cgtcgtattc gtgagcaaga aagggctgca aaagatcctc 600

aacgtgcaaa agaagctacc gatcatacaa aagatcatca tcatggatag caagaccgac 660

taccagggct tccaaagcat gtacaccttc gtgacttccc atttgccacc cggcttcaac 720

gagtacgact tcgtgcccga gagcttcgac cgggacaaaa ccatcgccct gatcatgaac 780

agtagtggca gtaccggatt gcccaagggc gtagccctac cgcaccgcac cgcttgtgtc 840

cgattcagtc atgcccgcga ccccatcttc ggcaaccaga tcatccccga caccgctatc 900

ctcagcgtgg tgccatttca ccacggcttc ggcatgttca ccacgctggg ctacttgatc 960

tgcggctttc gggtcgtgct catgtaccgc ttcgaggagg agctattctt gcgcagcttg 1020

caagactata agattcaatc tgccctgctg gtgcccacac tatttagctt cttcgctaag 1080

agcactctca tcgacaagta cgacctaagc aacttgcacg agatcgccag cggcggggcg 1140

ccgctcagca aggaggtagg tgaggccgtg gccaaacgct tccacctacc aggcatccgc 1200

cagggctacg gcctgacaga aacaaccagc gccattctga tcacccccga aggggacgac 1260

aagcctggcg cagtaggcaa ggtggtgccc ttcttcgagg ctaaggtggt ggacttggac 1320

accggtaaga cactgggtgt gaaccagcgc ggcgagctgt gcgtccgtgg ccccatgatc 1380

atgagcggct acgttaacaa ccccgaggct acaaacgctc tcatcgacaa ggacggctgg 1440

ctgcacagcg gcgacatcgc ctactgggac gaggacgagc acttcttcat cgtggaccgg 1500

ctgaagagcc tgatcaaata caagggctac caggtagccc cagccgaact ggagagcatc 1560

ctgctgcaac accccaacat cttcgacgcc ggggtcgccg gcctgcccga cgacgatgcc 1620

ggcgagctgc ccgccgcagt cgtcgtgctg gaacacggta aaaccatgac cgagaaggag 1680

atcgtggact atgtggccag ccaggttaca accgccaaga agctgcgcgg tggtgttgtg 1740

ttcgtggacg aggtgcctaa aggactgacc ggcaagttgg acgcccgcaa gatccgcgag 1800

attctcatta aggccaagaa gggcggcaag atcgccgtga attctcacgg cttccctccc 1860

gaggtggagg agcaggccgc cggcaccctg cccatgagct gcgcccagga gagcggcatg 1920

gatagacacc ctgctgcttg cgccagcgcc aggatcaacg tctaaggccg cgactctaga 1980

gtcggggcgg ccggccgctt cgagcagaca tgataagata cattgatgag tttggacaaa 2040

ccacaactag aatgcagtga aaaaaatgct ttatttgtga aatttgtgat gctattgctt 2100

tatttgtaac cattataagc tgcaataaac aagttaacaa caacaattgc attcatttta 2160

tgtttcaggt tcagggggag gtgtgggagg ttttttaaag caagtaaaac ctctacaaat 2220

gtggtaaaat cgataaggat ccgtttgcgt attgggcgct cttccgctga tctgcgcagc 2280

accatggcct gaaataacct ctgaaagagg aacttggtta gctaccttct gaggcggaaa 2340

gaaccagctg tggaatgtgt gtcagttagg gtgtggaaag tccccaggct ccccagcagg 2400

cagaagtatg caaagcatgc atctcaatta gtcagcaacc aggtgtggaa agtccccagg 2460

ctccccagca ggcagaagta tgcaaagcat gcatctcaat tagtcagcaa ccatagtccc 2520

gcccctaact ccgcccatcc cgcccctaac tccgcccagt tccgcccatt ctccgcccca 2580

tggctgacta atttttttta tttatgcaga ggccgaggcc gcctctgcct ctgagctatt 2640

ccagaagtag tgaggaggct tttttggagg cctaggcttt tgcaaaaagc tcgattcttc 2700

tgacactagc gccaccatga agaagcccga actcaccgct accagcgttg aaaaatttct 2760

catcgagaag ttcgacagtg tgagcgacct gatgcagttg tcggagggcg aagagagccg 2820

agccttcagc ttcgatgtcg gcggacgcgg ctatgtactg cgggtgaata gctgcgctga 2880

tggcttctac aaagaccgct acgtgtaccg ccacttcgcc agcgctgcac tacccatccc 2940

cgaagtgttg gacatcggcg agttcagcga gagcctgaca tactgcatca gtagacgcgc 3000

ccaaggcgtt actctccaag acctccccga aacagagctg cctgctgtgt tacagcctgt 3060

cgccgaagct atggatgcta ttgccgccgc cgacctcagt caaaccagcg gcttcggccc 3120

attcgggccc caaggcatcg gccagtacac aacctggcgg gatttcattt gcgccattgc 3180

tgatccccat gtctaccact ggcagaccgt gatggacgac accgtgtccg ccagcgtagc 3240

tcaagccctg gacgaactga tgctgtgggc cgaagactgt cccgaggtgc gccacctcgt 3300

ccatgccgac ttcggcagca acaacgtcct gaccgacaac ggccgcatca ccgccgtaat 3360

cgactggtcc gaagctatgt tcggggacag tcagtacgag gtggccaaca tcttcttctg 3420

gcggccctgg ctggcttgca tggagcagca gactcgctac ttcgagcgcc ggcatcccga 3480

gctggccggc agccctcgtc tgcgagccta catgctgcgc atcggcctgg atcagctcta 3540

ccagagcctc gtggacggca acttcgacga tgctgcctgg gctcaaggcc gctgcgatgc 3600

catcgtccgc agcggggccg gcaccgtcgg tcgcacacaa atcgctcgcc ggagcgcagc 3660

cgtatggacc gacggctgcg tcgaggtgct ggccgacagc ggcaaccgcc ggcccagtac 3720

acgaccgcgc gctaaggagg taggtcgagt ttaaactcta gaaccggtca tggccgcaat 3780

aaaatatctt tattttcatt acatctgtgt gttggttttt tgtgtgttcg aactagatgc 3840

tgtcgaccga tgcccttgag agccttcaac ccagtcagct ccttccggtg ggcgcggggc 3900

atgactatcg tcgccgcact tatgactgtc ttctttatca tgcaactcgt aggacaggtg 3960

ccggcagcgc tcttccgctt cctcgctcac tgactcgctg cgctcggtcg ttcggctgcg 4020

gcgagcggta tcagctcact caaaggcggt aatacggtta tccacagaat caggggataa 4080

cgcaggaaag aacatgtgag caaaaggcca gcaaaaggcc aggaaccgta aaaaggccgc 4140

gttgctggcg tttttccata ggctccgccc ccctgacgag catcacaaaa atcgacgctc 4200

aagtcagagg tggcgaaacc cgacaggact ataaagatac caggcgtttc cccctggaag 4260

ctccctcgtg cgctctcctg ttccgaccct gccgcttacc ggatacctgt ccgcctttct 4320

cccttcggga agcgtggcgc tttctcatag ctcacgctgt aggtatctca gttcggtgta 4380

ggtcgttcgc tccaagctgg gctgtgtgca cgaacccccc gttcagcccg accgctgcgc 4440

cttatccggt aactatcgtc ttgagtccaa cccggtaaga cacgacttat cgccactggc 4500

agcagccact ggtaacagga ttagcagagc gaggtatgta ggcggtgcta cagagttctt 4560

gaagtggtgg cctaactacg gctacactag aagaacagta tttggtatct gcgctctgct 4620

gaagccagtt accttcggaa aaagagttgg tagctcttga tccggcaaac aaaccaccgc 4680

tggtagcggt ggtttttttg tttgcaagca gcagattacg cgcagaaaaa aaggatctca 4740

agaagatcct ttgatctttt ctacggggtc tgacgctcag tggaacgaaa actcacgtta 4800

agggattttg gtcatgagat tatcaaaaag gatcttcacc tagatccttt taaattaaaa 4860

atgaagtttt aaatcaatct aaagtatata tgagtaaact tggtctgaca gcggccgcaa 4920

atgctaaacc actgcagtgg ttaccagtgc ttgatcagtg aggcaccgat ctcagcgatc 4980

tgcctatttc gttcgtccat agtggcctga ctccccgtcg tgtagatcac tacgattcgt 5040

gagggcttac catcaggccc cagcgcagca atgatgccgc gagagccgcg ttcaccggcc 5100

cccgatttgt cagcaatgaa ccagccagca gggagggccg agcgaagaag tggtcctgct 5160

actttgtccg cctccatcca gtctatgagc tgctgtcgtg atgctagagt aagaagttcg 5220

ccagtgagta gtttccgaag agttgtggcc attgctactg gcatcgtggt atcacgctcg 5280

tcgttcggta tggcttcgtt caactctggt tcccagcggt caagccgggt cacatgatca 5340

cccatattat gaagaaatgc agtcagctcc ttagggcctc cgatcgttgt cagaagtaag 5400

ttggccgcgg tgttgtcgct catggtaatg gcagcactac acaattctct taccgtcatg 5460

ccatccgtaa gatgcttttc cgtgaccggc gagtactcaa ccaagtcgtt ttgtgagtag 5520

tgtatacggc gaccaagctg ctcttgcccg gcgtctatac gggacaacac cgcgccacat 5580

agcagtactt tgaaagtgct catcatcggg aatcgttctt cggggcggaa agactcaagg 5640

atcttgccgc tattgagatc cagttcgata tagcccactc ttgcacccag ttgatcttca 5700

gcatctttta ctttcaccag cgtttcgggg tgtgcaaaaa caggcaagca aaatgccgca 5760

aagaagggaa tgagtgcgac acgaaaatgt tggatgctca tactcgtcct ttttcaatat 5820

tattgaagca tttatcaggg ttactagtac gtctctcaag gataagtaag taatattaag 5880

gtacgggagg tattggacag gccgcaataa aatatcttta ttttcattac atctgtgtgt 5940

tggttttttg tgtgaatcga tagtactaac atacgctctc catcaaaaca aaacgaaaca 6000

aaacaaacta gcaaaatagg ctgtccccag tgcaagtgca ggtgccagaa catttctct    6059

## Description

### BACKGROUND OF THE INVENTION

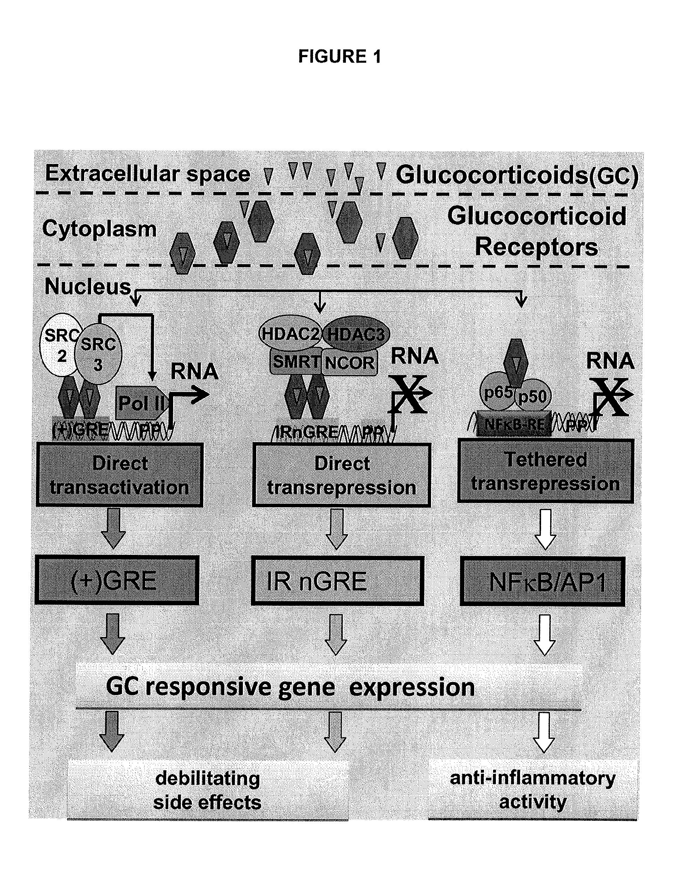

Glucocorticoids (GCs) are peripheral effectors of circadian and stress-related homeostatic functions fundamental for survival throughout vertebrate life span (Chrousos, 2009; Nader et al., 2010). They are widely used to combat inflammatory and allergic disorders and their therapeutic effects have been mainly ascribed to their capacity to suppress the production of proinflammatory cytokines (Rhen and Cidlowski 2005). GCs act by binding to the GC receptor (GR), a member of the nuclear receptor (NR) superfamily. In absence of GCs, GR is maintained in the cytoplasm by molecular chaperones. Binding of GCs generates a conformational switch in the GR ligand binding domain (LBD) which affects GR interactions with chaperones and facilitates nuclear translocation (Ricketson et al., 2007). Once in the nucleus, GR binds to GC response elements (GREs) in the promoter region of the target genes, resulting in the regulation of their transcription (Figure 1).

GREs are generally classified into "simple" GREs and "tethering" GREs. "Simple" GREs belong to a family of imperfect palindromes consisting of two inverted hexameric half-site motifs separated by 3 base pairs (bp) (Meijsing et al., 2009). Agonist-liganded GRs can bind directly to said palindromic DNA binding sites (DBS) for gene expression. On the contrary, "tethering" GREs do not contain DBS for GR *per se,* but instead contain binding sites for other DNA-bound regulators, such as NFκB and AP1, that recruit GR (Karin, 1998). Thus, "tethering" GREs confer "indirect" gene regulation to agonist liganded GR.

Many "simple" GREs have been identified so far. These GREs, also called "(+)GRE" confer direct transcriptional transactivation to agonist-liganded GR through association with co-activators (e.g. SRC1, TIF2/SRC2 and SRC3) (Lonard and O'Malley, 2007). The proteins encoded by these transactivated genes have a wide range of effects including for example regulation of gluconeogenesis.

"Simple" GREs are known to be linked to gene transactivation only. In contrast, "tethering" GREs are commonly associated with "tethered" indirect transrepression of target genes. These target genes are mainly pro-inflammatory genes, including interleukins, chemokines, and cytokines genes.

The anti-inflammatory properties of GCs represent the central target of pharmacological GC therapy. However, the physiological GCs and currently existing GC analogues do not distinguish among transactivation and transrepression, and control both the expression of "wanted" pro-infammatory genes and of "unwanted" genes inducing diabetogenic activity, osteoporosis, as well as skin atrophy. Intensive research has been therefore carried out for discovering selectively acting (so-called dissociated) GCs that will be able to modulate the expression of the desirable genes only.

Glucocorticoids are also involved in glucose metabolism. The metabolic effects of these compounds are at least i) stimulation of gluconeogenesis (in particular, in the liver), ii) mobilization of amino acids from extrahepatic tissues, iii) inhibition of glucose uptake in muscle and adipose tissue, iv) stimulation of fat breakdown in adipose tissue. These effects are often leading to insulin resistance and to diabetes, or result in hypertension. Other effects have been observed, such as inhibition of bone formation, suppression of calcium absorption (both leading to osteoporosis), delayed wound healing, muscle weakness, and/or increased risk of infection.

It has been proposed that debilitating effects of GC treatment are mostly due to (+)GRE-mediated gene transactivation, while GC beneficial anti-inflammatory effects have been ascribed to "tethered" indirect transrepression (Karin, 1998; Kassel and Herrlich, 2007). This hypothesis has consequently led to a search for selective "dissociated" GR ligands which would preferentially generate "tethered" indirect transrepression, and not direct transactivation. Such a ligand, RU24858 (a selective GR agonist with steroid structure), was found to exhibit the expected dissociated profile *in vitro* (Vayssière et al., 1997). However, upon RU24858 administration *in vivo,* pathophysiological studies failed to confirm this dissociation (Belvisi et al., 2001).

The present Inventors have discovered that GCs-mediated transrepression can be mediated not only via the tethering indirect pathway, but also through direct binding of GR to "simple" negative GREs (nGRE), which belongs to a novel family of evolutionary-conserved cis-acting negative response elements (IR nGREs), and are found in numerous GC-repressed genes. Thus, GR agonists that induce indirect transrepression of pro-inflammatory genes, could also generate debilitating side effects *via* direct transrepression. Therefore, previous attempts, aimed at identifying GC analogs exhibiting a "dissociated" profile may have failed, because such GCs had kept their IR nGRE-mediated transrepression activity.

The herein presented results show that "ideal" GR agonists aimed at pharmacological therapy of inflammatory diseases should repress gene expression through "tethered" transrepression, while exhibiting reduced IR nGRE-mediated transrepression and (+)GRE-mediated transactivation activities. Thus, screening processes for identifying anti-inflammatory "dissociated" GCs which are mostly devoid of debilitating side effects should look for compounds that would exhibit decreased (+)GRE-mediated transactivation activity, as well as IR nGRE-mediated direct transrepression activity. The present Invention discloses such screening processes. The present Invention also discloses luciferase reporter plasmids that are useful for such screenings, as well as for characterizing the mode of action of some non-GC-derived compounds that may exhibit some of the beneficial therapeutic activities of "dissociated" GCs, but be mostly devoid of their detrimental effects (De Bosscher and Haegeman, 2009).

### FIGURE LEGENDS

Figure 1 represents the biological mechanisms underlying the process of the present invention, and highlights the three main pathways involved in GR-dependent gene regulation: 1) direct transactivation, 2) direct transrepression, and 3) tethered indirect transrepression.
Figure 2 discloses the PGL4-AP1 vector of SEQ ID NO:55 which can be used in the *in vitro* process of the invention.
Figure 3 discloses the PGL4-NFκB vector of SEQ ID NO:56 which can be used in the *in vitro* process of the invention.
Figure 4 discloses the PGL3(VDRE) IR1 nGRE vector of SEQ ID NO:50, which can be used in the *in vitro* process of the invention.
Figure 5 discloses the PGL3 (VDRE) (+) GRE vector of SEQ ID NO:54 which can be used in the *in vitro* process of the invention.
Figure 6 discloses the sequence from position 1-656 of PGL3 (VDRE) IR0 nGRE vector of SEQ ID NO:51 which can be used in the *in vitro* process of the invention.
Figure 7 discloses the sequence from position 1-658 of PGL3 (VDRE) IR2 nGRE vector of SEQ ID NO:52 which can be used in the *in vitro* process of the invention.
Figure 8 discloses the PGL3 (SV40/VDRE) IR1 nGRE vector of SEQ ID NO:49 which can be used in the *in vitro* process of the invention.
Figure 9 discloses the PGL3 (SV40/VDRE) (+) GRE vector of SEQ ID NO:52 which can be used in the *in vitro* process of the invention.
Figure 10 discloses some of the experimental results obtained by the present Inventors and presented in the experimental part below. The transcript levels of TSLP, CYP26A1 and GPX3 are determined in ear epidermis after a 6 hrs topical treatment with the GR agonist FA, the GR antagonist RU486, the low calcemic Vitamin D3 analog MC903, alone or in combination, as indicated.

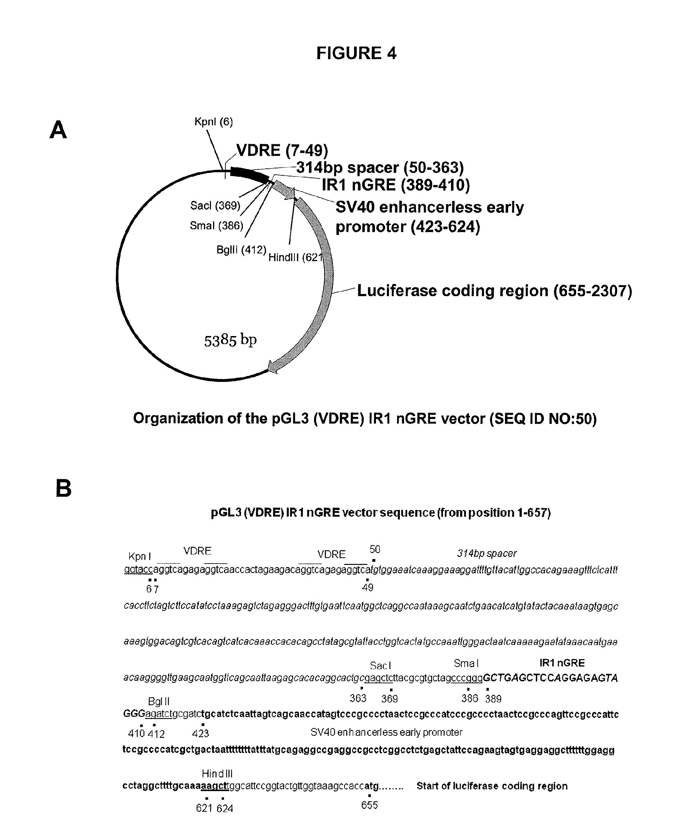

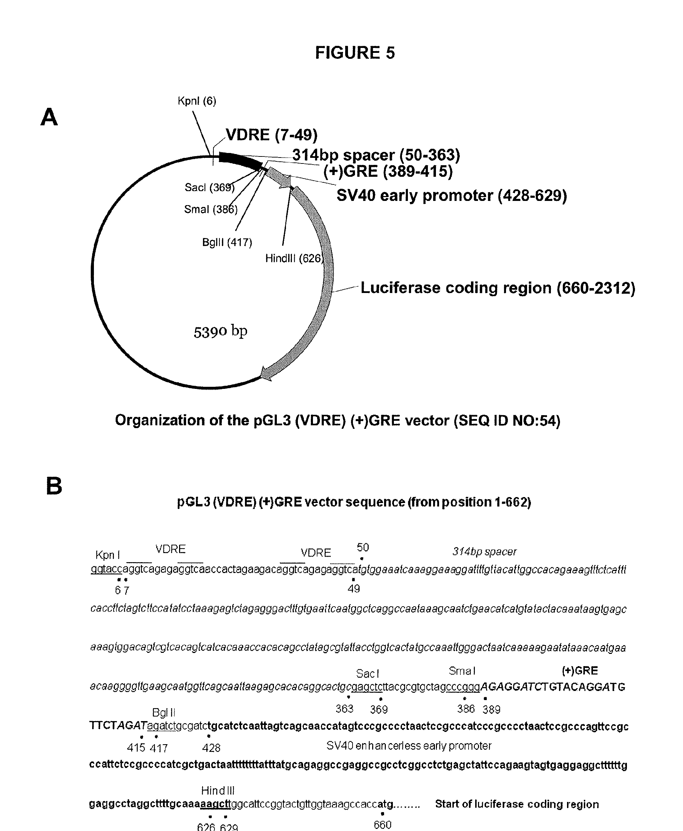

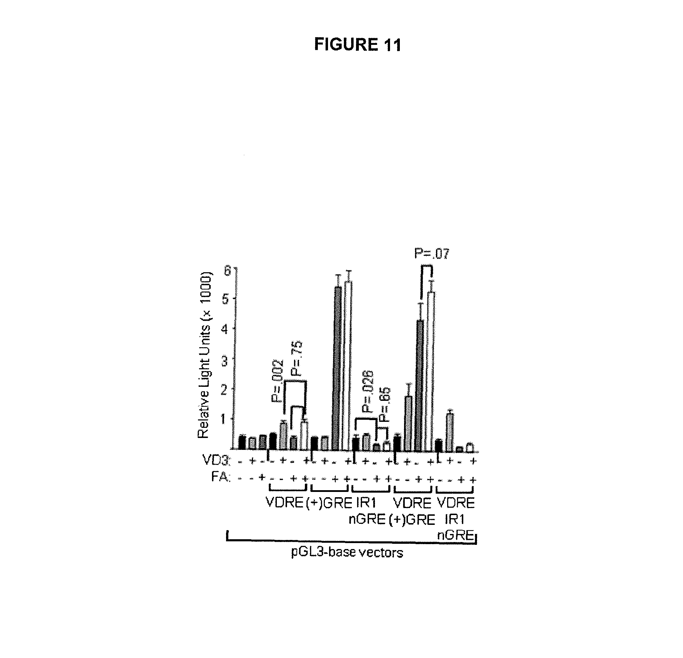

Figure 11 discloses some of the experimental results obtained by the present Inventors and presented in the experimental part below. Luciferase activity in A549 cells transfected with VDRE reporter plasmid (a derivative of PGL3(VDRE) IR nGRE vector in which the IR nGRE element is deleted), PGL3(+)GRE (a derivative of PGL3(VDRE) (+)GRE vector in which the VDRE element is deleted), PGL3 IR1 nGRE (a derivative of PGL3(VDRE) IR nGRE vector in which the VDRE element is deleted), PGL3(VDRE/(+)GRE (of SEQ ID NO 54; Figure 5), PGL3(VDRE/IR1 nGRE (of SEQ ID NO 50; Figure 4) and treated with FA and/or Vitamin D3 (VD3), as indicated.
Figure 12 discloses some of the experimental results obtained by the present Inventors and presented in the experimental part below, showing that IR nGREs of mouse and human orthologues may differ by a "tolerable" one base pair mutation.
   (A) Sequence and position (from the +1 transcription start site) of nGRE motifs present in human (h) and mouse (m) Keratin 5 (K5) gene. Bold letters in low case denote non canonical bases in IR1 nGREs. nGRE regions that assemble a GR and corepressor complex upon Dex-treatment is boxed.
   (B) Sequence and position (from the +1 transcription start site) of nGRE motifs present in human (h) and mouse (m) insulin receptor (insr) and insulin (ins) genes. Bold letters in low case denote non-canonical bases in IR1 nGREs. nGRE regions that assemble a GR and corepressor complex upon Dex-treatment is boxed.
   (C) Sequence and position (from the +1 transcription start site) of nGRE motifs present in human (h) and mouse (m) Reverbα gene. Bold letters in low case denote non canonical bases in IR1 nGREs. nGRE regions that assemble a GR and corepressor complex upon Dex-treatment is boxed.
   (D) ChIP analysis of epidermis, pancreas and liver showing the binding of GR and corepressors to the IR nGRE regions of indicated genes. WT mice were topically-treated with vehicle or Dex (in the case of K5) or IP-injected with vehicle or Dex (in the case of ins, insr and Reverbα) for 18 hours.

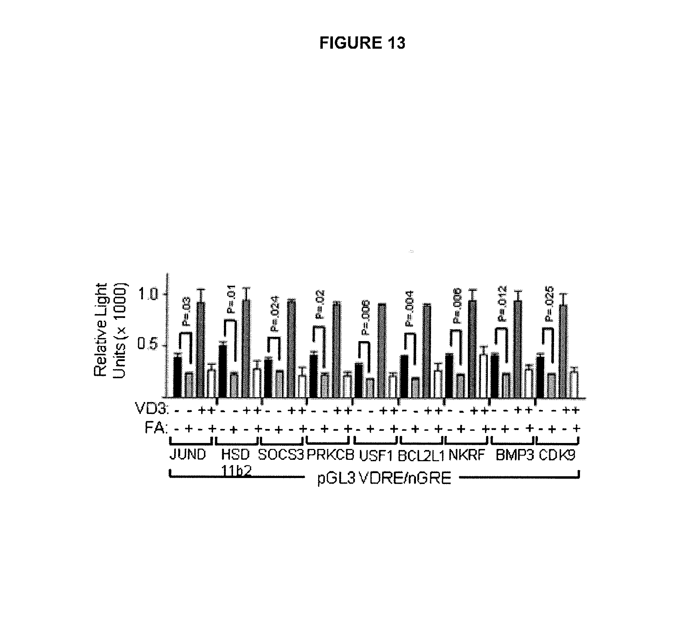

Figure 13 discloses some of the experimental results obtained by the present Inventors and presented in the experimental part below, showing that FA induces repression of reporter genes containing the IR1 nGRE DBS of various genes irrespective of their GC-induced repression *in vivo.* Luciferase assays on A549 cells transfected with various PGL3(VDRE) IR1 nGRE vectors containing the IR1 nGREs present in different genes (Table 4) as indicated, were treated for 6 hours with vehicle, VD3 and/or FA, as indicated.

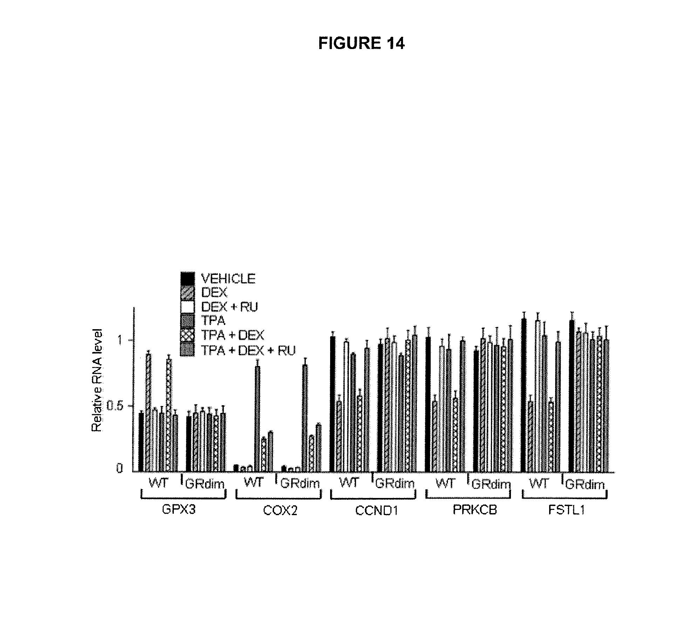

Figure 14 discloses some of the experimental results obtained by the present Inventors and presented in the experimental part below, showing differential effects of GRdim mutation and RU486 treatment on "tethered"- and IR nGRE-mediated transrepression. Gene transcripts in epidermis of WT and GRdim mutant mice topically treated as indicated for 6 hours are analysed by Q-RT-PCR.

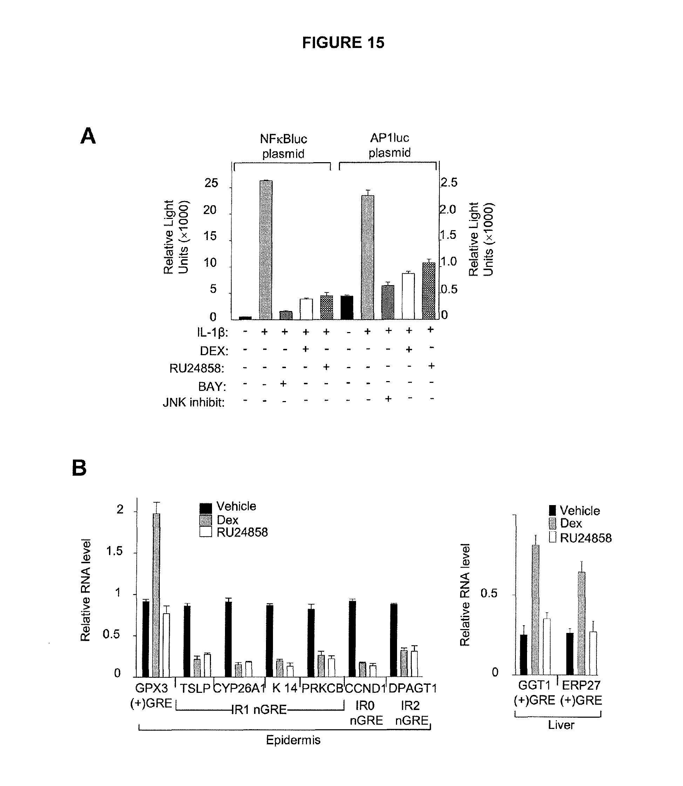

Figure 15 discloses some of the experimental results obtained by the present Inventors and presented in the experimental part below, showing that:
   A) Dexamethasone and RU24858 similarly repress NFκB and AP1-driven transcription *in vitro.* A549 cells transfected with PGL4-NFκB (NFκBluc plasmid) of SEQ ID NO:56 and PGL4-AP1 (AP1luc plasmid) of SEQ ID NO:55 were treated as indicated for 6 hours, followed by luciferase assay. "BAY" = NFκB-specific inhibitor BAY 11-7082 and "JNK inhibit" = JNK inhibitor II).
   B) Q-RT-PCR of gene transcripts from WT dorsal epidermis topically-treated with Dexamethasone or RU24858 for 18 hours (left panel) and from liver of WT mice IP-injected with Dexamethasone or RU24858 for 18 hours (right panel).

### DETAILED DESCRIPTION OF THE INVENTION

The present Inventors have discovered a widespread conserved family of "negative" palindromic GC-response elements (IR nGREs) that mediate transrepression by direct binding of GC-agonist-liganded GR. GC-induced IR nGRE-mediated direct transrepression is distinct from GC-induced "tethered" indirect transrepression, as: (i) "tethering" GREs do not contain DNA binding sites for GR per se, but instead binding sites for other DNA-bound transregulators (e.g. NFκB and AP1) that recruit GR, (ii) the GRdim mutation that does not affect "tethered" transrepression, abolishes IRnGRE-mediated direct transrepression, (iii) co-treatment with RU486 relieves GC-induced IRnGRE-mediated direct transrepression, whereas "tethered" transrepression is not or only slightly affected.

Investigating *in vitro* whether the integrity of the TSLP (Thymic Stromal Lymphopoietin) nGRE in which a 1 bp spacer separates the Inverted Repeated motifs (IR1 nGRE) is essential for its function, has shown that no (IR0 nGRE) or a 2 bp (IR2 nGRE) spacer is "tolerable". Moreover, one base changes at any position of the "canonical" repeated motifs of the TSLP IR1 nGRE and of its IR2 nGRE derivative is, with one exception, functionally "tolerable" *in vitro.* Mouse and human genome-wide analyses revealed the presence of hundreds of mouse and human orthologue genes containing conserved canonical IR0, IR1 and IR2 nGREs (see Table 1, below). In no case are these nGREs located in the near vicinity (<100 bp) of binding sites for regulatory factors (i.e. there is no evidence that IR nGREs are composite sites), and most of them are conserved throughout vertebrates (mammals, chicken and zebra fish, our unpublished data). A number of "IR0", "IR1" and "IR2" nGRE-containing genes expressed in mouse epidermis, intestinal epithelial cells or liver, were analyzed for (i) repression by the GC-agonist Dexamethasone (Dex)-treatment, (ii) prevention of this repression by the GC antagonist RU486 cotreatment, (iii) association of their IR nGREs with agonist liganded-GR and corepressors (ChIP assays), and (iv) repressing activity of their nGREs *in vitro,* which taken all together represent the signature of IR nGRE-mediated transrepression. This analysis demonstrates that IR0, IR1 and IR2 nGRE-containing genes can be efficiently transrepressed by agonist-liganded GRs bound to their nGREs together with corepressors. Assuming that the mouse/human genes that have been randomly selected are representative, the data of the present Inventors indicate that the expression of approximately 600 mouse/human orthologue genes could be negatively controlled through nGRE-mediated transrepression in epidermis, intestinal epithelium and liver. Therefore provided the remaining 400 mouse/human orthologue genes that contain canonical IR nGREs are expressed in other tissues, it is likely that the expression of at least 1000 orthologue genes are negatively controlled in the mouse by GC-induced IR nGRE-mediated transrepression. In fact, the actual number of genes whose expression could be negatively controlled by GC-induced IR nGRE-mediated transrepression might be much higher, as the existence of functionally "tolerable" single base changes in IR nGREs of human and/or mouse orthologue genes could result in underestimating by several hundreds the actual number of genes containing functional IR nGREs.

GCs that act as end-effectors of the HPA (hypothalamus-pituitary-adrenal) axis, are secreted by adrenal glands in a circadian and stress-related manner. They influence the functions of virtually all organs and tissues throughout life span, and are essential for maintenance of their homeostasis and important biological activities, such as intermediary metabolism, immune and inflammatory reactions, as well as circadian clock and stress systems (Chrousos, 2009; Nader et al., 2010). Our ontology search has revealed that a number of IR nGRE-containing genes are involved in such functions. We focus here on a few examples illustrated in the present study.

The data below unequivocally demonstrate that GC-induced IR nGRE-mediated repressions of both the insulin precursor gene (in pancreas β cells) and insulin receptor gene (in liver) are early stress-induced events. This ensures that, upon occurrence of a stress, an elevation of blood glucose level will rapidly follow the surge of GC secretion, thereby providing the increased nutrition of brain, heart and skeletal muscles, required for the central coordination of stress response (Chrousos, 2009). On the other hand, under conditions of chronic stress, GC-induced IR nGRE-mediated repression of the insulin receptor gene may cause insulin resistance and lead to diabetes. This study also reveals the existence of functional IR1 nGREs in ACTH receptor (melacortin 2 receptor, MC2R) gene, and ACTH receptor accessory protein (MRAP) gene, thereby adding, at the adrenal level, another step to the closed negative feedback loop that resets the HPA axis by regulating the synthesis of secreted GCs through GR-mediated repression of CRH and POMC gene expression in hypothalamus and pituitary, respectively (Dostert and Heinzel, 2004). The GC-induced IR nGRE-mediated repression for the Reverbα gene and the RORα gene were also found, both intervening in the control of the circadian timing system, and being likely to play an important role in communications of the molecular Clock and stress systems with intermediary metabolism, which are fundamental for survival (Duez and Staels, 2008; Nader et al., 2010; and Refs therein).

Importantly, it was known from previous studies that the expression of a number of genes is decreased upon GC therapy, but the underlying pathophysiological molecular mechanisms remained often unknown. The data below demonstrate that, upon GC therapy, decreases of keratins 5 and 14 in skin, as well as those in Cyclin D1 and CDK4, are due to GC-induced IR nGRE-mediated repression. Hsd11β2 is another important gene that is repressed by IR1 nGRE-mediated GC-treatment in both skin and colon tissues. The 11β-HSD2 enzyme (encoded in Hsd11β2 gene) is responsible for inactivating glucocorticoids in mineralocorticoid receptor (MR) target tissues (Gross and Cidlowski, 2008). In the absence of this enzyme, GCs (corticosterone in mice) activate MR despite the absence of aldosterone, resulting in hypertension (Stewart et al., 1996). That Hsd11β2 is an IR1 nGRE-containing gene provides a possible molecular explanation for GC therapy-induced hypertension.

Thus, the herein presented results show that a molecule exhibiting, *via* GR-dependent tethered indirect transrepression, anti-inflammatory activities of GC, while significantly lacking their unwanted side effects, would be most useful. Such a molecule should be low in IR nGRE-mediated transrepression and (+)GRE-mediated transactivation activities.

The present Invention discloses screening processes enabling to identify such molecules. These processes can be performed either with cultured GR-containing cells by testing the expression of reporter genes or the expression of genomic genes which are modulated by GC, as well as by testing the expression of GC-modulated genomic genes in tissues of laboratory animals.

In a first aspect, the present invention discloses a process for selecting a molecule exhibiting the anti-inflammatory activities of glucocorticoids (GCs), and having reduced GC-dependent IR nGRE-mediated direct transrepression activity, comprising at least the step of testing if a candidate molecule:
a) significantly transrepresses the transcription of at least one gene containing among its promoter elements NFκB or AP1 DNA binding sequence (DBS),
b) significantly transrepresses the transcription of at least one gene containing among its promoter elements an IR-type negative element (IR nGRE DBS),
c) significantly activates the transcription of at least one gene containing among its promoter elements a (+) GRE DBS,
wherein said IR nGRE DBS is chosen in the group consisting of: sequence SEQ ID NO:1 (IR1 nGRE), SEQ ID NO:2 (IR0 nGRE), SEQ ID NO:3 (IR2 nGRE) and tolerable variants thereof,
wherein said NFκB DBS has the sequence SEQ ID NO: 4, SEQ ID NO:5 or SEQ ID NO:6, or variants thereof,
wherein said AP1 DBS has the sequence SEQ ID NO:7, SEQ ID NO:8 or variants thereof, and wherein said (+) GRE DBS has the sequence SEQ ID NO:9 or SEQ ID NO:10 or variants thereof.

Said candidate molecule will be selected:
- if it significantly transrepresses the transcription of said gene in step a), but it does not significantly transrepress the transcription of said gene in step b), or
- if it significantly transrepresses the transcription of said gene in step a), but does not significantly transrepress the transcription of said gene in step b) and does not significantly increase the transactivation of the transcription of said gene in step c).

More precisely, the present invention targets a process for selecting a molecule exhibiting, via glucocorticoid receptor (GR)-dependent tethered indirect transrepression, the anti-inflammatory activities of GCs, and having reduced GC-dependent IR nGRE-mediated direct transrepression activity, comprising at least the following steps:
i) providing at least one candidate molecule,
ii) testing if said candidate molecule:
   a) significantly induces GR-dependent tethered indirect transrepression of the transcription of at least one gene containing among its promoter elements at least one NFκB or one AP1 DNA binding sequence (DBS),
   b) significantly induces GR-dependent direct transrepression of the transcription of at least one gene containing among its promoter elements an IR-type negative element (IR nGRE DBS),
   c) significantly induces GR-dependent transactivation of the transcription of at least one gene containing among its promoter elements a (+) GRE DBS,
iii) selecting said candidate molecule as a molecule exhibiting anti-inflammatory activities of GCs:
   - if it significantly transrepresses the transcription of said gene in step a), but it does not significantly transrepress the transcription of said gene in step b), or
   - if it significantly transrepresses the transcription of said gene in step a), but does not significantly transrepress the transcription of said gene in step b) and does not significantly increase the transactivation of the transcription of said gene in step c),
   wherein said IR nGRE DBS, NFκB DBS, AP1 DBS and (+) GRE DBS are as defined above.

In a particular embodiment of the invention, the candidate molecule which is selected in step iii) of the process of the invention significantly transrepresses the transcription of said gene in step a), but does not significantly transrepress the transcription of said gene in step b) and does not significantly transactivate the transcription of said gene in step c).

It should be noted that, as used in the present application, the expressions "transactivation of a gene", "transactivation of the transcription of a gene" and "transactivation of the expression of a gene" are equivalent. Similarly, the expressions "transrepression of a gene", "transrepression of the transcription of a gene" and "transrepression of the expression of a gene" are equivalent.

The process of the invention enables to screen any natural or synthetic compound which can be obtained (such compound will be hereafter referred to as a "candidate molecule"). It can be for example a GR ligand, a GC, a GC analogue, as well as any non-GC-derived molecule. GC analogues are defined as synthetic molecules exhibiting some or all of the physiological activities of GCs (Cortisone in humans, and corticosterone in rodents).

As used herein, the term "glucocorticoïd" (GC) relates to a class of steroid hormones that bind to the glucocorticoid receptor (GR). These GC therefore contains a specific arrangement of four cycloalkane rings that are joined to each other. They can be natural or synthetic. Known GCs are for example hydrocortisone (cortisol), prednisone, prednisolone, methylprednisolone, dexamethasone (Dex), fluocinolone acetonide (FA), ,betamethasone, triamcinolone, beclometasone, fludrocortisone, deoxycorticosterone, aldosterone, etc. These compounds are known to mediate effects of two major categories: immunological and metabolic. Through their interaction with the glucocorticoid receptor, they can:
- up-regulate the expression of anti-inflammatory proteins, and
- down-regulate the expression of pro-inflammatory proteins.

They are also shown to play a key role in the development and homeostasis of T lymphocytes (Rhen and Cidlowski 2005).

As used herein, a molecule "exhibits the anti-inflammatory activity of glucocorticoids (GCs)" when it induces similar regulation of the expression of anti-inflammatory proteins, or of pro-inflammatory proteins, as compared with known GCs such as hydrocortisone or dexamethasone. This regulation can be demonstrated in vitro at the protein level or at the mRNA level, by Q-RT-PCR assay. This similar regulation can also be observed in vivo, by the same assays.

As used herein, the term "having reduced GC-dependent IR nGRE-mediated direct transrepression activity" designates a reduction of this repression activity of at least 30%, preferably at least 60%, preferably at least 80% and more preferably 90%, in comparison with the reduction obtained with a glucocorticoid, preferably Dex, at the same dosage.

When used in the present application, the term "similar" means that the two compared phenomena are identical at least at 80%, preferably at least 85%, preferably at least at 90%, and more preferably at least at 95%. For example, two expression levels of a gene will be similar if the ratio of their mRNA levels (and/or their protein levels) is comprised between 0.80 and 1.25, preferably between 0.85 and 1.17, preferably between 0.9 and 1.1, and more preferably between 0.95 and 1.05.

A gene regulation is "glucocorticoid receptor (GR)-dependent" if it requires a functional GR to be effective. The person skilled in the art knows how to measure this property, for example by assessing in GR KO mice if said gene regulation can occur, or by blocking GR with an antagonist.

Genes containing a NFκB or AP1 DNA binding sequence (DBS) among their promoter elements and down-regulated by GC administration, have been already described. For example, such genes can be *COX2, MMP13, IL4,* and *IL10* genes (see Tables 2 and 3, below).

Genes containing a (+)GRE DBS among their promoter elements and are upregulated by GC administration have also been already described. For example, such genes can be the *GILZ, GPX3, GGT1* and *ERP27* genes (see Tables 2 and 3, below).

Genes containing an IR-type negative element (IR nGRE DBS) among their promoter elements have been identified for the first time by the present Inventors (see Table 1- 3 below). In a preferred embodiment, such genes are chosen in the group consisting of the IR0 nGRE, IR1 nGRE and IR2 nGRE -containing genes disclosed in Tables 2 and 3. Preferably, such genes can be *BCL3, BHLHB2, ENC1, FFGFR3, FOXa2, GEM, MAFK CCND1, CYP26A1, JUND, HSD11β2, PRKCB, K14, RDH11, STRA13, RORα* and *TNFRSF19* genes.

**Table 1: Genes containing IR0 nGRE, IR1 nGRE and IR2 nGRE DBS in their promoter**

| **IR0 nGRE containing Genes** | **IR1 nGRE containing genes** | **IR2 nGRE containing genes** |
|---|---|---|
| ADAMTS19 | ADRA2A | CCR10 |
| AIP | AGPAT2 | COL4A3BP |
| AN04 | AKAP6 | DPAGT1 |
| CCND1 | ARHGAP23 | ELMOD3 |
| CITED 1 | ATN1 | ERP27 |
| DDI1 | BCL2L1 | FKBP2 |
| DLAT | BEST2 | FLOT2 |
| FBXL18 | BMP3 | FOXJ1 |
| FSD2 | BTG2 | FOXQ1 |
| GPIHBP1 | B4GALT2 | FSTL1 |
| HCST | CACNA2D3 | GAS6 |
| INHBE | CADM4 | GAS2L2 |
| KLF5 | CDH11 | GGT1 |
| LAG3 | CDK9 | GIT2 |
| MAPK4 | CTSA | GKN1 |
| MTG1 | CYP26A1 | GLU1 |
| NMYC | DYNLT3 | GOT1 |
| NDUFA4L2 | FBLN2 | GPR68 |
| NEU1 | GBA2 | GPR97 |
| PPIL5 | GNG10 | GRASP |
| PTMS | GPR108 | GUK1 |
| SLC25A5 | GPR153 | HSPB2 |
| SP9 | HSD11B2 | HSPB3 |
| SPARC | JUND | ISL2 |
| TCF25 | KLC3 | ITPKA |
| TRPM7 | KRT14 | MUC20 |
| ZBED4 | LRRC | POMGNT1 |
| ZFP113 | MT2 | PRKRA |
| ZFP467 | MTHFR | RDH5 |
| ZFP784 | MXI1 | RORA |
| | NKRF | SLC25A34 |
| | PRKCB | SLC2A8 |
| | RDH11 | SOCS1 |
| | SCP2 | TGM6 |
| | SGK3 | TNFRSF25 |
| | SOCS3 | |
| | SREBF1 | |
| | SSTR4 | |
| | STAB1 | |
| | STRA13 | |
| | TNFRSF19 | |
| | USF1 | |

The expression of all these NFκB or AP1 DNA DBS and IR nGRE DBS-containing genes are known to be decreased upon GC treatment.

All of these NFκB or AP1 DNA DBS, (+) GRE and IR nGRE DBS-containing genes are hereafter designated as "GC-dependent genes".

The term "tethered indirect transrepression" encompasses the repression of a gene via the binding of agonist-GR bound to a transactivator (e.g. NFκB, AP1,...) interacting with a cognate DBS.

The term "direct transrepression" encompasses the repression of a gene via the direct binding of agonist-liganded GR to an IR nGRE DBS.

The term "direct transactivation" encompasses the transactivation of a gene via the binding of agonist-liganded GR to a (+)GRE DBS.

As used therein, the expression of a gene is "significantly transrepressed" in presence of a candidate molecule if the level of expression of said gene in presence of said candidate molecule is decreased by a factor of at least two, preferably at least three, and more preferably of at least four as compared to the level of expression of said gene in absence of said candidate molecule. In other words, the expression level of said gene in the presence of said candidate molecule is of maximally 60%, preferably 30%, and more preferably 25 % of the expression level of said gene in the absence of said candidate molecule. In this case, the expression level of said gene in the presence of said candidate molecule is also said to be "significantly lower" than the expression level of the gene in the absence of said candidate molecule.

On the contrary, the transcription of a gene is "significantly transactivated" in presence of a candidate molecule if the level of expression of said gene in presence of said candidate molecule is increased by a factor of at least two, preferably at least three, and more preferably of at least four as compared to the level of expression of said gene in absence of said candidate molecule. In other words, the expression level of said gene in the presence of said candidate molecule is of minimally 200%, preferably 300%, and more preferably 400 % of the expression level of said gene in the absence of said candidate molecule. In this case, the expression level of said gene in the presence of said candidate molecule is also said to be "significantly upper" than the expression level of the gene in the absence of said candidate molecule.

Said gene expression level can be detected by any mean known by the person skilled in the art, for example by quantifying the mRNA level of said gene by RT-PCR and/or QRT-PCR, northern Blot, RNA micro-arrays, or any other RNA detecting mean. Said gene expression level can also be detected by measuring the amount of the gene-encoded protein by Elisa test, western blot, ImmunoHistochemistry, or HPLC or any other protein detecting mean.

As previously mentioned, the term "IR nGRE DBS" corresponds to a IR-type negative DNA binding site (DBS) which is present in the promoter of particular GR-responding genes (such as those listed in Table 1). This DBS is an imperfect palindromic sequence chosen in the group consisting of: CTCCxGGAGA with x= A, T, G or C (SEQ ID NO:1, also called "IR1 nGRE"), CTCCGGAGA (SEQ ID NO:2, also called IR0 nGRE), and CTCCxyGGAGA with x= A, T, G or C and y= A, T, G or C independently of each other (SEQ ID NO:3, also called IR2 nGRE) and tolerable variants thereof.

The present Inventors have found that various IR nGRE variants having a one base pair change as compared with said IR nGRE DBS, still enable GR-dependent direct transrepression. In a preferred embodiment, the tolerable variant of the IR nGRE DBS is thus derived by at least one base pair change from the sequence SEQ ID NO:1 (IR1 nGRE), SEQ ID NO:2 (IR0 nGRE), or SEQ ID NO:3 (IR2 nGRE), and has a GR-dependent direct transrepression activity.

More precisely, said tolerable variant of SEQ ID NO:1 (IR1 nGRE) can be chosen in the group consisting of: SEQ ID NO:11 to 27, and said tolerable variant of SEQ ID NO:3 (IR2 nGRE) can be chosen in the group consisting of: SEQ ID NO:28 to 48.

The process of the invention involves genes containing, among their promoter elements, a NFκB DBS having a sequence SEQ ID NO: 4, SEQ ID NO:5 or SEQ ID NO:6, or variants thereof. Preferably, said variants have a sequence which is homologous to SEQ ID NO: 4, SEQ ID NO:5 or SEQ ID NO:6.

The process of the invention involves genes containing, among their promoter elements, a AP1 DBS having a sequence SEQ ID NO:7, SEQ ID NO:8 or variants thereof. Preferably, said variants have a sequence which is homologous to SEQ ID NO:7 or SEQ ID NO:8.

The process of the invention involves genes containing, among their promoter elements, a (+) GRE DBS having a sequence SEQ ID NO:9, SEQ ID NO:10 or variants thereof. Preferably, said variants have a sequence which is homologous to SEQ ID NO:9 or SEQ ID NO:10.

As used herein, the term "homologous" refers to sequences that have sequence similarity. The term "sequence similarity", in all its grammatical forms, refers to the degree of identity or correspondence between nucleic acid sequences. In the context of the invention, two nucleic acid sequences are "homologous" when at least about 80%, alternatively at least about 81%, alternatively at least about 82%, alternatively at least about 83%, alternatively at least about 84%, alternatively at least about 85%, alternatively at least about 86%, alternatively at least about 87%, alternatively at least about 88%, alternatively at least about 89%, alternatively at least about 90%, alternatively at least about 91%, alternatively at least about 92%, alternatively at least about 93%, alternatively at least about 94%, alternatively at least about 95%, alternatively at least about 96%, alternatively at least about 97%, alternatively at least about 98%, alternatively at least about 99% of nucleic acids are similar. Preferably the similar or homologous nucleic acid sequences are identified by alignment using, for example, the GCG (Genetics Computer Group, Program Manual for the GCG Package, Version 7, Madison Wis.) pileup program, or using any of the proposed programs and algorithms dedicated thereto (for example, BLAST, or FASTA).

In a particular embodiment, the process of the invention comprises at least the following *in vitro* steps in cultured GR-expressing cells:
a) providing at least one candidate molecule,
b) providing a first vector comprising at least one NFκB or one AP1 DBS operatively coupled to a reporter gene and a promoter thereof active in said cell,
c) providing a second vector comprising an IR nGRE DBS operatively coupled to a reporter gene and a promoter thereof active in said cell,
d) providing a third vector comprising a (+) GRE DBS sequence operatively coupled to a reporter gene and a promoter thereof active in said cell,
e) selecting conditions in step b) wherein in NFκB and/or an AP1 factors are activated,
f) selecting conditions wherein said reporter genes of steps b) and c) are significantly expressed and significantly repressed by GCs, and wherein the expression of said reporter gene of step d) is significantly increased by the addition of GC,
g) measuring in said cells the level of expression of each reporter gene in the presence and absence of said candidate molecule, and under conditions selected in steps e) and f),
h) selecting said candidate molecule :
   - if it significantly transrepresses the expression of said reporter gene of step b), but does not significantly transrepress the expression of said reporter gene of step c), or
   - if it significantly transrepresses the expression of said reporter gene of step b), but does not significantly transrepress the expression of said reporter gene of step c), and does not significantly increase the expression of said reporter gene of step d).

As used herein, the term "vector" means the vehicle by which a DNA or RNA sequence of a foreign reporter gene can be introduced into an host cell so as to transform it and promote expression of the introduced reporter gene sequence. Vectors may include for example, plasmids, cosmids, YACs, phages, and viruses. For the purpose of the screening process of the invention, such vectors are preferably plasmids.

As used herein, the term "promoter" is a sequence of nucleotides from which transcription may be initiated of DNA operably linked downstream (i.e. in the 3' direction on the sense strand of double stranded DNA). Within the promoter sequence will be found a transcription initiation site (conveniently found, for example, by mapping with nuclease S1), as well as protein binding domains (consensus sequences) responsible for the binding of RNA polymerase. Promoters which may be used to control the reporter gene expression in the context of the present invention are for example: the SV40 early promoter and retroviral LTRs, to name few. Other suitable promoters are well known from the person skilled in the art. Preferably, the promoter which is used in the vector of the invention is the enhancer-less SV40 early promoter coupled to the SV40 enhancer and/or the vitamin D3 response element (VDRE).

The vectors which are used in the process of the invention can also contain sites for transcription initiation, termination, and, in the transcribed region, a ribosome binding site for translation.

The reporter gene sequence is "operatively coupled with" an expression control sequence (for example a promoter) in a vector, when RNA polymerase is able, in define conditions, to transcribe the reporter sequence into RNA, which is then trans-RNA spliced (if it contains introns) and is translated into that reporter protein.

Such reporter protein can be any protein having a quantifiable expression level. It is preferably chosen from the group consisting of: luciferase, β-galactosidase and fluorescent proteins.

The person skilled in the art knows will easily define the appropriate conditions wherein the reporter genes of said first and second vectors will be expressed, especially in the absence of said candidate molecule. For example, the vectors can be transfected into appropriate host cells, in appropriate culture mediums.

Representative examples of appropriate hosts include, but are not limited to animal cells such as CV1, CHO, COS-1, 293 and A549 cells. Appropriate culture mediums and conditions for the above-described host cells are known in the art. Among preferred eukaryotic vectors are pWLNEO, pSV2CAT, pOG44, pXT1 and pSG available from Stratagene; and pSVK3, pBPV, pMSG and pSVL available from Pharmacia, and pSGL3 and 4 available from Promega. Other suitable vectors will be readily apparent to the skilled artisan. Introduction of the construct into the host cell can be effected by calcium phosphate transfection, DEAE-dextran mediated transfection, cationic lipid-mediated transfection, electroporation, transduction, infection or other methods. Such methods are described in many standard laboratory manuals.

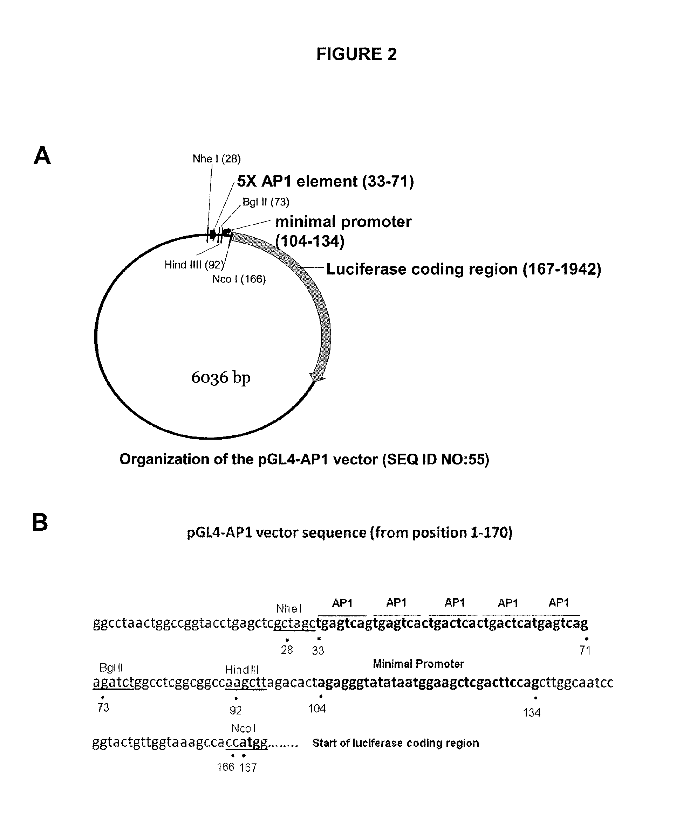

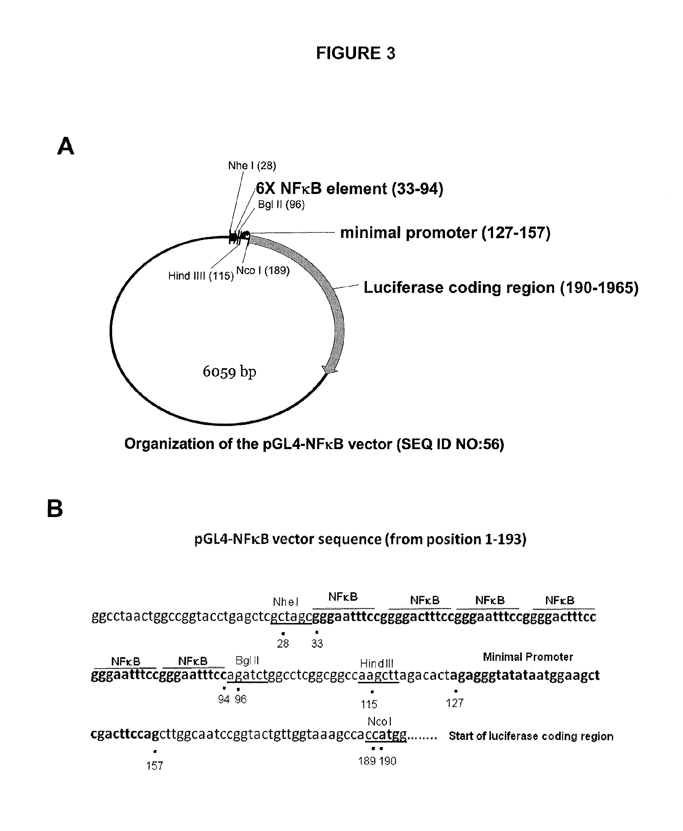

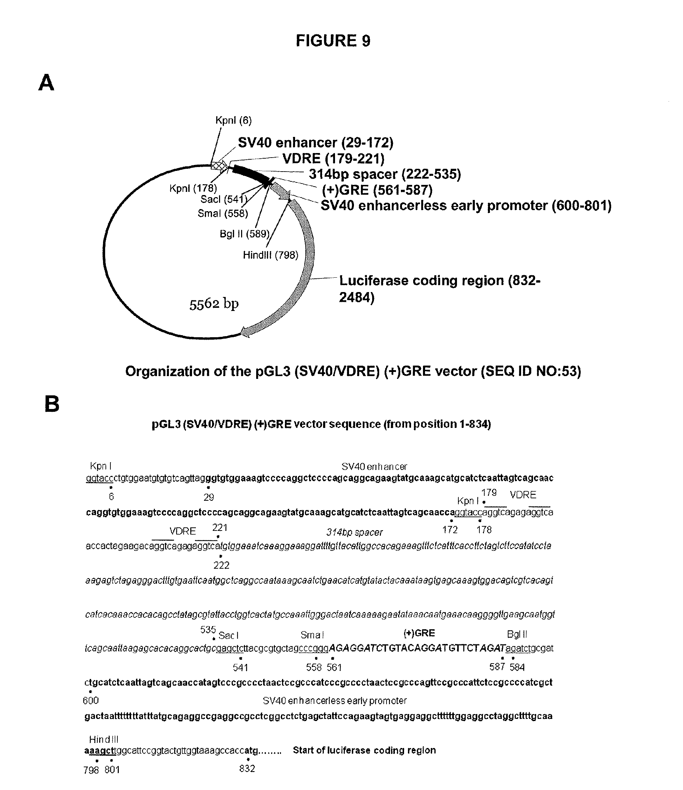

Preferably, in the context of the present invention, and as shown in the experimental section below, said (+) GRE or IR nGRE containing vector is a PGL3 promoter vector plasmid (Promega) containing a SV40 enhancerless early promoter upstream of the luciferase coding region, and containing the SV40 enhancer and/or two copies of consensus VDRE (VDR response element) upstream of the (+) GRE or IR nGRE element (see for example figures 5 and 9). Moreover, said AP1 or NFκB containing vector is preferably a PGL4-NFκB luc plasmid which contains 6 tandem copies of consensus NFκB response element upstream of a SV40 minimal promoter derived from (Promega) PGL4 vector or 6 tandem copies of consensus AP1 response element cloned into said pGL4 vector between the XhoI and BglII restriction sites (see Figures 2 and 3). Preferably, said PGL3 and PGL4 vectors are transfected into human A549 cells (CCL-185 ATTC).

Advantageously, AP1 and NFκB are activated with IL-1β (5ng/ml) in the case of A549 cells in culture, and by topical application of the phorbolester TPA in the case of skin (1nmole/cm²), and by LPS (IP, 1mg/kg) in the case of the liver and intestine. Activation and repression of reporter genes by GC is preferably performed by addition of 1µM Dex in culture medium. Expression of the reporter genes containing a VDRE (e.g./ vectors in Figures 4, 5, 6 and 7) is preferably induced by addition of 1µM vit D3 in the culture medium.

In a particular embodiment, the measuring step g) comprises the following sub-steps:
g0) measuring the expression level E0 of each reporter gene in the absence of said candidate molecule and under conditions wherein the expression of said reporter genes of steps b) and c) is significantly repressed by GC, whereas the expression of said reporter gene of step d) is significantly increased by the addition of GC,
g1) measuring the expression level E1 of each reporter gene in the presence of said candidate molecule, all other conditions being the same as in step g0),
and wherein, in said step h), said candidate molecule is selected if:
   - E1 is significantly lower than E0 for said reporter gene of step b), and E1 is not significantly lower than E0 for said reporter gene of step c), or
   - E1 is significantly lower than E0 for said reporter gene of step b), and E1 is not significantly lower than E0 for said reporter gene of step c), and E1 is not significantly higher than E0 for said reporter gene of step d).

Said reporter gene expression level can be detected by any mean known by the person skilled in the art, for example by quantifying the mRNA level of said gene or by measuring the amount of the reporter-encoded protein as previously described. Preferably, said reporter gene is luciferase, β galactosidase or a fluorescent protein. These assays are well known in the art.

In a preferred embodiment, the first vector which is provided in step b) of the process of the invention has the sequence SEQ ID NO:55 (PGL4-AP1 vector, figure 2A and B) or SEQ ID NO:56 (PGL4-NFKB vector, Figure 3A and B).

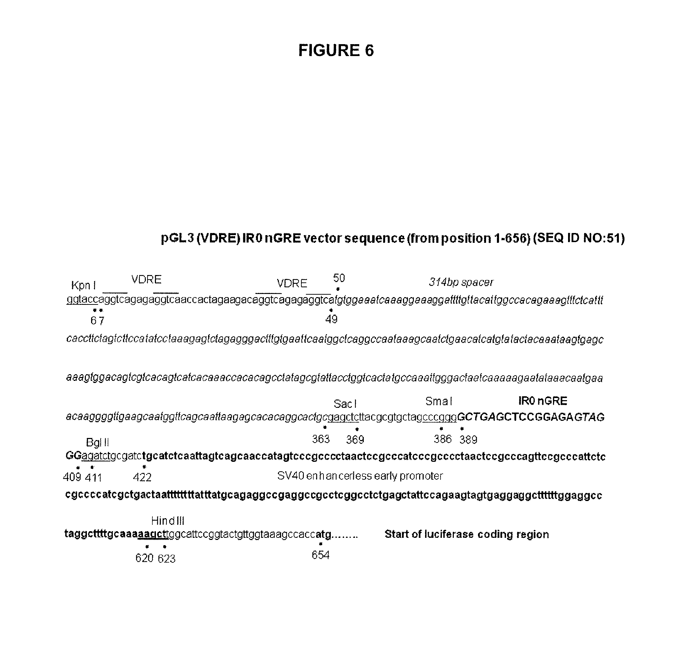

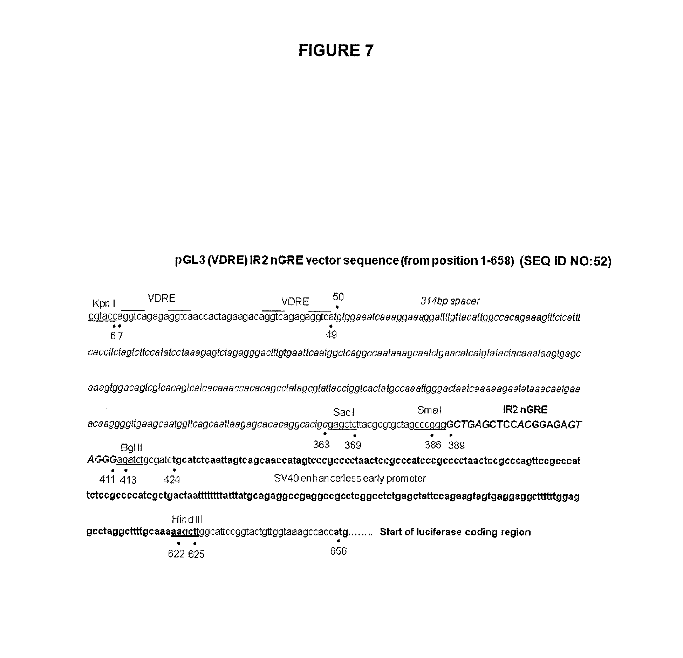

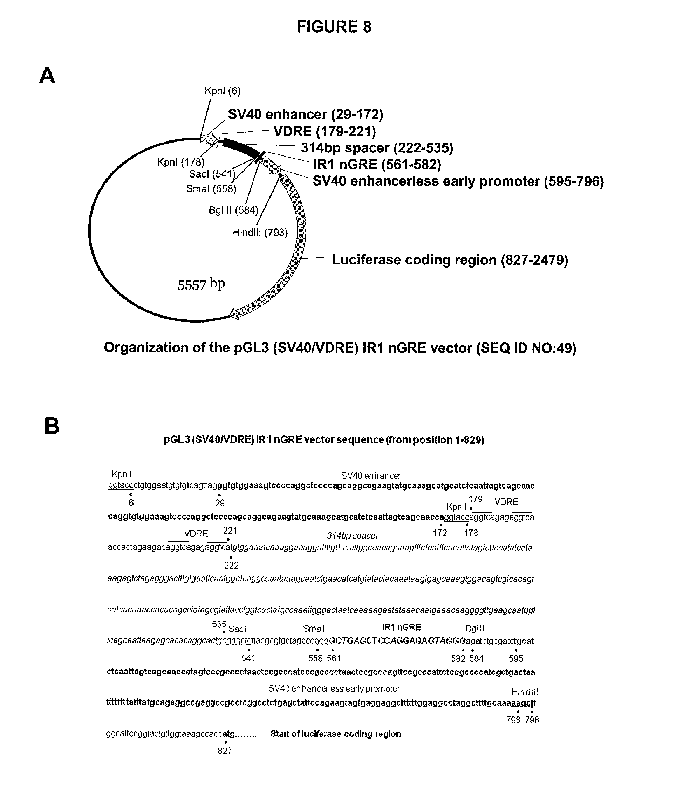

In another preferred embodiment, the second vector which is provided in step c) of the process of the invention has the sequence SEQ ID NO: 49 (PGL3 (SV40/VDRE) IR1 nGRE, Figures 8A and B), SEQ ID NO:50 (PGL3(VDRE) IR1 nGRE, Figure 4 A and B), SEQ ID NO:52 (PGL3(VDRE) IR2 nGRE, Figure 7) or SEQ ID NO:51 (PGL3(VDRE) IR0 nGRE, Figure 6).

In another preferred embodiment, the third vector which is provided in step d) of the process of the invention has the sequence SEQ ID NO:53 (PGL3(SV40/VDRE) (+) GRE, Figure 9A and B) or SEQ ID NO:54 (PGL3(VDRE) (+) GRE, Figure 5A and B).

As used herein, two gene expression levels will be considered as "similar" or "not significantly lower" or "not significantly higher" if their ratio (i.e. the ratio of their mRNA levels and/or of their protein levels) is comprised between 0.80 and 1.25, preferably between 0.85 and 1.17, preferably between 0.9 and 1.1, and more preferably between 0.95 and 1.05. On the other hand, the expression level of a gene in the presence of said candidate molecule is said to be "significantly lower" than the expression level of the same gene in the absence of said candidate molecule if the expression level of said gene in the presence of said candidate molecule is of maximally 50%, preferably 30%, and more preferably 25 % of the expression level of said gene in the absence of said candidate molecule.

In a particular embodiment, the process of the invention comprises the following steps:
a) providing at least one candidate molecule,
b) choosing at least one proinflammatory genomic gene which is known to be transrepressed by GC, and containing among its promoter elements at least one NFκB and/or one AP1 DBS, said gene being significantly expressed in said cells,
c) choosing at least one genomic gene which is known to be transrepressed by GC, and containing among its promoter elements at least one IR nGRE DBS, said gene being significantly expressed in said cells,
d) choosing at least one genomic gene containing among its promoter elements at least one (+) GRE DBS, the expression of said gene being significantly increased by the addition of GC in said cells,
e) selecting conditions wherein NFκB and/or AP1 factors are activated in step b), and selecting conditions wherein said reporter genes of steps b) and c) are significantly expressed and significantly repressed by GCs, and wherein the expression of said reporter gene of step d) is significantly increased by the addition of GC,
f) measuring the expression level E0 of each gene in said cells in the absence of said candidate molecule, under conditions selected in step e),
g) providing said candidate molecule to said cells and measuring the expression level E1 of each gene in the presence of said candidate molecule, all other conditions being the same as in step f),
h) selecting said candidate molecule if:
   - E1 is significantly lower than E0 for said gene of step b), and E1 is not significantly lower than E0 for said gene of step c), or
   - E1 is significantly lower than E0 for said gene of step b), and E1 is not significantly lower than E0 for said gene of step c), and E1 is not significantly higher than E0 for said gene of step d).

The present Inventors have found that, when cells are treated with said candidate molecule together with a GR antagonist known to relieve IRnGRE-mediated transrepression (such as RU486), the direct transrepression and transactivation of GC-dependent genes is affected, whereas the indirect transrepression of GC-dependent genes is not significantly affected.

In particular, when cells are treated with said candidate molecule with or without a co-treatment with any GR antagonist known to relieve IRnGRE-mediated transrepression, said candidate molecule is selected only if the expression of a gene which is known to be decreased by GC treatment is decreased by addition of said candidate molecule irrespective of the presence of said antagonist.

In a particular embodiment, the process of the invention therefore preferably further comprises the step of:
- providing concomitantly to said candidate molecule the antiglucocorticoid RU486, and
- measuring the expression level E2 of one gene chosen in step b) in the presence of said candidate molecule and said antiglucocorticoid, all other conditions being the same as in step f),
and wherein said candidate molecule is selected if E2 is significantly lower than E0 for said gene of step b).

In a preferred embodiment, in step b), said proinflammatory genomic gene which is known to be transrepressed by GC, and which contains among its promoter elements at least one NFκB and/or one AP1 DBS, is chosen in the group consisting of: the *COX2* and *MMP13, IL4,* and *IL10* genes.

In another preferred embodiment, in step c), said genomic gene which is known to be transrepressed by GC, and which contains among its promoter elements at least one IR nGRE DBS is chosen in the group consisting of: the *BCL3, BHLHB2, ENC1, FGFR3, FOXa2, GEM, MAFK CCND1, CYP26A1, JUND, HSD11β2, PRKCB, K14, RDH11, STRA13, RORα* and *TNFRSF19* genes.

In another preferred embodiment, in step d), said genomic gene containing among its promoter elements at least one (+) GRE DBS is chosen in the group consisting of: the *GILZ, GPX3, GGT1* and *ERP27* genes.

Said GC-dependent genes are further identified in Table 2 and Table 3 below:

**Table 2**

| **NFkB/AP1 DBS containing-, IR nGRE containing-, and (+)GRE containing genes analyzed in A549 cells** | |
|---|---|
| | **Ensembl accession number** |
| **NFkB/AP1 DBS containing genes** | |
| COX2 | ENSG00000073756 |
| MMP13 | ENSG00000137745 |
| | |
| **IR nGRE containing genes** | |
| BCL3 | ENSG00000069399 |
| BHLHB2 | ENSG00000134107 |
| ENC 1 | ENSG00000171617 |
| FGFR3 | ENSG00000068078 |
| FOXA2 | ENSG00000125798 |
| GEM | ENSG00000164949 |
| MAFK | ENSG00000198517 |
| | |
| **(+)GRE containing genes** | |
| GILZ | ENSG00000157514 |

| **Table 3 NFkB/AP1 DBS containing-, IR nGRE containing-, and (+)GRE containing genes analyzed in three mouse tissues** | | | | | |
|---|---|---|---|---|---|
| **Genes** | **Ensembl accession number** | **IR nGRE containing genes** | **Ensembl accession number** | **IR nGRE containing genes** | **Ensembl accession number** |
| **NFkB/AP1 DBS containing genes** | | | | | |
| COX2 | ENSMUSG00000032487 | COX2 | ENSMUSG00000032487 | COX2 | ENSMUSG00000032487 |
| IL4 | ENSMUSG00000000869 | IL1β | ENSMUSG00000027398 | | |
| IL10 | ENSMUSG00000016529 | | | | |
| IL1β | ENSMUSG00000027398 | | | | |
| TNFα | ENSMUSG00000024401 | | | | |
| MMP13 | ENSMUSG00000050578 | | | | |
| | | | | | |

| **IR nGRE containing genes** | | | | | |
|---|---|---|---|---|---|
| CCND1 | ENSMUSG00000070348 | CCND1 | ENSMUSG00000070348 | CCND1 | ENSMUSG00000070348 |
| CYP26A1 | ENSMUSG00000024987 | JUND | ENSMUSG00000071076 | JUND | ENSMUSG00000071076 |
| HSD11b2 | ENSMUSG00000031891 | PRKCB | ENSMUSG00000052889 | PRKCB | ENSMUSG00000052889 |
| K14 | ENSMUSG00000045545 | RDH11 | ENSMUSG00000066441 | RDH11 | ENSMUSG00000066441 |
| PRKCB | ENSMUSG00000052889 | RORα | ENSMUSG00000032238 | RORα | ENSMUSG00000032238 |
| STRAL3 | ENSMUSG00000030103 | STRAL3 | ENSMUSG00000030103 | STRAL3 | ENSMUSG00000030103 |
| TNFRSF19 | ENSMUSG00000060548 | TNFRSF19 | ENSMUSG00000060548 | TNFRSF19 | ENSMUSG00000060548 |
| | | | | | |

| **(+)GRE containing genes** | | | | | |
|---|---|---|---|---|---|
| GPX3 | ENSMUSG00000018339 | GPX3 | ENSMUSG00000018339 | GPX3 | ENSMUSG00000018339 |
| | | | | GGT1 | ENSMUSG00000006345 |
| | | | | ERP27 | ENSMUSG00000030219 |

In a preferred embodiment, GR-expressing cells are *in vitro* cultured cells, preferably human lung epithelial carcinoma cells A549, or cells from an animal tissue. More preferably, said animal is a mouse and said tissue is epidermis, liver or intestinal epithelium.

When GR-expressing cells are human lung epithelial carcinoma cells A549, said gene of step b) is preferably chosen from the *COX2* or *MMP13* genes, said gene of step c) is preferably chosen from the group consisting of: *BCL3, BHLHB2, ENC1, FGFR3, FOXa2, GEM* and *MAFK* genes, and said gene of step d) is preferably the *GILZ* gene.

When GR-expressing cells are from a tissue of an animal, said gene of step b) is preferably chosen in the group consisting of: the *COX2, IL4, IL1*β, *TNF*α, *MMP13* and *IL10* genes, said gene of step c) is preferably chosen in the group consisting of: *CCND1, CYP26A1, JUND, HSD11*β*2, PRKCB, K14, RDH11, STRA13, ROR*α and *TNFRSF19* genes and said gene of step d) is preferably chosen in the group consisting of the *GPX3, GGT1* and *ERP27* genes.

All these genes have been defined in Tables 2 and 3 above.

In a particular embodiment, said animal tissue is epidermis and the process of the invention comprises the following steps:
a) providing a candidate molecule and laboratory animals,
b) measuring, in said laboratory animals, after topical treatment of skin with the phorbol ester TPA (for example :1 nmole/cm²) in order to activate NFκB and/or AP1, the expression level E0 of a gene belonging to the group of inflammatory genes containing at least one NFκB and/or one AP1 DBS, said gene being preferably chosen in the group consisting of: the *COX2, IL4, IL1*β, *TNF*α*, MMP13 and IL10* genes,
c) measuring the expression level E0 of a gene containing a IR nGRE DBS, said gene being preferably chosen in the group consisting of: the *CCND1, CYP26A1, HSD11*β*2, PRKCB, K14*, *STRA13,* and *TNFRSF19* genes,
d) measuring the expression level E0 of a gene containing a (+) GRE DBS, said gene being preferably the *GPX3* gene,
e) topically treating the skin of said laboratory animals with said candidate molecule,
f) measuring the expression level E1 of each gene of steps b), c) and d) in the presence of said candidate molecule,
g) selecting said candidate molecule if:
   - E1 is significantly lower than E0 for said gene of step b), and E1 is not significantly lower than E0 for said gene of step c), or
   - E1 is significantly lower than E0 for said gene of step b), and E1 is not significantly lower than E0 for said gene of step c), and E1 is not significantly higher than E0 for said gene of step d).

In a particular embodiment, said animal tissue is liver and the process of the invention comprises the following steps:
a) providing a candidate molecule and laboratory animals,
b) measuring, in the liver tissue of said laboratory animals, after intraperitoneal treatment of lipopolysaccharide (LPS) (for example : IP, 1 mg/kg) in order to activate NFκB and/or AP1, the expression level E0 of a gene belonging to the group of inflammatory genes containing at least one NFκB and/or one AP1 DBS, said gene being preferably the *COX2* gene,
c) measuring in the same tissue as in b) the expression level E0 of a gene containing a IR nGRE DBS, said gene being preferably chosen in the group consisting of: the *CCND1, JUND, PRKCB, RDH11, RORα, STRA13* and *TNFRSF19* genes,
d) measuring in the same tissue as in b) the expression level E0 of a gene containing a (+) GRE DBS, said gene being preferably chosen in the group consisting of: the *GPX3, GGT1* and *ERP27* genes,
e) administering said candidate molecule intraperitoneally in said laboratory animals,
f) measuring in the same tissue as in b) the expression level E1 of each gene of steps b), c) and d) in the presence of said candidate molecule,
g) selecting said candidate molecule if:
   - E1 is significantly lower than E0 for said gene of step b), and E1 is not significantly lower than E0 for said gene of step c), or
   - E1 is significantly lower than E0 for said gene of step b), and E1 is not significantly lower than E0 for said gene of step c), and E1 is not significantly higher than E0 for said gene of step d).

In another particular embodiment, said animal tissue is intestinal epithelium and the process of the invention comprises the following steps:
a) providing a candidate molecule and laboratory animals,
b) measuring, in the intestinal epithelium tissue of said laboratory animals, after intraperitoneal treatment of lipopolysaccharide (LPS) (for example : 1mg/kg) in order to activate NFκB and/or AP1, the expression level E0 of a gene belonging to the group of inflammatory genes containing at least one NFκB and/or one AP1 DBS, said gene being preferably chosen in the group consisting of: the *COX2* and the *IL1*β genes,
c) measuring in the same tissue as in b) the expression level E0 of a gene containing a IR nGRE DBS, said gene being preferably chosen in the group consisting of: the *CCND1, JUND, PRKCB, RDH11, STRA13, ROR*α and *TNFRSF19* genes,
d) measuring in the same tissue as in b) the expression level E0 of a gene containing a (+) GRE DBS, said gene being preferably the *GPX3* gene,
e) administering said candidate molecule intraperitoneally in said laboratory animals,
f) measuring in the same tissue as in b) the expression level E1 of each gene of steps b), c) and d) in the presence of said candidate molecule,
g) selecting said candidate molecule if:
   - E1 is significantly lower than E0 for said gene of step b), and E1 is not significantly lower than E0 for said gene of step c), or
   - E1 is significantly lower than E0 for said gene of step b), and E1 is not significantly lower than E0 for said gene of step c), and E1 is not significantly higher than E0 for said gene of step d).

Said laboratory animals are preferably chosen from mice, rats, and rabbits, and are more preferably mice.

In another embodiment, the process of the invention comprises the steps of:
a) providing a candidate molecule and laboratory animals,
b) measuring, in one tissue of said laboratory animals, after topical or intraperitoneal treatment of lipopolysaccharide (LPS) or TPA in order to activate NFκB and/or AP1, the expression level E0 of at least one pro-inflammatory gene containing at least one NFκB and/or one AP1 DBS,
c) administering said candidate molecule topically or intraperitoneally in said laboratory animals,
d) measuring in the same tissue as in b) the expression level E1 of said gene in the presence of said candidate molecule,
e) administering topically or intraperitoneally in said laboratory animals, concomitantly to said candidate molecule, a GR antagonist having an IR nGRE mediated transrepression activity similar to that of the antiglucocorticoid RU486,
f) measuring in the same tissue as in b) the expression level E2 of said gene in the presence of said candidate molecule and said GR antagonist,
g) selecting said candidate molecule if E2 is significantly lower than E0 for said gene.

Said pro-inflammatory gene is chosen from: the *COX2, IL4, IL1*β*, TNF*α*, MMP13* and *IL10* genes if said tissue is epidermis, from the *COX2* and the *IL1*β genes, if said tissue is intestinal epithelium and is COX2 gene if the tissue is liver.

The administration of said candidate molecule and/or GR antagonist in steps c) and/or e) is performed either by topical application (for example for regulating the genes expressed in epidermis) or by injection (intraperitoneally or intraveinously), said administration being thus not a chirurgical act for said laboratory animal.

Preferably, said "GR antagonist having the same IR nGRE mediated transrepression activity as the antiglucocorticoid RU486" designates RU486 itself.

It is reminded that the expression level of a gene in the presence of said candidate molecule is said to be "significantly lower" than the expression level of the same gene in the absence of said candidate molecule if the expression level of said gene in the presence of said candidate molecule is of maximally 50%, preferably 30%, and more preferably 25 % of the expression level of said gene in the absence of said candidate molecule.

In other words, in the process of the invention, said GR-expressing cells are:
a) epidermal cells of the skin of an animal, preferably a mouse, on which said candidate molecule has been administered topically without or with the antiglucocorticoid RU486, wherein said gene in step b) is chosen in the group consisting in: the *COX2, IL4, IL1*β, *TNF*α, *MMP13* and *IL10* genes, said gene of step c) is chosen in the group consisting in: the *CCND1, CYP26A1, HSD11*β*2, PRKCB, K14, STRA13,* and *TNFRSF19,* and said gene of step d) is the *GPX3* gene,
b) cells obtained from the liver of an animal, preferably a mouse, on which said candidate molecule has been administered without or with the antiglucocorticoid RU486, wherein said gene in step b) is the *COX2* gene, said gene of step c) is chosen in the group consisting in: the *CCND1, JUND, PRKCB, RDH11, STRA13, ROR*α and *TNFRSF19* genes, and said gene of step d) is chosen in the group consisting of: the *GPX3, GGT1* and *ERP27* genes, or
c) cells obtained from the intestinal epithelium of an animal, preferably a mouse, on which said candidate molecule has been administered without or with the antiglucocorticoid RU486, wherein said gene in step b) is chosen in the group consisting in: the *COX2* and *IL1*β genes, said gene of step c) is chosen in the group consisting in: the *CCND1, JUND, PRKCB, RDH11, STRA13, ROR*α and *TNFRSF19* genes, and said gene of step d) is the *GPX3* gene.

In a second aspect, the present invention is drawn to the use of an IR nGRE DBS present in recombinant vectors and animal genomes for identifying a molecule exhibiting anti-inflammatory activities of GCs and having reduced GC-dependent IR nGRE-mediated direct transrepression activity.

Preferably, said IR nGRE DBS is chosen in the group consisting of: sequence SEQ ID NO:1 (IR1 nGRE), SEQ ID NO:2 (IR0 nGRE), SEQ ID NO:3 (IR2 nGRE), or tolerable variants thereof.

More preferably, said tolerable variant of SEQ ID NO:1 (IR1 nGRE) being preferably chosen in the group consisting of: SEQ ID NO:11 to 27, and said tolerable variant of SEQ ID NO:3 (IR2 nGRE) being preferably chosen in the group consisting of: SEQ ID NO:28 to 48.

In a third aspect, the present invention is drawn to the use of the antiglucocorticoid RU486 or of any GR antagonist having an IR nGRE-mediated transrepression activity similar to that of RU486, for identifying a molecule exhibiting anti-inflammatory activities of GCs and having reduced GC-dependent IR nGRE-mediated direct transrepression activity.

In a fourth aspect, the present invention is drawn to an isolated IR nGRE DNA binding site, chosen in the group consisting of: sequence SEQ ID NO:1 (IR1 nGRE), SEQ ID NO:2 (IR0 nGRE), SEQ ID NO:3 (IR2 nGRE), or tolerable variants thereof.

In a fifth aspect, the present invention is drawn to an isolated vector containing an IR nGRE DBS as defined above. Preferably, said vector is chosen in the group consisting of: SEQ ID NO: 49 (PGL3 (SV40/VDRE) IR1 nGRE), SEQ ID NO:50 (PGL3 (VDRE) IR1 nGRE), SEQ ID NO:51 (PGL3(VDRE) IR0 nGRE), and SEQ ID NO:52 (PGL3(VDRE) IR2 nGRE).

By "isolated" vector is intended a nucleic acid molecule which has been removed from its native environment. Examples of isolated DNA molecules include recombinant DNA molecules maintained in heterologous host cells or purified (partially or substantially) DNA molecules in solution.

This isolated vector can be advantageously used in the screening process of the invention or in any other process intended to select molecules exhibiting anti-inflammatory of GCs, with reduced GC-dependent IR nGRE-mediated direct transrepression activity.

### EXAMPLES

### I. MATERIAL AND METHODS

**Mice**. For topical treatment, 1 nmole (nm)/cm² MC903, at-RA or TPA; 6 nm/cm² FA, Dex or RU24858; and 90 nm/cm² RU486 were used. For systemic use, 100 ng/kg body weight active Vit D3, 8 mg/kg Dex and 64 mg/kg RU486 was intraperitoneally injected. GRdim mice were from the European Mouse Mutant Archives (EM:02123). Breeding, maintenance and experimental manipulation of mice were approved by the Animal Care and Use Committee of the IGBMC.

**Cell culture experiments**. A549 human lung epithelial cells (CCL-185, ATCC) were maintained in DMEM/HAM F12 (1:1) medium containing 10% foetal calf serum (FCS) and gentamycin. MLE12 mouse lung epithelial cells (CRL-2110, ATCC) were maintained in DMEM/Ham-F12 (1:1) medium containing 2% FCS, 5ug/ml insulin, 10ug/ml apo-trans bovine, 35 nM sodium selenite, 10nM β estradiol, 10mM HEPES and gentamycin.Cells were transfected using Fugene 6 reagent, as instructed (Roche). For RNA isolation from A549 cells, cells were seeded at 50% density in 60mm plates. 24 hours post-seeding, complete medium was replaced with medium containing charcoal-treated serum. 24 hours later, 1µM active vitamin D3 [1α, 25 (OH)2 Vitamin D3,], 300 nM FA, 4.5 µM RU486 (final concentration) or vehicle (acetone) were added to the medium for 6 hours. For ChIP assay, cells were treated with indicated compounds for 2 hours, followed by cross-linking by addition of formaldehyde (1% final concentration) to the medium and incubation on a rotary shaker for 10 minutes at room temperature. Cross-linking was stopped by adding 2M glycine (0.125M final concentration) and incubation for 5 minutes under similar condition, followed by 2X washing in ice-cold PBS. Cells were scraped using a rubber policeman, pelleted (400g, 5 minutes) at 4 °C, washed once in ice-cold PBS, snap frozen in liquid nitrogen and stored at -70 °C before proceeding for ChIP assay.

For ChIP assays to test GR and cofactor binding to the nGRE, +GRE or NFκB region of various luciferase reporter plasmids, ~60% confluent A549 cells in 100mm TC dish were transfected with 2µg of respective plasmids using fugene 6 reagent (Roche) and maintained in charcoal treated FCS containing medium. 44 hours post-transfection, 500nM FA or Dex or RU24858 and/or 5µM RU486, 5ng/ml IL-1β (final conc.) was added to the medium for 2 hours, followed by formaldehyde treatment and processing as described above.

**Luciferase reporter assay**. A549 cells seeded on 24-well tissue culture plates overnight at 70% confluency were transfected with 100 ng pCMV β galactosidase, 200 ng pGL3 reporter plasmids and wherever indicated, with 600 ng pSG5 hVDR plasmid (Green et al., 1988) into each well and maintained in medium containing charcoal treated FCS. 24 hours post-transfection, medium was changed and different compounds were added to it for 6 hours. Final concentrations of the compounds are: FA, 500nM; Dex or RU24858, 300nM or 500 nM (wherever 300nM Dex or RU was used, it is indicated in the figure, in all other cases, 500nM concentration was used); active vitamin D3, 1µM; IL-1β, 5ng/ml; BAY 11-7082, 5µM, JNK inhibitor II, 25µM. Luciferase assay was carried out as instructed (Promega). Normalized values are reported as the mean ± SEM; each value originates from at least three individual transfections with assays performed in duplicate.

**ChIP assay.** Isolated epidermis and intestinal epithelial cells were cross-linked in 1% formaldehyde followed by ChIP assay, as reported (Vaisanen et al., 2005).

**Nuclear run-on, EMSA, 3C and Luciferase assays.** Nuclear Run-on and EMSA (Carey and Smale., 2001), 3C (Liu et al., 2005) and Luciferase assays (Promega kit) were as described.

**Real time PCR.** Total RNA was reverse transcribed using hexamers, followed by Q-PCR, as reported (Li et al., 2005).

**Statis**tics. Data are represented as mean ± SEM of at least three independent experiments, and were analyzed using sigmastat (Systat Software) by the Student *t* test. *P* < 0.05 was considered significant.

**Bioinformatics analysis**. hg19 (human) and mm9 (mouse) repeat masked genome assembly was used to identify genome wide distribution of IR nGRE motifs. Gene functional annotation was performed using DAVID programme.

### II. RESULTS

### A) Glucocorticoid-induced GR-mediated transcriptional repression of TSLP expression involves a negative GRE located in the TSLP promoter region.

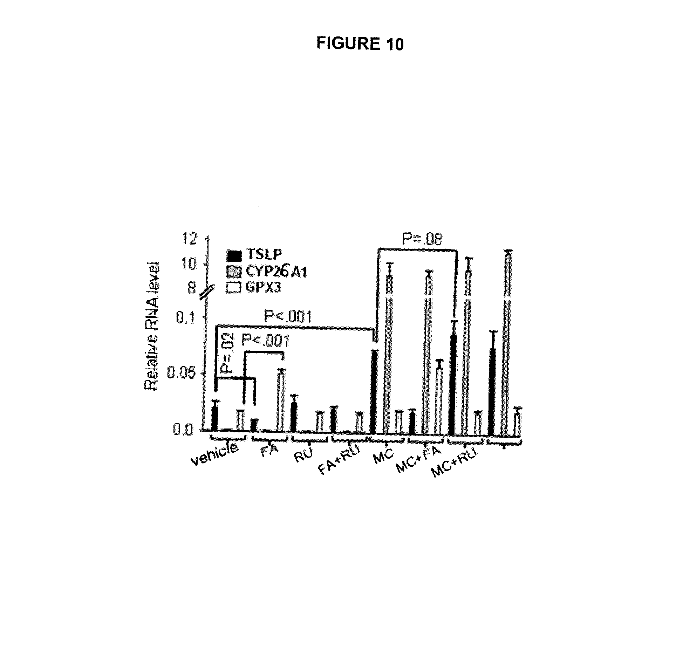

The GC agonist fluocinolone acetonide (FA) was applied to ears of mice concomitantly treated with the "low-calcemic" Vitamin D3 (VitD3) analog MC903 (Calcipotriol; hereafter called MC) to trigger TSLP expression (Li et al., 2006). In wild type (WT) mice, FA application inhibited basal TSLP RNA level by ~50%, which interestingly could be relieved by co-application of the GC antagonist RU486 (mifepristone, hereafter named RU), while MC-induced increase of TSLP RNA, which was fully blocked by FA, was also restored by RU co-treatment. As expected, the expression of the GC-inducible GPX3 (glutathione peroxidase 3) gene which harbours a (+)GRE (Tuckermann et al., 1999) was enhanced by FA (Figure 10) and inhibited by RU co-treatment, while FA or RU had no effect on MC-dependent expression of the CYP24A1 gene (a VitD3 target) (Figure 10).

The GR involvement in FA-induced inhibition of MC-induced TSLP expression in keratinocytes was demonstrated using adult mice in which GR was selectively ablated in keratinocytes (GR^{ep-/-} mice). Although the basal TSLP level was similar in vehicle-treated WT and GR^{ep-/-} mice, FA blocked MC-induced TSLP expression in WT, but not in GR^{ep-/-} mice (not shown). MC treatment was more efficient in GR^{ep-/-} than in WT mice, indicating that endogenous GCs may partially inhibit MC-induced TSLP expression in WT epidermis. Nuclear run-on assays demonstrated that GR-mediated FA inhibition of TSLP expression was transcriptional (not shown).

As neither NFκB nor AP1 are involved in TSLP induction by MC in epidermis (unpublished data), its repression was unlikely to be mediated by a "tethering" GRE. A bioinformatics analysis of 20 kb of DNA located upstream and downstream from the mouse and human TSLP translation start-site (+1) did not reveal any (+)GRE or known "composite" activating or repressing GRE, but unveiled the presence of a palindromic sequence consisting of two inverted repeated (IR) motifs separated by one bp (called hereafter IR1 nGRE), in the upstream promoter region of both mouse (m) and human (h) TSLP genes. Recombinant human GR protein in electrophoretic mobility shift (EMSA) and supershift assays with GR antibody showed that this putative mTSLP IR1 nGRE and its human counterpart, as well as the TAT (+)GRE (Meijsing et al., 2009), bound to the GR protein. These bindings were specific, as shown by lack of GR binding to a mutant (+)GRE and to three mTSLP IR1 nGRE mutants. Complexes formed between the recombinant GR and either putative IR1 nGREs or (+)GRE similarly migrated (data not shown).

### B) The repressing activity of the TSLP nGRE tolerates changes in spacing and/or sequence of its repeated motifs.

To investigate whether additional DNA elements could be required to generate a repressing activity, the TSLP IR1 nGRE was inserted upstream of an enhancerless SV40 early promoter located 5' to the luciferase coding sequence of pGL3 vector. A VDRE separated from the IR1 nGRE by a 314 bp-long DNA segment devoid of any known transregulator binding site (not shown) was inserted to generate a luciferase-expressing reporter plasmid (pGL3(VDRE) IR1 nGRE, Figure 4), which was transfected into A549 cells, followed by addition of VitD3 and/or FA. FA addition did not affect luciferase expression in absence of IR1 nGRE, whereas its presence resulted in decreased expression (which could be prevented by RU addition) of basal and VDRE-mediated VitD3-induced transcription (Figure 11). As expected, FA-induced increase in luciferase expression was observed when IR1 nGRE was replaced by a (+)GRE (PGL3(VDRE) (+)GRE vector, Figure 5). The TSLP IR1 nGRE exhibited a similar FA-inducible repressing activity when embedded in 413 bp of its natural environment (not shown). Thus, on its own, TSLP IR1 nGRE is sufficient to mediate a FA-inducible repressing activity. Replacing, in the reporter, IR1 nGRE by a mutant to which recombinant GR does not bind resulted in the loss of GR binding to the IR nGRE region, and in no FA-inducible repressing activity (data not shown), thus supporting the conclusion that GR binds directly to TSLP nGRE in cultured cells.

To investigate whether the 1 bp spacer between the inverted repeated motifs of TSLP IR1 nGRE was critical for its repressing function, pGL3-based luciferase plasmids containing the VDRE and TSLP nGRE motifs spaced by 0 to 5 bases (IR0 to IR5 nGREs) were transfected into A549 cells. ChIP assays showed that, upon FA addition, GR similarly bound IR1 and IR2 nGREs, whereas its binding was less efficient on IR0 nGRE, and not detectable on IR3, IR4 and IR5 nGREs (not shown). Accordingly, upon FA addition, the decrease in basal and VitD3-induced luciferase activity was stronger for IR1 and IR2 than for IR0 nGRE, whereas no significant change could be detected for IR3, IR4 and IR5 nGREs (not shown).

pGL3-based luciferase plasmids containing VDRE and TSLP IR1 nGREs (Figure 4A), in which individual bases had been changed one by one, were used to study whether non-canonical IR1 nGREs could function as efficient nGREs. With one exception, at least a one base pair change was "tolerable" at any position of the TSLP IR1 nGRE and did not impair its activity *in vitro,* suggesting that GR bound to "degenerate" IR1 elements might also mediate GC-induced direct repression *in vivo* (see below). A similar analysis carried out with plasmids containing IR2 nGREs showed that a one base pair change was "tolerable" at any position of IR2 nGRE (not shown). The IR1 nGRE having efficient GR binding are SEQ ID NO: 11 to SEQ ID NO:27, and the IR2 nGRE having efficient GR binding are SEQ ID NO:28 to SEQ ID NO:48.

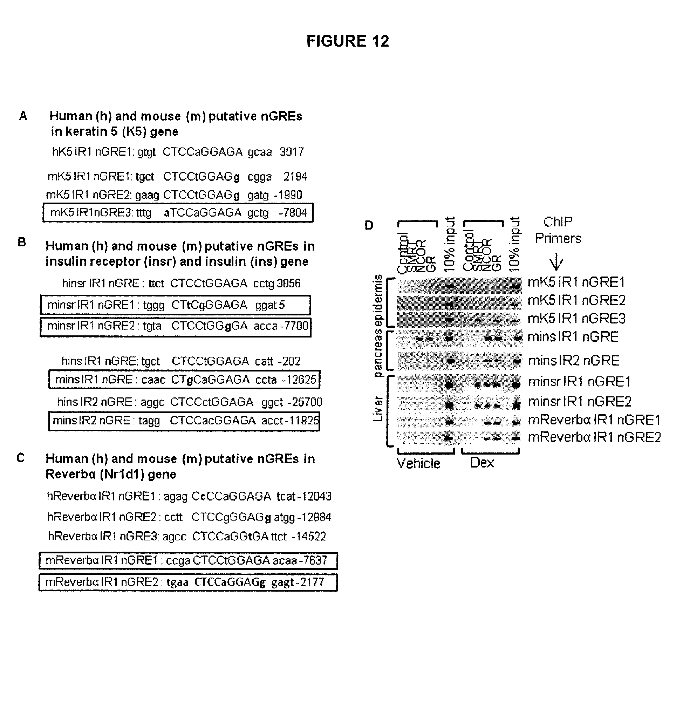

As Keratin 5 (K5), known to be down-regulated by GCs (Ramot et al., 2009), was not present in the list of human/mouse orthologues that contain a canonical nGRE, it was studied whether this absence could reflect the presence of a canonical IR1 nGRE in human K5, while a "tolerable" change would exist in its mouse orthologue, or vice-versa. One canonical IR1 nGRE in human K5 gene and 3 putative nGREs in its mouse orthologue were found, each of them exhibiting one "tolerable" change *in vitro* (Figure 12 A). Only one of them (mK5 IR1 nGRE3, Figure 12 D) allowed formation of a GR-SMRT repressing complex in epidermis of Dex-treated mice, suggesting that not all *in vitro* "tolerable" IR1 nGRE variants are functional in a given tissue.

Similarly, the insulin (ins) and insulin receptor (insr) genes were studied, as their expression was reported to be down-regulated by GCs (Delaunay et al., 1997). A canonical IR1 nGRE was found in the human insulin receptor gene (hinsr IR1 nGRE), and two IR1 nGREs, each bearing one "tolerable" change, were present in the mouse (Figure 12B). Upon Dex treatment, functional GR-SMRT/NCoR repressing complexes were assembled on both mouse insulin receptor IR1 nGRE variants (minsr IR1 nGRE1 and 2 in Figure 12D). Remarkably, in addition to a canonical IR2 nGRE in both human and mouse insulin genes, a "tolerable" IR1 nGRE variant was present in mouse insulin gene, while a canonical IR1 nGRE was present in the human gene (Figure 12 B).

It was also looked at Reverbα, as its down-regulation by GCs was previously reported (Duez and Staels, 2008, and Refs therein). A canonical IR1 nGRE was found in mouse, but not in human Reverbα gene, which contains 3 IR1 nGREs each bearing one "tolerable" variant (Figure 12C). Note that, in the mouse, this gene also contains an IR1 nGRE variant (mReverbα IR1 nGRE2), that is identical to one of the human nGRE variants, and appears to be as functional as the mouse canonical IR1 nGRE1, as judged from ChIP assays with liver extracts (Figure 12D).

### C) Mouse genes that contain IR0, IR1 and IR2 nGREs conserved in their human orthologues are repressed upon GC agonist treatment in vivo.

Bioinformatics analyses of mouse and human genomes revealed thousands of genes containing IR elements made up of inverted repeated motifs identical to those of TSLP (CTCC and GGAGA) with either no (IR0), 1 (IR1) or 2 (IR2) bp spacers. A comparison of mouse and human orthologues indicated conservations of such IR elements (51 IR0, 379 IR1 and 566 IR2). Within these orthologue gene families, 35 IR0, 50 IR1 and 50 IR2 nGRE-containing mouse genes (Table 4) were randomly chosen to investigate whether (i) they were expressed in epidermis, intestinal epithelium and liver, (ii) their expression was inhibited by the GC agonist Dexamethasone (Dex), (iii) this inhibition could be relieved by RU486 co-administration, and also if it could be correlated with GR and corepressor binding to their putative IR nGREs.

For 9 of the above "IR1" genes, the repressing activity of their putative IR1 nGREs was tested *in vitro* using the luciferase assay. In all cases, and irrespective of the tissue pattern of GC-induced repression, these putative IR1 nGREs "repressed" luciferase activity (Figure 13), indicating that these IR1 nGREs elements are *bona fide* IR1 nGREs. Importantly, upon GC treatment *in vivo,* there was a tight correlation between repression of a given gene in a given tissue (and its relief by RU co-administration), and GR and corepressor association with the IR1 nGRE of that gene in that tissue. In contrast there was no GR and corepressor association with the IR1 element of BMP3 gene which is not "repressed" in either tissue.

The "IR0" genes (Table 4) and "IR2" genes (Table 4) were also cell-specifically or non-cell-specifically expressed and repressed in the three tissues. The GC-induced repressing potential of putative IR nGREs of some of the selected "IR0" and "IR2" genes was tested using the luciferase assay, and it was found that they exhibited a repressing activity, irrespective of their activity *in vivo* (not shown). Upon Dex treatment, a decrease in gene expression in a given tissue always correlated with GR and corepressor association with the IR0 or IR2 element in that tissue, and in all cases the GC-induced repression was relieved by co-administration of RU. It can be concluded that, as expected from the *in vitro* luciferase assays, not only IR1, but also IR0 and IR2 nGREs can efficiently mediate the cis-acting GC agonist-induced repressing activity of the GR*, in vivo.*

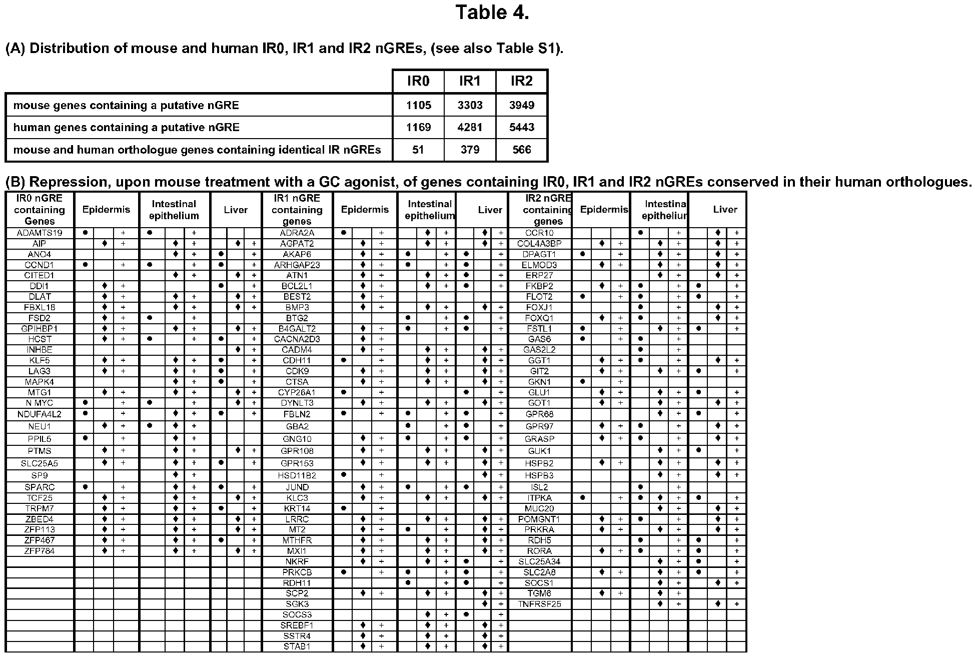

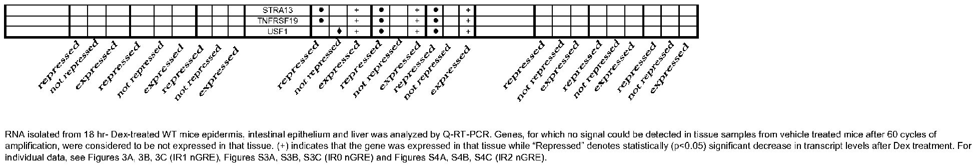

### D) Differential effects of the GRdim mutation and RU486 treatment on GC-induced "tethered"- and IR nGRE-mediated transrepressions.

The GRdim mutation does not affect GC-induced NFκB and AP1-mediated "tethered" transrepression, whereas it impairs (+)GRE transactivation (Tuckermann et al., 1999). In contrast, we found that Dex-induced IR nGRE-mediated gene repression was abolished by this mutation (data not shown). Expression of "IR0 nGRE" CCND1, "IR1 nGRE" PRKCB and "IR2 nGRE" FSTL1 genes, was repressed by Dex treatment in WT epidermis, but not in GRdim mutant. Note that epidermis was treated as indicated with TPA, to activate NFκB and AP1 factors. As expected, the repression of the Cox2 gene that contains NFκB and AP1 binding sites, was not impaired in mutant epidermis, while the (+)GRE-containing GPX3 gene was not GC-induced (Figure 14). Accordingly, ChIP assays showed that Dex-induced repressing complexes were formed on IR nGREs of CCND1, PRKCB and FSTL1 genes of WT but not of GRdim mice, while no activating complex was formed on the GPX3 (+)GRE gene in Dex-treated epidermis of GRdim mutants (not shown), and repressing complexes were assembled, upon epidermis Dex treatment, on the Cox2 NFκB/AP1-containing region in both WT and dim mutants (not shown).

Importantly, the effect of Dex treatment was reverted by excess RU co-treatment in the case of IR nGRE-mediated transrepression, whereas RU had little effect on NFKB/AP1-mediated "tethered" transrepression (Figure 14). Similar results concerning the differential RU effects were obtained when NFκB and AP1 factors were activated in MLE12 mouse cells by addition of IL-1β, instead of TPA treatment (not shown).

### E) Failure of "dissociated GCs" to prevent undesirable side effects of corticoid therapy could be due to IR nGREs-mediated transrepression.

The present results led to posit that GC-induced transcriptional repression of IR nGRE-containing genes could contribute to the GC undesirable effects.

IL-1 β-"activated" A549 cells transfected with pGL4- and pGL3-based reporter plasmids were used to examine the activities exhibited *in vitro* by RU24858 for (i) "tethered" transrepression (Figures 2 and 3, PGL4-NFκB and PGL4-AP1 vector), and (ii) (+)GRE-mediated transactivation (Figure 5, PGL3(VDRE) (+)GRE vector). As expected from its "dissociated" profile *in vitro,* RU24858 was almost as efficient as Dex at repressing IL-1β-induced activation of transcription by NFκB (Figure 15A, left panel) and AP1 (Figure 15A, right panel). Accordingly, RU24858 was as efficient as Dex at recruiting a repressing complex "tethered" to NFκB bound to its cognate element in the PGL4-NFκB vector (not shown). In contrast, RU24858 was much less efficient than Dex at inducing transactivation of (+)GRE pGL3 luciferase plasmid (not shown).

The "activity profile" of RU24858 was also investigated *in vivo.* Unlike Dex, a topical RU24858 treatment did not activate GC-dependent expression of the (+)GRE-containing GPX3 gene in epidermis, nor of the GGT1 and ERP27 genes in liver (not shown), and did not induce assembly of an activating complex on their (+)GRE (data not shown). In contrast, RU24858 was as efficient as Dex at down-regulating, through NFκB-mediated "tethered" transrepression, genes of which the skin expression was enhanced by topical TPA treatment (not shown). Most interestingly, and as *in vitro,* RU24858 was also as efficient as Dex at inducing transrepression of IR0 (CCND1), IR1 (TSLP, CYP26A1, K14, PRKCB) and IR2 (DPAGT1) nGRE-containing genes (Figure 15B), through recruitment of GR-SMRT/NCoR repressing complexes on nGRE regions (data not shown).

Long-term treatments with GCs generate numerous debilitating effects. An ontology search revealed that the known or putative functions of almost 15% of IR nGRE-containing orthologue genes could possibly be implicated in physiological homeostatic processes leading to side-effects upon GC therapy. Moreover, there is evidence that repression of expression of a number of these latter genes could actually be instrumental to the generation of defects subsequent to GC administration, either because their expression is known to be decreased upon GC treatment, and/or because their decreased expression is known to generate defects related to those produced by GC-therapy. Note, however, that many of the NFκB and AP1 binding site-containing genes that encode regulatory components (e.g. cytokines) of the immune system, also contain IR nGREs, while genes encoding anti-apoptotic proteins (Bcl2 and Bcl-XL), as well as mitogenic proteins involved in cell cycle progression at the G1/S phase (Cyclin D1 and CDK4) can also be GC-transrepressed via IRnGREs (Table 5).

**Table 5.**

| **A. IR nGRE- containing genes whose GC-induced transrepression could generate side effects related to those produced by GC therapy (see also Table S3)** | | | | | |
|---|---|---|---|---|---|
| **Debilitating side effects upon GC therapy** | **Gene symbol** | **Gene name** | **a** | **b** | **References** |
| **Skin atrophy, bruising,** | *Krt 14, Krt* | Keratin 14 **(IR1)**, Kerafin 5 **(IR1)** | + | - | Ramot et al , 2009 |
| **thinning, brittle skin,** | *5* | | | | |
| **disturbed wound** | | Transforming growth factor beta 1 precursor **(IR1)** | + | - | Frank et al , 1996 |
| **healing** | *TGFβ1* | SMAD family member 4 **(IR2)** | - | + | Chen et al , 2000 |
| **(Schacke et al., 2002)** | *Smad4* | Tenascin C **(IR2)** | + | - | Fassler et al , 1996 |
| | *Tnc* | Transient receptor potential cation channel | - | + | Cheng et al , 2010 |
| | *Trpv3* | subfamily V member 3 **(IR2)** | | | |
| | | Cyclin D1 **(IR0)** | + | + | |
| | *Ccnd1* | Cyclin-dependent kinase 4 **(IR2)** | + | + | Rogatsky et al , 1999 |
| | *Cdk4* | | | | |
| **Impaired skeletal** | *Tnfrsf11b* | Osteoprotegerin **(IR2)** | + | + | Sasaki et al , 2001 |
| **growth and** | *Bcl2* | Bcl- 2 **(IR1)** | + | - | Mocetti et al , 2001 |
| **osteoporosis** | *Bcl2l1* | Bcl- XL **(IR1)** | + | - | Lu et al , 2007 |
| **(Schacke et al., 2002,** | *TGFβ1* | Transforming growth factor beta 1 precursor **(IR1)** | - | + | Geiser et al , 1998 |
| **Kleiman and** | *Smad* 4 | SMAD family member 4 **(IR2)** | - | + | Tan et al , 2007 |
| **Tuckermann, 2007)** | *Ghr* | Growth hormone receptor **(IR1)** | + | + | Gevers et al , 2002 |
| | *Gnas* | Adenylate cyclase stimulating G-alpha protein **(IR1)** | - | + | Weinstein et al , 2004 |
| | *Wnt5a* | Wingless-related MMTV Integration site 5A **(IR1)** | - | + | Yang et al , 2003 |
| | *Ahsg* | Alpha -2- HS- glycoprotein precursor **(IR0)** | - | + | Szweras et al , 2002 |
| | *Col11a2* | Collagen, type XI, alpha 2 chain precursor **(IR2)** | - | + | Li et al , 2001 |
| **Hyperglycemia and Diabetes (Schacke et** | *Ins* | Insulin precursor **(IR1, IR2)** | + | + | Delaunay et al , 1997, |
| **al., 2002, Kleiman and Tuckermann, 2007)** | *Insr* | Insulin receptor **(IR1)** | + | + | Caro and Amatruda , 1982, |
| **Muscle** | *ctnnb1* | Beta -catenin **(IR1)** | + | + | Schakman et al , 2008b |
| **atrophy/Myopathy** | *Akt1* | Protein kinase B **(IR2)** | - | + | Schakman et al , 2008a |
| **(Schakman et al., 2008a)** | *Tpm2* | Tropomysin beta chain **(IR1)** | - | + | Ochala et al , 2007 |
| **Impaired HPA axis, Adrenal insufficiency** | *Mc2r* | ACTH receptor **(IR1)** | + | + | Chida et al , 2007 |
| **(Schacke et al., 2002)** | *Mrap* | ACTH receptor accessory protein **(IR1)** | + | + | Metherell et al , 2005 |
| **Circadian rhythm disorder, metabolic** | *Clock* | Circadian locomoter output cycle kaput protein **(IR1)** | - | + | Roybal et al , 2007 |
| **syndrome, bipolar disorder and mania** | *Nr1d1* | Reverbα **(IR1)** | + | - | Torra et al , 2000, Preitner et al , 2002 |
| **(Bechtold et al., 2010, Duez and Staels, 2008)** | *Rora* | RORα **(IR2)** | + | - | This study |
| **Anxiety and depression (Schacke** | *Ucn2* | Urocortin 2 **(IR1)** | + | + | Chen et al , 2004, 2006, Neufeld-Cohen et al , 2010 |
| **et al., 2002)** | *Crhr2* | Corticotropin releasing hormone receptor 2 **(IR1)** | - | + | Bale et al , 2000 |
| **Hypertension (Schacke et al., 2002)** | *Hsd11b2* | Corticosteroid 11-beta-dehydrogenase isozyme 2 (11β-HSD2) **(IR1)** | + | + | Stewart et al , 1996 |
| **a**.Genes whose expression is known to be decreased upon GC treatment. | | | | | |
| **b**.Genes, whose decreased expression is known to generate effects related to those produced by GC treatment. | | | | | |
| **(IR0), (IR1)** and **(IR2)** indicate the type of nGRE motif present in that gene. | | | | | |

| **B. IR nGRE- containing genes involved in GC-anti-inflammatory therapy (see also Table S3)** | | | | | |
|---|---|---|---|---|---|
| **Gene symbol** | | **Gene name*** | **Gene symbol** | | **Gene name*** |
| *C1qb* | | Complement C1q subcomponent subunit B Precursor | *II16* | | Interleukin-16 Precursor **(IR1)** |
| *C1ql1* | | **(IR2)** | *II17rb* | | Interleukin-17 receptor b **(IR1)** |
| C3 | | C1q-related factor Precursor **(IR1)** | *II11ra1* | | Interleukin-11 receptor **(IR1)** |
| Cfd | | Complement C3 Precursor **(IR1)** | *II17b* | | Interleukin-17b Precursor **(IR1)** |
| *II6* | | Complement factor D Precursor **(IR1)** | *II17f* | | Interleukin-17f Precursor **(IR1)** |
| *II20* | | Interleukin-6 Precursor **(IR1)** | *II28a* | | Interleukin-28a Precursor **(IR1)** |
| *Ccr10* | | Interleukin-20 Precursor **(IR2)** | *II28b* | | Interleukin-28b Precursor **(IR1)** |
| *Stat3* | | C-C chemokine receptor type 10 **(IR2)** | *II24* | | Interleukin-24 Precursor **(IR1)** |
| *Nfatc1* | | Signal transducer and activator of transcription 3 | *II34* | | Interleukin-34 Precursor **(IR1)** |
| *II8ra* | | **(IR2)** | *II1rn* | | Interleukin-1 receptor antagonist protein |
| *II12rb1* | | Nuclear factor of activated T-cells, cytoplasmic 1 | *II22ra1* | | Precursor **(IR2)** |
| *II17ra* | | **(IR1)** | *TSLP* | | Interleukin-22 receptor a1 **(IR1)** |
| | | Interleukin-8 receptor a **(IR1)** | | | Thymic Stromal Lymphopoietin **(IR1)** |
| | | Interleukin-1 receptor b1 **(IR1)** | | | |
| | | Interleukin-17 receptor a **(IR1)** | | | |
| * All of these genes also contain NFκB and AP1 binding sites. | | | | | |

### BIBLIOGRAPHY

Belchtold, D.A., Gibbs, J.E., and Loudon, A.S. (2010). Circadian dysfunction in disease. Trends
Pharmacol. Sci. 31, 191-198.
Belvisi, M.G., et al.. (2001). J. Immunol. 166, 1975-1982*.*
Carey, M., et al.. (2001). Nex York Cold Spring Harbor Laboratory Press.
Chrousos, G.P. (2009). Nat. Rev. Endocrinol. 5, 374-381*.*
De Bosscher, K., and Haegeman, G. (2009). Mol. Endocrinol. 23, 281-291.
Delaunay, F., et al.. (1997). J. Clin. Invest. 100, 2094-2098*.*
Dostert, A., and Heinzel, T. (2004). Curr. Pharm. Des. 10, 2807-2816*.*
Duez, H., and Staels, B. (2008) FEBS Lett. 582, 19-25.
Elbe-Burger, A., et al.. (1997) Immunology Methods Manual, ed. Lefkovits, pp1490-1501.
Flores-Langarica, A., et al.. (2005). Proc. Natl. Acad. Sci. USA 102, 19039-19044*.*
Green, S., et al.. (1988). Nuc. Acid Res. 16, 369.
Gross, K.L., and Cidlowski J.A. (2008). Trends Endocrinol. Metab. 19, 331-339*.*
Huang, Da.W., et al.. (2009). Nat. Protoc. 4, 44-57.
Karin, M. (1998). Cell 93, 487-490.
Kassel, O., and Herrlich, P. (2007). Mol. Cell. Endocrinol. 275, 13-29.
Kleiman, A., and Tuckerman, J.P. (2007). Glucocorticoid receptor action in beneficial and side effects of steroid therapy: Lessons from conditional knockout mice. Mol. Cell. Endocrinol. 275, 98-108.
Li, M., et al.. (2005). Proc. Natl. Acad. Sci. USA 102, 14795-14800
Liu, Z. and Garrard, W. T. (2005). Mol. Cell Biol. 25, 3220-3231.
Lonard, D.M., and O'Malley, B.W. (2007). Nuclear receptor coregulators: judges, juries and executioners of cellular regulation. Mol. Cell 27, 691-700*.*
Meijsing, S.H., et al.. (2009). Science 324, 407-410.
Nader, N., et al.. (2010). Trends Endocrinol. Metab. 21, 277-286.
Ramot, Y., et al.. (2009). Bioessays 31, 389-399.
Reddy, T.E., et al.. (2009). Genome Res. 19, 2163-2171*.*
Reichardt, H.M., et al.. (1998). Cell 93, 531-41.
Rhen, T., and Cidlowski, J.A. (2005). N. Engl. J. Med. 353, 1711-23.
Ricketson, D., et al.. (2007). J. Mol. Biol. 368, 729-741
Sambrook, J., et al.. (1989). Molecular Cloning: A Laboratory Manual, 2nd Ed. (New York : Cold Schake, H., Docke, W.D., and Asadullah, K. (2002). Mechanisms involved in the side effects of glucocorticoids. Pharmacol. Ther. 96, 23-43*.*
Schakman, O., Gilson, H., and Thissen, J.P. (2008a). Mechanisms of glucocorticoid-induced myopathy. J. Endrocrinol, 197, 1-10*.*
*Spring Harbor Laboratory Press).*
Stewart, P.M., et al.. (1996). Lancet 347, 88-91
Tuckermann, J.P. et al.. (1999). J. Cell Biol. 147, 1365-1370.
Vaisanen, S., Dunlop, et al.. (2005). J. Mol. Biol. 350, 65-77*.*
Vayssiere, B.M., et al.. (1997). Mol. Endocrinol, 11, 1245-1255.
Wang J.C., Derynck et al.. (2004). Proc. Natl. Acad. Sci. USA 101, 15603-15608*.*

## Claims

1. A process for selecting a molecule exhibiting anti-inflammatory activities of glucocorticoids (GCs), *via* glucocorticoid receptor (GR)-dependent tethered indirect transrepression, and having reduced GC-dependent IR nGRE-mediated direct transrepression activity, comprising the following steps:
i) providing at least one candidate molecule,
ii) testing if said candidate molecule:
d) significantly induces GR-dependent tethered indirect transrepression of the transcription of at least one gene containing among its promoter elements at least one NFκB or one AP1 DNA binding sequence (DBS),
e) significantly induces GR-dependent direct transrepression of the transcription of at least one gene containing among its promoter elements an IR-type negative element (IR nGRE DBS),
f) significantly induces GR-dependent transactivation of the transcription of at least one gene containing among its promoter elements a (+) GRE DBS,
iii) selecting said candidate molecule:
• if it significantly transrepresses the transcription of said gene in step a), but it does not significantly transrepress the transcription of said gene in step b), or
• if it significantly transrepresses the transcription of said gene in step a), but does not significantly transrepress the transcription of said gene in step b) and does not significantly increase the transactivation of the transcription of said gene in step c),
wherein said IR nGRE DBS is chosen in the group consisting of: sequence SEQ ID NO: (IR1 nGRE), SEQ ID NO:2 (IR0 nGRE), SEQ ID NO:3 (IR2 nGRE) and tolerable variants thereof,
wherein said NFκB DBS has the sequence SEQ ID NO: 4, SEQ ID NO:5 or SEQ ID NO:6, or variants thereof,
wherein said AP1 DBS has the sequence SEQ ID NO:7, SEQ ID NO:8 or variants thereof, and wherein said (+) GRE DBS has the sequence SEQ ID NO:9 or SEQ ID NO:10 or variants thereof.

2. The process according to claim 1, comprising at least the following *in vitro* steps, in cultured GR-expressing cells:
a) providing at least one candidate molecule,
b) providing a first vector comprising at least one NFκB or one AP1 DBS operatively coupled to a reporter gene and a promoter thereof active in said cells,
c) providing a second vector comprising an IR nGRE DBS operatively coupled to a reporter gene and a promoter thereof active in said cells,
d) providing a third vector comprising a (+) GRE DBS sequence operatively coupled to a reporter gene and a promoter thereof active in said cells,
e) selecting conditions wherein NFκB and/or AP1 factors are activated in step b),
f) selecting conditions wherein said reporter genes of steps b) and c) are significantly expressed and significantly repressed by GCs, and wherein the expression of said reporter gene of step d) is significantly increased by the addition of GC,
g) measuring in said GR-expressing cells the level of expression of each reporter gene in the presence and absence of said candidate molecule, and under conditions selected in steps e) and f),
h) selecting said candidate molecule:
- if it significantly transrepresses the expression of said reporter gene of step b), but does not significantly transrepress the expression of said reporter gene of step c), or
- if it significantly transrepresses the expression of said reporter gene of step b), but does not significantly transrepress the expression of said reporter gene of step c), and does not significantly increase the expression of said reporter gene of step d).

3. The process according to claim 2, wherein said step g) comprises the following sub-steps:
g0) measuring the expression level E0 of each reporter gene in the absence of said candidate molecule and under conditions wherein the expression of said reporter genes of steps b) and c) is significantly repressed by GC, whereas the expression of said reporter gene of step d) is significantly increased by the addition of GC,
g1) measuring the expression level E1 of each reporter gene in the presence of said candidate molecule, all other conditions being the same as in step g0), and wherein, in said step h), said candidate molecule is selected if:
- E1 is significantly lower than E0 for said reporter gene of step b), and E1 is not significantly lower than E0 for said reporter gene of step c), or
- E1 is significantly lower than E0 for said reporter gene of step b), and E1 is not significantly lower than E0 for said reporter gene of step c), and E1 is not significantly higher than E0 for said reporter gene of step d).

4. The process according to any one of claims 1 to 3, wherein said tolerable variant is an IR nGRE DBS derived by at least one base pair change from the sequence SEQ ID NO:1 (IR1 nGRE), SEQ ID NO:2 (IR0 nGRE), or SEQ ID NO:3 (IR2 nGRE), and has a GR-dependent direct transrepression activity.

5. The process according to any one of claims 1 to 4, wherein said tolerable variant of SEQ ID NO:1 (IR1 nGRE) is chosen in the group consisting of: SEQ ID NO:11 to 27, and said tolerable variant of SEQ ID NO:3 (IR2 nGRE) is chosen in the group consisting of: SEQ ID NO:28 to 48.

6. The process according to any one of claims 2 to 5, wherein said first vector is SEQ ID NO:55 (PGL4-AP1) or SEQ ID NO:56 (PGL4-NFκB), wherein said second vector is SEQ ID NO: 49 (PGL3 (SV40/VDRE) IR1 nGRE), SEQ ID NO:50 (PGL3 (VDRE) IR1 nGRE), SEQ ID NO:51 (PGL3(VDRE) IR0 nGRE), or SEQ ID NO:52 (PGL3(VDRE) IR2 nGRE) and wherein said third vector is SEQ ID NO:53 (PGL3 (SV40/VDRE) (+)GRE) or SEQ ID NO:54 (PGL3 (VDRE) (+) GRE).

7. The process according to any one of claims 1, and 4 to 6, comprising the following steps in GR-expressing cells:
a) providing at least one candidate molecule,
b) choosing at least one proinflammatory genomic gene which is known to be transrepressed by GC, and containing among its promoter elements at least one NFκB and/or one AP1 DBS, said gene being significantly expressed in said cells,
c) choosing at least one genomic gene which is known to be transrepressed by GC, and containing among its promoter elements at least one IR nGRE DBS, said gene being significantly expressed in said cells,
d) choosing at least one genomic gene containing among its promoter elements at least one (+) GRE DBS, the expression of said gene being significantly increased by the addition of GC in said cells,
e) selecting conditions wherein NFκB and/or AP1 factors are activated in step b), and selecting conditions wherein said reporter genes of steps b) and c) are significantly expressed and significantly repressed by GCs, and wherein the expression of said reporter gene of step d) is significantly increased by the addition of GC,
f) measuring the expression level E0 of each gene in said cells in the absence of said candidate molecule, under conditions selected in step e),
g) providing said candidate molecule to said cells and measuring the expression level E1 of each gene in the presence of said candidate molecule, all other conditions being the same as in step f),
h) selecting said candidate molecule if:
- E1 is significantly lower than E0 for said gene of step b), and E1 is not significantly lower than E0 for said gene of step c), or
- E1 is significantly lower than E0 for said gene of step b), and E1 is not significantly lower than E0 for said gene of step c), and E1 is not significantly higher than E0 for said gene of step d).

8. The process according to claim 7, further comprising the step of providing concomitantly to said candidate molecule the antiglucocorticoid RU486 and measuring the expression level E2 of one gene chosen in step b) in the presence of said candidate molecule and said antiglucocorticoid, all other conditions being the same as in step f), and wherein said candidate molecule is selected if E2 is significantly lower than E0 for said gene of step b).

9. The process according to any one of claims 7 to 8, wherein said GR-expressing cells are *in vitro* cultured cells, preferably human lung epithelial carcinoma cells A549, and wherein said gene of step b) is chosen from the *COX2* or *MMP13* genes, said gene of step c) is chosen from the *BCL3, BHLHB2, ENC1 , FGFR3, FOXa2, GEM* and *MAFK* genes, and said gene of step d) is the *GILZ* gene.

10. The process according to any one of claims 7 to 8, wherein said GR-expressing cells are obtained from a tissue of an animal, preferably a mouse, and wherein said gene of step b) is chosen in the group consisting of: the *COX2, IL4, IL1β, TNFα, MMP13* and *IL10* genes, said gene of step c) is chosen in the group consisting of: *CCND1, CYP26A1, JUND, HSD11β2, PRKCB, K14, RDH11, STRA13, RORα* and *TNFRSF19* genes and said gene of step d) is chosen in the group consisting of: the *GPX3, GGT1* and *ERP27* genes.

11. The process according to any one of claims 7 to 8, wherein said GR-expressing cells are:
a) epidermal cells of the skin of an animal, preferably a mouse, on which said candidate molecule has been administered topically without or with the antiglucocorticoid RU486, wherein said gene in step b) is chosen in the group consisting in: the *COX2, IL4, IL1β, TNFα, MMP13* and *IL10* genes, said gene of step c) is chosen in the group consisting in: the *CCND1, CYP26A1, HSD11β2, PRKCB, K14, STRA13,* and *TNFRSF19,* and said gene of step d) is the *GPX3* gene,
b) cells obtained from the liver of an animal, preferably a mouse, on which said candidate molecule has been administered without or with the antiglucocorticoid RU486, wherein said gene in step b) is the *COX2* gene, said gene of step c) is chosen in the group consisting in: the *CCND1, JUND, PRKCB, RDH11, STRA13, RORα* and *TNFRSF19* genes, and said gene of step d) is chosen in the group consisting of: the *GPX3, GGT1* and *ERP27* genes, or
c) cells obtained from the intestinal epithelium of an animal, preferably a mouse, on which said candidate molecule has been administered without or with the antiglucocorticoid RU486, wherein said gene in step b) is chosen in the group consisting in: the *COX2* and *IL1β* genes, said gene of step c) is chosen in the group consisting in: the *CCND1, JUND, PRKCB, RDH11, STRA13, RORα* and *TNFRSF19* genes, and said gene of step d) is the *GPX3* gene.

12. Use of an IR nGRE DBS present in recombinant vectors and animal genomes for identifying a molecule exhibiting anti-inflammatory activities of GCs and having reduced GC-dependent IR nGRE-mediated direct transrepression activity.

13. The use according to claim 12, wherein said IR nGRE DBS is chosen in the group consisting of:
sequence SEQ ID NO:1 (IR1 nGRE), SEQ ID NO:2 (IR0 nGRE), SEQ ID NO:3 (IR2 nGRE), or tolerable variants thereof, said tolerable variant of SEQ ID NO: 1 (IR1 nGRE) being preferably chosen in the group consisting of: SEQ ID NO:11 to 27, and said tolerable variant of SEQ ID NO:3 (IR2 nGRE) being preferably chosen in the group consisting of: SEQ ID NO:28 to 48.

14. Use of the antiglucocorticoid RU486 or of any GR antagonist having an IR nGRE-mediated transrepression activity similar to that of the antiglucocorticoid RU486, for identifying a molecule exhibiting anti-inflammatory activities of GCs and having reduced GC-dependent IR nGRE-mediated direct transrepression activity.

15. An isolated IR nGRE DNA binding site, chosen in the group consisting of: sequence SEQ ID NO:1 (IR1 nGRE), SEQ ID NO:2 (IR0 nGRE), SEQ ID NO:3 (IR2 nGRE), or tolerable variants thereof.

16. An isolated vector containing an IR nGRE DBS as defined in claim 1,4 or 5, said vector being preferably chosen in the group consisting of: SEQ ID NO: 49 (PGL3 (SV40/VDRE) IR1 nGRE), SEQ ID NO:50 (PGL3 (VDRE) IR1 nGRE), SEQ ID NO:51 (PGL3(VDRE) IR0 nGRE), and SEQ ID NO:52 (PGL3(VDRE) IR2 nGRE).
